# EUROPEAN PATENT APPLICATION

(11) **EP 4 663 753 A1**
(43) Date of publication of application: **17.12.2025**
(21) Application number: 24752881.3
(22) Date of filing: 06.02.2024
(51) Int. Cl.: C12N 9/12, C12N 5/10, C12N 15/54, C12N 15/62, C12N 15/63, C12N 15/85, A61K 38/45

(54) **TRANSPOSASE, TRANSPOSON, TRANSPOSON SYSTEM, AND USE THEREOF**

(30) Priority: 08.02.2023 CN 202310081106
(71) Applicant: Maxirna (Shanghai) Pharmaceutical Co., Ltd., Songjiang District Shanghai 201601 (CN); Maxirna (Zhejiang) Technology Co., Ltd., Shaoxing, Zhejiang 312467 (CN); Shanghai Cell Therapy Group Co., Ltd, Jiading District Anting Town Shanghai 201805 (CN)
(72) Inventor: LIU, Tao, Shanghai 201805 (CN); ZHANG, Pingjing, Shanghai 201805 (CN); SONG, Chengyi, Shanghai 201805 (CN); GAO, Bo, Shanghai 201805 (CN); WANG, Yali, Shanghai 201805 (CN); WANG, Saisai, Shanghai 201805 (CN); SUN, Yan, Shanghai 201805 (CN); QIAN, Qijun, Shanghai 201805 (CN)
(74) Representative: Nederlandsch Octrooibureau
(86) International application number: PCT/CN2024/076381
(87) International publication number: WO 2024/165026

(57) **Abstract**

Provided are a transposase, a transposon, a transposon system and the use thereof. The transposase is a mutant transposase or a fusion transposase. Compared with a wild-type PS transposase represented as SEQ ID NO: 1, the mutant transposase contains amino acid mutation. The fusion transposase is formed by fusing a functional polypeptide onto the wild-type PS transposase or a mutant thereof. Compared with a wild-type PS transposon, the transposon of the present invention contains nucleotide mutation and/or TIR multi-copies in tandem and/or TA multi-copies in tandem. The transposon system contains the transposase or a polynucleotide encoding same, and the transposon or the PS transposon. A JL transposase, a JL transposon, and a JL transposon system containing said JL transposase and JL transposon, which are obtained by engineering transformation of the wild-type PS transposase by the means described above remarkably improve the transposition gene integration efficiency.

## Description

The present application claims priority to Chinese Patent Application No. 202310081106.8, filed on February 8, 2023, the contents of which are incorporated herein by reference in their entirety.

### TECHNICAL FIELD

The present invention relates to the field of genetic engineering, and specifically to an engineered modified transposase, an engineered modified transposon, an engineered modified transposon system and applications thereof.

### BACKGROUND

A transposable genetic element, also known as a transposon, is a DNA segment that can be moved from one genomic location to another within a single cell. The transposon can be categorized into two broad groups based on a transposition mechanism thereof: (1) for an element known as a reverse transcriptional transposon, transposition can occur through reverse transcription of an RNA intermediate, and (2) for a DNA transposon, the transposition can occur through the direct transposition of a DNA flanked by inverted terminal repeats (TIRs or ITRs). An active transposon encodes one or more proteins required for the transposition. A natural active DNA transposon carries a transposase gene.

The transposon has been widely used as a gene transfer vector in research in the fields of transgenesis, gene capture and gene therapy, and has achieved excellent application results. The transposon can be used as a natural DNA transfer vector and can efficiently carry an exogenous gene to be inserted into a genome. The role of the transposon is similar to that of a viral vector. Moreover, the transposition process can be controlled by regulating the supply volume of the transposase. Therefore, the transposon can be divided into a transposon donor vector and a transposase auxiliary vector. A DNA fragment of interest, such as a fluorescent marker gene GFP/RFP, a gene capture kit, a therapeutic gene construct, can be cloned between the two sides of the TIR and used as a plasmid for the transposon donor vector. The transposase vector comprises a promoter, a transposase gene expression frame, PolyA, and other components. The transposase can also be in the form of mRNA or a protein. The transposon donor vector and the transposase can be co-transfected into a cell or injected into a receptor to be able to achieve stable gene insertion in a target cell.

A Tc1/Mariner transposon is the most widely distributed class of DNA transposon superfamily in nature, and includes a bacterium, an invertebrate and a vertebrate. For the vertebrate, the Tc1/Mariner transposon is most widely distributed in an osteichthyan. By means of bioinformatics and experimental validation, 16 members of a Tc1/Mariner superfamily transposon with a natural transposition activity have been reported, namely Tc1, Tc3, Minos, Mos1, Bari3, Fot1, impala, Famar1, Osmar5, ISY100, Mboumar-9, Passport, Tana1, Thm3, SB, ZB, and PS transposons.

Prior patent applications CN110257425A and CN112159822A disclose a PS transposon with a unique transposition catalytic structural domain "D35D", which belongs to a pogo transposon superfamily and can exhibit a certain degree of transpositional and shearing activities in a mammalian cell. Although having a certain transgenic application value, the PS transposon system has lower transposition efficiency because the PS transposase sequence is a wild-type natural amino acid sequence. In some types of cell or gene therapies, PS does not have a significant advantage over a *Piggybac* (PB) efficient transposon or a Sleeping Beauty (SB) efficient transposon in the art.

It is well known that a naturally active transposon may cause genomic instability. Therefore, during evolution, the transposon accumulates a mutation and gradually becomes less active or even inactive, e.g., most of the Tc1/Mariner superfamily transposons found in a higher animal (e.g., a mammal) lose their transpositional activity due to a defect (e.g., the mutation, frameshift, insertion, absence, or presence of a termination codon) in an open reading frame of the transposase. For these reasons, the Sleeping Beauty (SB) transposon and the *PiggyBac* transposon, which are currently most widely used in a clinical gene therapy, are often highly efficient mutant transposases/transposon systems that are molecularly reconstructed and/or engineered by bioinformatics.

In view of the above, the PS transposon system still needs to be mutated with an amino acid/a DNA and/or engineered to obtain a transposase and/or a transposon system that is more efficient and is of more genetically practical value.

### SUMMARY

In one aspect, the present disclosure provides a transposase (a JL transposase), which is a mutant transposase or a fusion transposase.

The mutant transposase comprises an amino acid mutation as compared to a wild-type PS transposase as shown in SEQ ID NO: 1. The amino acid mutation is that any one or more amino acids in a double-stranded DNA binding oligomerization domain of the wild-type PS transposase are mutated to a positively charged amino acid; and/or the amino acid mutation comprises one or more mutations selected from the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V.

The fusion transposase is formed by fusing a functional polypeptide onto the wild-type PS transposase or a mutant thereof. The functional polypeptide is a DNA sequence-specific or non-specific binding domain, and/or a cell-nucleus localization signal structural domain.

In one or more embodiments, the positively charged amino acid is selected from histidine, lysine, or arginine, and/or, the amino acid mutations are selected from one or more mutations of the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K.

In one or more embodiments, the mutant comprises one or more mutations selected from the following group compared to the wild-type PS transposase as shown in SEQ ID NO: 1. The mutant, compared to the wild-type PS transposase as shown in SEQ ID NO: 1, includes one or more mutations selected from the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V, Q69R, L100I, K96R, Q162R, A271K, R372K, H20Y, TYCR150-153KFVI, N232K, A363N, A377C, Q346M, A17V, N105G, Q76E, I354V, VS133-134IA, S253K, V370I, SV297-298TE, EH286-286DS, A352S, ANS241-243VHE, S297T, A79I, T147S, I178M, I276V, Q331E, R257E, T339S, A79V, H104L, V74L, S107E, Q142E, R375K, S243E, R195K, Q22E, A328Q, G111A, R110K, I365V, VTE380-382SNA, R190K, RA372-373KS, K73L, K121R, R102K, C152V, IY276-277VW, V292I, K73E, H104M, I239V, K42E, K356Q, T150K, G316S, IY259-260VW, V53M, E108D, V53L, DKV72-74EEL, M280F, S85G, N63K, V91L, K251L, H124N, K308S, N10D, H7R, G112S, H7K, TA327-328KE.

In one or more embodiments, the mutant, compared to the wild-type PS transposase as shown in SEQ ID NO: 1, includes one or more mutations selected from the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V, Q69R, L100I, K96R, Q162R, A271K, R372K, H20Y, TYCR150-153KFVI, N232K, A363N, A377C, Q346M, A17V, N105G, Q76E, I354V, VS133-134IA, S253K, V370I, SV297-298TE, EH286-286DS, A352S, ANS241-243VHE, S297T, A79I, T147S, I178M, I276V, Q331E, R257E, T339S, A79V, H104L, V74L, S107E, Q142E, R375K, S243E, R195K, Q22E, A328Q, G111A, R110K, I365V, VTE380-382SNA.

In one or more embodiments, the DNA sequence-specific or non-specific binding domain includes a leucine zipper structural domain, a CRISPR/Cas structural domain, a TALE domain, a zinc finger domain, an AAV Rep DNA-binding domain, or any combination thereof, and preferably, the leucine zipper structural domain has an amino acid sequence as shown in any one of SEQ ID NOs: 28-31.

In one or more embodiments, the nuclear localization signal domain comprises SV40 NLS, C-myc NLS, TAF1 NLS, TP53 NLS, STAT3 NLS or any combination thereof.

In one or more embodiments, the functional polypeptide comprises or does not comprise a linker for connecting to the wild-type PS transposase or the mutant thereof.

In a further aspect, the present disclosure provides a polynucleotide for encoding a transposase of any embodiment herein.

In one or more embodiments, the polynucleotide is a DNA or a messenger RNA.

In a further aspect, the present disclosure provides a transposon which is recognizable by a transposase of any one of the above embodiments. The transposon has any one, two or three features selected from (1) to (3) below:
(1) the transposon has a mutation inverted terminal repeat, the mutation inverted terminal repeat has 1-5 nucleotide mutations compared to a wild-type PS transposon inverted terminal repeat shown in SEQ ID NO: 2 or 3;
(2) the transposon has more than two inverted terminal repeats at least one end; and
(3) the transposon has more than two repeats of the TA sequence outside the inverted terminal repeat at least one end.

In one or more embodiments, a mutation ITR is as shown in any one of SEQ ID NOs: 4-27, preferably as shown in any one of SEQ ID NOs: 4-6, or is a nucleotide sequence comprising any combination of nucleotide mutations in SEQ ID NOs: 4-27, or is a reverse complementary sequence thereof.

In one or more embodiments, the transposon has two inverted terminal repeats at each of two ends and comprises four TA sequence repeats outside of the inverted terminal repeats at each of the two ends.

In some embodiments, the transposon comprises a combination of DNA elements positioned between the two ITRs. The combination of DNA elements includes but is not limited to, a promoter, an enhancer, an expression gene, 5-UTR, 3-UTR, and other sequence elements known to a person skilled in the art. In some embodiments, either of the two ITRs (both are reversely complementary) includes a sequence having at least 50%, at least 60%, at least 70%, at least 80%, at least 90%, at least 95%, at least 98%, or at least 99% identity to SEQ ID NO: 2 or 3.

In one or more embodiments, the transposon comprises or does not comprise a target gene (or an exogenous gene) expression frame positioned between the ITRs. The target gene expression frame may comprise a gene functional element such as a promoter, a target gene, and a polyA signal sequence.

In one or more embodiments, the target gene encodes a protein selected from a cellular receptor, an immune checkpoint protein, a cytokine, a T cell receptor, a B cell receptor, a chimeric antigen receptor, and any combination thereof.

In a further aspect, the present disclosure provides a transposon system, comprising a transposase of any embodiment herein or a polynucleotide for encoding the transposase; and a transposon which is the transposon of any embodiment herein or a PS transposon.

In one or more embodiments, the polynucleotide for encoding the transposase is an mRNA. Preferably, the mRNA is chemically modified.

In one or more embodiments, the transposon is present in a DNA vector. Preferably, the DNA vector is selected from a conventional cyclic DNA plasmid and a conventional linear DNA plasmid, and also selected from a microcyclic plasmid, a nanoplasmid, a doggybone linear miniplasmid, and other forms of DNA that does not comprise an antibiotic or/and a replicon DNA sequence. Preferably, the DNA vector is an antibiotic resistance gene-free miniplasmid.

In one or more embodiments, the DNA vector is a microvector. A backbone sequence of the microvector has a length of 600 bp or less, and has a reduced DNA motif and/or a CpG-free DNA motif.

In one or more embodiments, the polynucleotide for encoding the transposase is DNA. The DNA and the transposon are present in the same DNA vector or in different DNA vectors.

In some embodiments, a polynucleotide molecule comprising a sequence for encoding a JL transposase and/or a transposon TIR is present in a viral vector, including but not limited to an AAV, a lentivirus, a retrovirus, an adenovirus, and the like.

In a further aspect, the present disclosure provides a host cell, comprising or being capable of producing a transposase of any embodiment herein, a polynucleotide of any embodiment herein, a transposon of any embodiment herein, or a transposon system of any embodiment herein.

In a further aspect, the present disclosure provides a method of preparing a cell, including: the step of introducing into a cell a transposase of any embodiment herein, a polynucleotide of any embodiment herein, a transposon of any embodiment herein, or a transposon system of any embodiment herein.

In some embodiments, the introduction comprises contacting the cell with the polynucleotide. In some embodiments, the introduction comprises contacting the cell with a DNA vector comprising the transposon. In some embodiments, the introduction comprises contacting the cell with mRNA for encoding the transposase and a plasmid vector comprising the transposon. In some embodiments, the introduction comprises contacting the cell with a plasmid vector comprising the polynucleotide and the transposon. In some embodiments, the introduction comprises contacting the cell with the plasmid vector comprising the polynucleotide and the plasmid vector comprising the transposon. In some embodiments, the introduction comprises contacting the cell with the transposase.

In some embodiments, the cell is a primary cell isolated from a subject. In some embodiments, the subject is a human. In some embodiments, the subject is a patient suffering from a disease. In some embodiments, the subject has been diagnosed with a cancer or a tumor. In some embodiments, the cell is isolated from the blood of the subject. In some embodiments, the cell comprises a primary immune cell. In some embodiments, the cell comprises a primary white blood cell. In some embodiments, the cell comprises a primary T cell. In some embodiments, the primary T cell comprises a γδ T cell, a T helper cell, a memory T cell, a natural killer T cell, an effector T cell, or any combination thereof. In some embodiments, the primary immune cell comprises a CD3+ cell. In some embodiments, the cell comprises a stem cell. In some embodiments, the stem cell is selected from: an embryonic stem cell, a hematopoietic stem cell, an epidermal stem cell, an epithelial stem cell, a bronchoalveolar stem cell, a mammary stem cell, a mesenchymal stem cell, an intestinal stem cell, an endothelial stem cell, a neural stem cell, an olfactory adult stem cell, a neural crest stem cell, a testicular cell, and any combination thereof. In some embodiments, the stem cell comprises an induced pluripotent stem cell.

In some embodiments, the cell is derived from an animal, such as a vertebrate or an invertebrate, preferably a mammal, further preferably a human. In some embodiments, the cell is an immune cell, and preferably a T cell.

In some embodiments, the introduction comprises transfecting the cell with aid of electroporation, microinjection, calcium phosphate precipitation, a cationic polymer, a dendrimer, a liposome, a lipid nanoparticle, particulate bombardment, fugene, direct acoustic loading, cell extrusion, optical transfection, protoplasmic fusion, impalefection, magneto-transfection, nuclear transfection, virus-mediated transformation or any combination thereof.

In some embodiments, the introduction comprises electroporation of the cell.

In some embodiments, the introduction comprises contacting the cell with mRNA for encoding the transposase and a plasmid comprising the transposon, preferably, the mRNA is used in a dosage of 1-30 µg per 1 × 10⁷ cell, and the plasmid is used in a dosage of 0.1-5 µg per 1 × 10⁷ cell. Most preferably, the mRNA is used at a dosage of 1-5 µg per 1 × 10⁷ cell, and the plasmid is used at a dosage of a concentration of 0.1-2 µg per 1 × 10⁷ cell.

In a further aspect, the present disclosure provides a transgenic animal or a transgenic cell produced using a transposase of any embodiment herein, a polynucleotide of any embodiment herein, a transposon of any embodiment herein, or a transposon system of any embodiment herein. A transgene expression element comprises a gene expression frame and a transposon TIR sequence element at two sides.

In a further aspect, the present disclosure provides a kit, comprising: a transposase of any embodiment herein, a polynucleotide of any embodiment herein, a transposon of any embodiment herein, or a transposon system of any embodiment herein, or a cell of any embodiment herein.

In a further aspect, the present disclosure provides a pharmaceutical composition, comprising a pharmaceutically acceptable excipient and a cell prepared by a method of any embodiment herein.

In a further aspect, the present disclosure provides application of a transposase of any embodiment herein, a polynucleotide of any embodiment herein, a transposon of any embodiment herein, a transposon system of any embodiment herein, a cell of any embodiment herein, or a cell of any embodiment herein, which is selected from any one of (1)-(6) below:
(1) application in preparation of a drug or a reagent for integrating a target gene expression frame into a host cell genome;
(2) application in preparation of a tool for integrating the target gene expression frame into the host cell genome;
(3) application in preparation of a transgenic animal and a transgenic cell;
(4) application in preparation of a drug or a formulation for genomic research, gene therapy, cell therapy, or stem cell induction and post-induction differentiation
(5) application in preparation of a tool for the genomic research, the gene therapy, the cell therapy, or the stem cell induction and the post-induction differentiation; and
(6) application in preparation of a kit, an engineered immune cell, or the pharmaceutical composition.

In a further aspect, the present disclosure provides a therapeutic method, including: (a) generating a genetically modified cell by introducing into a cell a transposon as described herein and a transposase as described herein that recognizes the transposon; and (b) administering the genetically modified cell to a patient in need of a therapy. In some embodiments, the genetically modified cell comprises a transgene introduced by the transposon. In some embodiments, the patient has been diagnosed with a cancer or tumor. In some embodiments, the administering comprises infusing the genetically modified cell into a blood vessel of the patient.

Beneficial effects resulting from the technical solution of the present invention:
1) The wild-type PS transposase is engineered and modified by means of the mutation and/or the fusion of a protein peptide to finally obtain the JL transposase, which significantly improves the integration efficiency of the transposition gene and is of great value for a transgenic industrial application.
2) The engineered JL transposase in mRNA form is applied to cell therapy and transgenesis, which reduces the expression time of the transposase in the cell and further improves safety.
3) A DNA functional sequence structure of the transposon is optimized by means of the mutation of a TIR sequence, the multi-copy tandem of the TIR sequence, and the multi-copy tandem of a TA cleavage site sequence, which further significantly improves the JL transposase-mediated transposon gene integration efficiency.
4) Minivectors with DNA backbone sequences ≤600 bp and reduced or eliminated CpG DNA motifs are used as transposon expression vectors, thereby further enhancing JL transposase-mediated gene transposition efficiency.

### BRIEF DESCRIPTION OF THE DRAWINGS

FIG. 1 is a schematic structural diagram and a three-dimensional structural diagram of a PS transposase;
FIG. 2 is a schematic structural diagram of a JL transposase of an embodiment of the present invention (a schematic structural diagram of fusion JL transposases comprising a leucine zipper structural domain and a c-myc NLS structural domain);
FIG. 3 is a schematic structural diagram of a leucine zipper dimer;
FIG. 4 is a schematic structural diagram of a JL transposon comprising a 2-fold TIR sequence and a 2-fold cleavage site sequence;
FIG. 5 is a graph of an expression plasmid of a wild-type PS transposase;
FIG. 6 is a schematic diagram of a PPS-DT-GFP(ta) plasmid;
FIG. 7 is a schematic structural diagram of a fusion functional polypeptide of a PS transposase;
FIG. 8 is a fluorescent photograph of a fusion functional polypeptide for improving the transposition efficiency of a PS transposase;
FIG. 9 is a schematic diagram of an n-fold sequence repeat multicopy design conducted for a transposon TIR element DNA sequence and a TA shear recognition sequence at a flank thereof in a plasmid expression vector PPS-DT-GFP(ta);
FIG. 10 is a fluorescent photograph of a CHO cell in Example 2;
FIG. 11 is a cell test result of PBMC in Example 2, with a positive rate on the left and a fluorescence expression intensity on the right;
FIG. 12 is statistics of a plurality of positively charged amino acid mutants (three replicates) with elevated integration efficiency (a positive rate) relative to a wild-type PS transposase;
FIG. 13 is EGFP expression at Day 14 in Example 4;
FIG. 14 is a positive rate and an eGFP expression level of a mutant and a control group in Example 4;
FIG. 15 is a schematic structural diagram of a plasmid structure of pPG-PGK-NEO (PSTIR0) in Example 5;
FIG. 16 is a screening and evaluation result of a TIR DNA motif mutation;
FIG. 17 is a screening and evaluation result of the multi-copy tandem of a TIR DNA motif mutation;
FIG. 18 is an evaluation result of Example 6, with a positive rate of the integration of transposons in different groups at the upper, and the level of eGFP expression amount of the integration of the transposons at the lower;
FIG. 19 is an evaluation result of Example 7, with each subfigure showing a CAR positive rate, an expression level, a total cell number, a total number of CAR-positive cells, and a change in the total number of the CAR-positive cells, respectively;
FIG. 20 is a schematic diagram of a pCpGfree MCS-0637 no-load miniplasmid with a corresponding nucleotide sequence shown as SEQ ID NO: 59; and
FIG. 21 is a graph of a pCpGfree MCS-43009 no-load miniplasmid with a corresponding nucleotide sequence shown as SEQ ID NO: 60.

### DETAILED DESCRIPTION OF EMBODIMENTS

Because a wild-type PS transposase or a PS transposon system suffers the disadvantage of low transposition activity so as not to achieve a desired optimal result in a real-world application scenario of transgenesis or cell therapy, the present invention improves the overall activity of the PS transposon system by optimizing the transposase and/or a transposon through an engineering method. The engineered PS transposase, the engineered PS transposon, and the engineered PS transposon system are referred to a JL transposase, a JL transposon, and a JL transposon system in the present invention, respectively.

Delivery of a transgene via a DNA transposon has a plurality of advantages compared to viral transduction of an immune cell (e.g., a T-lymphocyte): ease of use, the potential for the delivery of a large gene fragment, a high speed in realizing clinical application, a low production cost, the stable expression of the transgene over a long period of time and at a high level, and significantly less mutagenicity, non-oncogenicity, and reversibility as compared to retroviruses. Ex vivo genetic modification of a non-transformed primary human T lymphocyte conducted by a gene transfer delivery system based on a non-viral vector is extremely difficult to achieve and requires a high level of transposition efficiency to be achieved. The currently reported mature cases are only limited to a Sleeping beauty transposon, a *PiggyBac* transposon, and a TcBuster transposon. The present invention improves the efficiency of a JL transposase-mediated transposition so that the JL transposase-mediated transposition is suitable for clinical application in cellular immunotherapy.

Prior patent application CN110257425B (the contents of which are incorporated herein by reference in their entirety) demonstrates that a PS transposon is an effective non-viral tool for integrative insertion of a transgene into a cellular chromosome, but the transposition efficiency of the PS transposon system is still unsatisfactory for a cellular or gene therapy. Further, there is a safety risk associated with a method of transfecting and expressing a transposase enzyme with a DNA. Using the DNA encoding the transposase results in prolonged and sustained expression of a transposase protein in a target cell. The lack of control over the timing and kinetics of transposase exposure poses a risk of sustained and uncontrolled retransposition, which raises safety concerns regarding an unfavorable translation of a therapeutic cellular product. To ensure transposase's clearance and avoid the input of an aberrant or unstable cell product, an engineered T cell in an ongoing trial is cultured for 2-4 weeks after the delivery of a CAR gene, which reduces cellular fitness and therapeutic efficacy. Therefore, there is an urgent need to improve the control and safety of the transposase/transposon system, which is a key requirement for cell and gene therapy in general. To control this risk of transposase exposure, some embodiments of the present invention use a method of engineering and modifying a highly active JL transposase based on mRNA expression, which shortens the time of protein expression and reduces cytotoxicity in a cell such as an immune cell, a hematopoietic stem cell, and a progenitor cell (HSPC).

The DNA transposon can be transposed by a non-replicative "cleavage and paste" mechanism, which requires the recognition of two ITRs by a catalytic enzyme, namely a transposase. The catalytic enzyme can cleave a target thereof to release the DNA transposon from a donor template thereof. Upon cleavage, the DNA transposon can subsequently be integrated into an acceptor DNA that is cleaved by the same transposase. In some natural configurations thereof, the DNA transposon is flanked by two ITRs and may comprise a gene for encoding a transposase that catalyzes the transposition.

Genome editing application based on the DNA transposon comprises a binary system comprising a transposase assembly and a transposon assembly. The transposase assembly is a transposase enzyme or a coding nucleic acid (mRNA or DNA) thereof. The transposon assembly is a transposon DNA comprising an ITR at a flank is a terminal, and a target gene for insertion into a host genome. Co-delivery of the transposon assembly and the transposase assembly into the target cell achieves the effect of transposition integration depending on a cleavage-and-paste mechanism.

Discussed herein are various devices, systems, and methods related to a synergistic method for engineering a highly active JL transposase and applied to cellular gene integration, particularly enhancing gene transfer into a cell of a hematopoietic system and/or an immune system of a human. The present disclosure relates to an improved delivery method for engineering a highly active JL transposase, a transposon system, a transposon vector sequence, a transposase, a transposon, and the like. In one embodiment, the present study identifies a specific mutation site for preparing the highly active JL transposase. In another embodiment, described herein is a highly active JL transposase of a fusion polypeptide. An example of the fusion polypeptide comprises a leucine zipper polypeptide, and/or a nuclear localization signal structural domain. In another embodiment, described herein is an improved method for using a chemically modified in vitro transcribed mRNA to express a highly active JL transposase. In another embodiment, described herein is an improved method for optimizing a transposon TIR DNA element to conduct mutation and/or multicopy tandem, and/or the multicopy tandem of a TA element at a TIR flanking shear site. In another embodiment, described herein is a method for delivering a JL transposon vector by utilizing a DNA microvector. A DNA backbone sequence of the DNA microvector does not comprise an expression frame of an antibiotic, is preferably limited in a length of 600 bp or less, has a reduced DNA motif and/or a CpG-free DNA motif. The reduction of the size of the expression vector further enhances the JL transposase-mediated gene transposition efficiency. The reduced DNA motif and/or the CpG-free DNA motif can reduce DNA vector-promoted cellular immunotoxicity and improve transposition efficiency. The above embodiments can be used individually or in combination to improve the efficiency of transposon's gene transfer to various target cells.

### JL Transposase

One aspect of the present disclosure provides a JL transposase, comprising an amino acid mutation and/or fusing a polypeptide as compared to a PS transposase.

In some embodiments, the JL transposase is a mutation transposase that comprises amino acid mutations compared to the PS transposase. In some embodiments, the JL transposase may comprise one or more amino acid substitutions compared to a wild-type PS transposase (SEQ ID NO: 1). The JL transposase may comprise an amino acid sequence having at least 70% sequence identity with the full-length sequence of the wild-type PS transposase (SEQ ID NO: 1). In some embodiments, the JL transposase may comprise an amino acid sequence having at least 40%, at least 50%, at least 60%, at least 70%, at least 80%, at least 90%, at least 95%, at least 96%, at least 97%, at least 98%, or at least 99% sequence identity with the full-length sequence of the wild-type PS transposase (SEQ ID NO: 1). In some embodiments, one or more amino acid substitutions of the JL transposase are mutated to positively charged amino acids, such as histidine, lysine, or arginine, as compared to SEQ ID NO: 1.

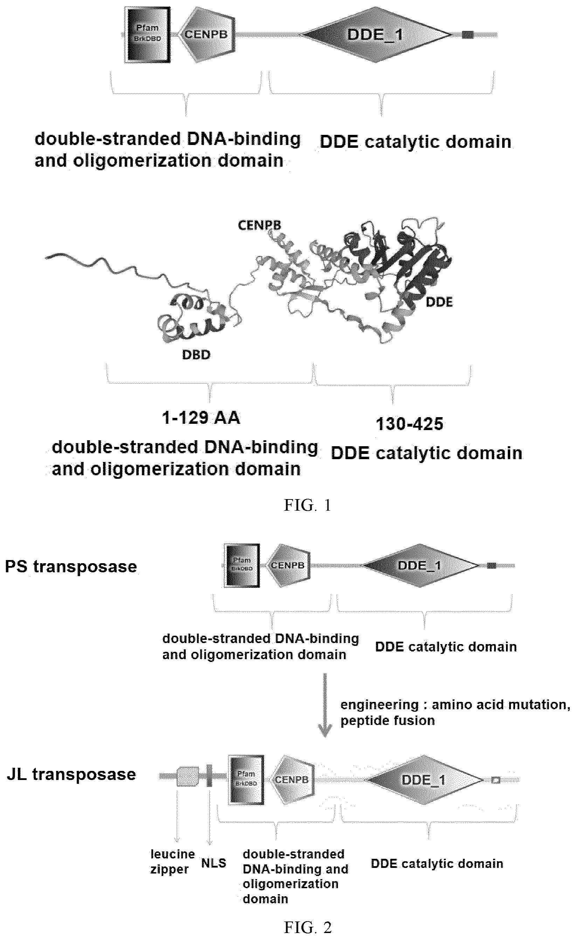

The JL transposase may comprise an amino acid sequence having at least one amino acid that is different from the full-length sequence of the wild-type PS transposase (SEQ ID NO: 1). In some embodiments, the JL transposase may comprise an amino acid sequence having at least 2, at least 3, at least 4, at least 5, at least 6, at least 7, at least 8, at least 9, at least 10, or more amino acids that are different from the full-length sequence of the wild-type PS transposase (SEQ ID NO: 1). The JL transposase can comprise one or more amino acid substitutions in any of a double-stranded DNA-binding oligomerization domain (two HTH structures, and amino acids 1-129), a DDE catalytic structural domain, and an inter-domain region between structural domains of the PS transposase, or any combination thereof (as shown in FIG. 1). A CENPB-type HTH structural domain is a DNA-binding, helix-turn-helix (HTH) structural domain of approximately 70-75 amino acids that is present in a eukaryotic filament protein and the transposase. The structural domain is named after a major mitotic autoantigen B or a mitotic protein B (CENP-B) of a mammal. The mitotic protein B (CENP-B) is a basic mitotic component of a chromosome. A N-terminal of the CENP-B comprises two DNA-binding HTH structural domains that bind to an adjacent DNA major groove. The structure of the CENPB-type HTH structural domain consists of three α-helices. Helices 2 and 3, which are connected by a turn, comprise a helix-turn-helix pattern. The helix 3 is referred to as a recognition helix because the helix 3 binds to the DNA major groove in other HTHs. Preferably, the JL transposase can be substituted at the double-stranded DNA binding oligomerization domain of the PS transposase as a positively charged amino acid, such as histidine (H), lysine (K), or arginine (R). In some embodiments, the JL transposase may comprise one or more amino acid substitutions in the double-stranded DNA binding domain oligomerization domain, the DDE catalytic structural domain, the inter-domain regions between structural domains, or a combination thereof.

In some embodiments, the JL transposase, compared to the full-length sequence of SEQ ID NO: 1, comprises an amino acid sequence having mutations at one or more of the following positions: 7, 8, 16, 20, 22, 24, 32, 35, 42, 45, 46, 47, 51, 55, 57, 58, 59, 69, 73, 74, 76, 79, 84, 95, 98, 123, 129, 136, 159, 165, 178, 187, 193, 199, 213, 215, 228, wherein the position numbers are those of SEQ ID NO: 1. In some embodiments, the one or more amino acids are mutated to histidine, lysine, or arginine.

In some embodiments, the JL transposase, compared to the full-length sequence of SEQ ID NO: 1, comprises an amino acid sequence having mutations at one or more of the following positions: 8, 16, 22, 24, 32, 35, 45, 46, 47, 51, 55, 57, 58, 59, 69, 73, 84, 95, 98, 123, 129, 136, 150, 159, 165, 187, 193, 199, 213, 215, 228, 237, 284, 335, 359, 368, wherein the position numbers are those of SEQ ID NO: 1. In some embodiments, the one or more amino acids are mutated to histidine, lysine, or arginine.

In some embodiments, the JL transposase of the present invention is a JL transposase having any one or any combination of the following mutations: TQS57-59KKA, T129R, T129K, I98K, TQ57-58RK (i.e., containing both T57R and Q58K) , TQ57-58RK\T129K (i.e., containing T57R, Q58K and T129K) , TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V, as compared to the full-length sequence of SEQ ID NO: 1. A numerical value denotes the position of the mutation. The letters before and after the numerical value denote amino acid residues before and after the mutation.

In some embodiments, the JL transposase of the present invention is a JL transposase having any one or any combination of the following mutations: H7K, H7R, A8S, K16R, H20Y, Q22K, Q22E, H24R, E32K, I35V, K42E, K45R, Y46Q, E47K, R51K, K55R, TQ57-58RK (T57R\Q58K) , TQS57-59KKA (T57K\Q58K\S59A) , Q69E, Q69R, K73R, K73E, V74L, Q76E, A79I, A84L, M95L, I98K, R123H, T129K, T129R, T129Q, Q136K, K159H, H165D, I178M, T187K, N193S, N199H, N213D, H215Q, H215E, L228M, as compared to the full-length sequence of SEQ ID NO: 1.

In some embodiments, the JL transposase of the present invention is a JL transposase having any one or any combination of the following mutations: A8S, K16R, Q22K, H24R, E32K, I35V, K45R, Y46Q, K55R, TQ57-58RK, TQS57-59KKA, Q69E, K73R, A84L, M95L, I98K, R123H, T129K, T129R, Q136K, K159H, H165D, T187K, or H215Q, as compared to the full-length sequence of SEQ ID NO: 1.

In some embodiments, the JL transposase of the present invention has substitution mutations at positions 57-58 as compared to SEQ ID NO: 1. In some embodiments, in addition to the substitution mutations at the positions 57-58, the JL transposase of the present invention further has a substitution mutation at one or more positions selected from: 8, 16, 22, 24, 32, 35, 45, 46, 47, 51, 55, 59, 69, 73, 84, 95, 98, 123, 129, 136, 150, 159, 165, 187, 193, 199, 213, 215, 228, 237, 284, 335, 359, 368. The position number is the position number of SEQ ID NO: 1. Preferably, the substitution mutation at position 57 is R or K, and the substitution mutation at position 58 is R or K. Further preferably, the JL transposase of the present invention also has one or more substitution mutations selected from: T129R, T129K, I98K, E32K, R123H, Q136K, K16R, E47K, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V.

In some embodiments, the JL transposase of the present invention has substitution mutations at least at positions 57-59 as compared to SEQ ID NO: 1. In some embodiments, in addition to the substitution mutation at the positions 57-59, the JL transposase of the present invention has a substitution mutation at one or more positions selected from: 8, 16, 22, 24, 32, 35, 45, 46, 47, 51, 55, 69, 73, 84, 95, 98, 123, 129, 136, 150, 159, 165, 187, 193, 199, 213, 215, 228, 237, 284, 335, 359, 368. The position number is the position number of SEQ ID NO: 1. Preferably, the substitution mutations at the positions 57-59 are KKA. Further preferably, the JL transposase of the present invention also has one or more substitution mutations selected from: T129R, T129K, I98K, E32K, R123H, Q136K, K16R, E47K, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, 1284L, K45R, H215E, H215Q, I237V.

In some embodiments, the JL transposase of the present invention has substitution mutations at positions 57- 58 and 129 as compared to SEQ ID NO: 1. In some embodiments, in addition to the substitution mutations at the positions 57- 58 and 129, the JL transposase of the present invention has a substitution mutation at one or more positions selected from: 8, 16, 22, 24, 32, 35, 45, 46, 47, 51, 55, 59, 69, 73, 84, 95, 98, 123, 136, 150, 159, 165, 187, 193, 199, 213, 215, 228, 237, 284, 335, 359, 368. The position number is the position number of SEQ ID NO: 1. Preferably, the substitution mutation at the position 57 is R or K. The substitution mutation at the position 58 is R or K. The substitution mutation at the position 129 is K or R. Further, the JL transposase of the present invention also has one or more substitution mutations selected from: I98K, E32K, R123H, Q136K, K16R, E47K, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V.

In some embodiments, the JL transposase of the present invention has a substitution mutation at least at position 98 as compared to SEQ ID NO: 1. In some embodiments, in addition to the substitution mutation at the position 98, the JL transposase of the present invention has a substitution mutation at one or more positions selected from: 8, 16, 22, 24, 32, 35, 45, 46, 47, 51, 55, 57, 58, 59, 69, 73, 84, 95, 123, 129, 136, 150, 159, 165, 187, 193, 199, 213, 215, 228, 237, 284, 335, 359, 368. The position number is the position number of SEQ ID NO: 1. Preferably, the substitution mutation at the position 98 is K. Further, the JL transposase of the present invention also has one or more substitution mutations selected from: TQS57-59KKA, T129R, T129K, TQ57-58RK, TQ57-58RK\T129K, TQ57-58RK\T129R, E32K, E32K\T129K, E32K\T129R, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V.

In some embodiments, the JL transposase of the present invention has a substitution mutation at least at position 129 as compared to SEQ ID NO: 1. In some embodiments, in addition to the substitution mutation at the position 129, the JL transposase of the present invention has a substitution mutation at one or more positions selected from: 8, 16, 22, 24, 32, 35, 45, 46, 47, 51, 55, 57, 58, 59, 69, 73, 84, 95, 98, 123, 136, 150, 159, 165, 187, 193, 199, 213, 215, 228, 237, 284, 335, 359, 368. The position number is the position number of SEQ ID NO: 1. Preferably, the substitution mutation at the position 129 is K or R. Further, the JL transposase of the present invention also has one or more substitution mutations selected from: TQS57-59KKA, I98K, TQ57-58RK, E32K, TQ57-58RK\I98K, TQ57-58RK\I98K\T129K, TQS57-59KKA\I98K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V.

An exemplary JL transposase may comprise one or more amino acid substitutions from Table 1. Sometimes, the JL transposase may comprise at least one amino acid substitution from Table 1. The JL transposase may comprise at least 2, at least 3, at least 4, at least 5, at least 6, at least 7, at least 8, at least 9, at least 10, at least 20, at least 30 or more amino acid substitutions from Table 1. Preferably, the JL transposase comprises one or more amino acid substitutions or substitution combinations selected from: H24R, E32K, TQS57-59KKA, TQ57-58RK, I98K, T129K, T187K, N193S, TQ57-58RK\T129K, TQ57-58RK\I98K, TQ57-58RK\I98K\T129K, TQS57-59KKA\T129K, TQS57-59KKA\I98K TQS57-59KKA\I98K\T129K, E32K\T57R\Q58R or E32K\T129K.

The inventors have also found that the PS transposase or a mutant thereof can be fused to other functional polypeptide or protein structural domains without affecting but even enhancing the gene integration function of the transposase or the transposon. Thus, the JL transposase of some embodiments of the present invention is a fusion transposase, comprising a PS transposase or a mutant thereof, and a functional polypeptide linked to the PS transposase or the mutant thereof.

The mutant of the PS transposase in the fusion transposase is not limited in the present invention. The present inventors have found that in the case of a fused functional polypeptide (in particular, a leucine zipper structural domain as described below), a higher gene integration function than a wild-type PS transposase can be obtained regardless of which mutation of the PS transposase is.

As used herein, "a mutant" of a polypeptide sequence includes an amino acid sequence having an insertion, a substitution and/or an absence of the one or more amino acid residue as compared to a reference sequence. In some embodiments, "the mutant" as described herein includes the mutant having at least 80%, at least 85%, at least 90%, at least 95%, at least 96%, at least 97%, at least 98%, or at least 99% sequence identity with a sequence for comparison. Sequence identity between two compared sequences may be calculated using, for example, BLASTp of NCBI. Alternatively, in some embodiments, the mutant described herein has the insertion, the substitution, or the absence of the one or more (e.g., within 20, within 15, within 10, within 8, within 5, or within 3, e.g., 1-20, 1-10, etc.) amino acid residues, as compared to the sequence for comparison.

In some preferable embodiments, the mutant of the PS transposase in the fusion transposase has 1-5 (e.g., 1, 2, 3, 4, 5) amino acid mutations such as substitutions compared to the PS transposase. In some preferable embodiments, compared to the wild-type PS transposase as shown in SEQ ID NO: 1, the mutant of the PS transposase in the fusion transposase comprises the one or more mutations selected from the group consisting of: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V, Q69R, L100I, K96R, Q162R, A271K, R372K, H20Y, TYCR150-153KFVI, N232K, A363N, A377C, Q346M, A17V, N105G, Q76E, I354V, VS133-134IA, S253K, V370I, SV297-298TE, EH286-286DS, A352S, ANS241-243VHE, S297T, A79I, T147S, I178M, I276V, Q331E, R257E, T339S, A79V, H104L, V74L, S107E, Q142E, R375K, S243E, R195K, Q22E, A328Q, G111A, R110K, 1365V, VTE380-382SNA, R190K, RA372-373KS, K73L, K121R, R102K, C152V, IY276-277VW, V292I, K73E, H104M, 1239V, K42E, K356Q, T150K, G316S, IY259-260VW, V53M, E108D, V53L, DKV72-74EEL, M280F, S85G, N63K, V91L, K251L, H124N, K308S, N10D, H7R, G112S, H7K, TA327-328KE, preferably, comprises the one or more mutations selected from the group consisting of: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V, Q69R, L100I, K96R, Q162R, A271K, R372K, H20Y, TYCR150-153KFVI, N232K, A363N, A377C, Q346M, A17V, N105G, Q76E, I354V, VS133-134IA, S253K, V370I, SV297-298TE, EH286-286DS, A352S, ANS241-243VHE, S297T, A79I, T147S, I178M, I276V, Q331E, R257E, T339S, A79V, H104L, V74L, S107E, Q142E, R375K, S243E, R195K, Q22E, A328Q, G111A, R110K, 1365V, VTE380-382SNA, more preferably, comprises the one or more mutations selected from the group consisting of: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, 135V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V.

In preferable embodiments, the mutant of the PS transposase is a mutant transposase as described in any embodiment herein.

"Functional polypeptide" as used herein refers to a polypeptide having its own function, such as a DNA sequence-specific or non-specific binding domain and/or a nuclear localization signal (NLS) domain. The term 'DNA sequence-specific or sequence-nonspecific binding domain' refers to a structural domain that functions based on DNA sequence specificity or non-specificity, for example a leucine zipper structural domain, a CRISPR/Cas structural domain, a TALE domain, a zinc finger domain, an AAV Rep DNA-binding domain, or any combination thereof. The nuclear localization signal domain comprises SV40 NLS, C-myc NLS, TAF1 NLS, TP53 NLS, STAT3 NLS or any combination thereof. The amino acid sequences of these NLS domains can be found in Chinese patent application CN202211150935.9 and the like.

In some embodiments, a leucine zipper structural domain is derived from a portion of a structural domain that mediates homodimerization in Basic_leucine-zipper_C protein family of IPR020983, bZIP protein family of IPR004827, and bZIP_sf protein family of IPR046347 in InterPro Protein Database (https://www.ebi.ac.uk/interpro/ ). In some embodiments, the leucine zipper structural domain is selected from a group consisting of C/EBPα (which is also referred to C-EBPO or C/EBPO herein, the amino acid sequence of which is shown in SEQ ID NO:28, and the nucleotide sequence for encoding C/EBPα is shown in SEQ ID NO: 34), LZIP (the amino acid sequence of which is shown in SEQ ID NO: 29, and the nucleotide sequence for encoding LZIP is shown in SEQ ID NO: 35), CREPT (the amino acid sequence of which is shown in SEQ ID NO: 30, and the nucleotide sequence for encoding CREPT is shown in SEQ ID NO: 36), and TEF (the amino acid sequence of which is shown in SEQ ID NO: 31, and the nucleotide sequence for encoding TEF is shown in SEQ ID NO: 37).

In some embodiments, the DNA sequence-specific or non-specific binding domain can be positioned at a C-terminal or a N-terminal of the transposase. In some embodiments, the NLS domain can be positioned at the C-terminal or the N-terminal, preferably the N-terminal, of the transposase. In some embodiments, the DNA sequence-specific or non-specific binding domain and the NLS 1 domain can be positioned at the same terminal or different terminals of the transposase. When positioned at the same terminal of the transposase, it can be the DNA sequence-specific or non-specific binding domain-NLS domain, or the NLS domain-DNA sequence-specific or non-specific binding domain from the N-terminal to the C-terminal. When positioned at the different terminals of the transposase, the DNA sequence-specific or non-specific binding domain and the NLS domain may be positioned at the N-terminal and the C-terminal, or the C-terminal and the N-terminal, respectively, of the transposase.

In one exemplary structure, as shown in FIG. 2, when a C-myc NLS structural domain is present, the N-terminal-C-terminal are the leucine zipper structural domain, the C-myc NLS structural domain, and the transposase, respectively,. By adding the leucine zipper structural domain, homodimerization may be mediated, thereby improving the transposition efficiency.

In the fusion transposase, the transposase, the DNA sequence-specific or non-specific binding domain, and the NLS domain may be directly connected or separated by a linker. Typically, the linker comprises at least 1, at least 2, at least 3, at least 4, at least 5, at least 6, at least 7, at least 8, at least 9, at least 10, at least 15, at least 20 or at least 50 amino acids. Exemplarily, the sequence of the linker is (GGGS)ₙ or A(EAAAK)ₙA, wherein n is a positive integer greater than 0, such as 1, 2, 3, 4 or 5. Preferably, the sequence of the linker is GGGS.

The sequence of the transposase described herein may be a modified polypeptide sequence. A form of modification (which typically does not alter a primary structure) includes a chemically derived form of a polypeptide in vivo or in vitro such as acetylation or carboxylation. The modification also includes glycosylation, such as those polypeptides produced from the glycosylation modification in the synthesis and processing or further processing steps of the polypeptide. The modification may be accomplished by exposing the polypeptide to an enzyme that performs glycosylation (e.g., a glycosylase or deglycosylase of a mammal). The form of the modification also includes a sequence having a phosphorylated amino acid residue (e.g., phosphotyrosine, phosphoserine, and phosphothreonine). The form of the modification also includes a polypeptide that is modified to improve the polypeptide's resistance to protein hydrolysis or to optimize a solubility property.

It should be understood that in a gene cloning operation, it is often necessary to design a suitable enzyme cleavage site, which inevitably introduces one or more extraneous residues at the terminal of the expressed amino acid sequence, which however does not affect the activity of a target sequence. To construct a fusion protein, facilitate the expression of a recombinant protein, obtain the recombinant protein that is automatically secreted outside a host cell, or facilitate the purification of the recombinant protein, it is often necessary to add the plurality of amino acids to a N-terminal and a C-terminal of the recombinant protein or other suitable regions within the recombinant protein, for example, including, but not limited to, a suitable splice peptide, a signal peptide, a leader peptide, a terminal extension, and the like. Accordingly, an amino-terminal or a carboxy-terminal of the transposase of the present invention may also comprise one or more peptide fragments that serve as protein tags. Any suitable tag may be used herein. For example, the tags may be FLAG, HA, HA1, c-Myc, Poly-His, Poly-Arg, Strep-TagII, AU1, EE, T7, and 4A6. These tags can be used to purify the protein.

The JL transposase of the embodiment of the present invention has a higher activity than the wild-type PS transposase. As used herein, "the higher activity" may refer to having an increased transposition efficiency compared to the wild-type PS transposase having amino acid sequence SEQ ID NO: 1.

The transposition efficiency can be measured based on a percentage of successful transposition events that occur in a population of the host cells. The percentage is normalized by the amounts of the transposon and the transposase introduced into the population of the host cells. In many instances, when comparing the transposition efficiency of the two or more transposases, a construct of the same transposon is paired with each of the two or more transposases in response to the transfection of the host cells under the same or similar transfection conditions. The amount of the transposition events in the host cells can be examined by various methods. For example, the construct of the transposon can be designed to comprise a reporter gene positioned between ITRs. Further, a transfected cell that is positive for that reporter gene can be counted as a cell in which a successful transposition event occurs, which can yield an estimate value of the amount of the transposition events. In some embodiments, when comparing the transposition efficiency of the two or more different transposons, the same transposase can be paired with each kind of the different transposons in response to the transfection of the host cells under the same or similar transfection conditions. Similar methods can be used to measure the transposition efficiency. Other methods known to a person skilled in the art can also be implemented to compare the transposition efficiencies.

### Polynucleotide Molecule Encoding JL Transposase

The present invention provides a polynucleotide molecule which encodes a JL transposase described herein. The present invention also provides a complementary sequence of a coding sequence of a transposase. The polynucleotide can be a recombinant nucleic acid molecule or can be synthetic. The polynucleotide may comprise DNA, RNA, and PNA (a peptide nucleic acid) and can be a heterozygote thereof. The DNA may be single-stranded or double-stranded. The DNA may be a coding strand or a non-coding strand. The present invention also includes a degenerate variant of a polynucleotide sequence for encoding a JL transposase, i.e., a nucleotide sequence for encoding the same amino acid sequence but being different.

In some embodiments, the polynucleotide molecule comprises DNA for encoding the JL transposase. In some embodiments, the polynucleotide is present in a nucleic acid construct, e.g., a DNA vector. The vector may be a cloning vector or an expression vector. The expression vector comprises an expression frame of the transposase, which is a nucleic acid construct, i.e., comprising a promoter, a coding sequence of the transposase and a PolyA tailing signal sequence. The expression vector typically also comprises other elements typically comprised in the vector, such as a polyclonal site, a resistance gene, a replication initiation site, and the like. The nucleic acid construct may also comprise other elements required for expression, including, but not limited to, an enhancer and the like. The DNA vector includes a conventional cyclic DNA plasmid and a conventional linear DNA plasmid, and also includes a microcyclic plasmid, a nanoplasmid, a doggybone linear miniplasmid, and other forms of DNA that do not comprise an antibiotic and/or a replicon DNA sequence. In some embodiments, the polynucleotide is present in a viral vector, including, but not limited to, an AAV, a lentivirus, a retrovirus, an adenovirus, and the like. In some embodiments, the DNA vector is a DNA microvector. A DNA backbone sequence of the DNA minivector does not comprise an expression frame of the antibiotic, is preferably limited in a length of 600 bp or less, and/or does not comprise a CpG DNA motif.

In some embodiments, the polynucleotide comprises a messenger RNA (mRNA) for encoding the JL transposase. In some embodiments, the mRNA is chemically modified, e.g., pseudouridine (ψ), N1-methylpseudouridine (m1ψ), 5-methylcytosine nucleoside (m5C), 5-methoxyuridine (5moU), and the like.

The present invention also relates to a polynucleotide which hybridizes to the above polynucleotide sequence, and has at least 50%, preferably at least 70%, better preferably at least 80% identity between the two sequences. The present invention particularly relates to a polynucleotide which is hybridizable to the polynucleotide described herein under stringent conditions. In the present invention, "the stringent conditions" means: (1) hybridization and elution at a lower ionic strength and a higher temperature, e.g., 0.2 x SSC, 0.1% SDS, 60°C; or (2) hybridization in the presence of a denaturant, e.g., 50% (v/v) formamide, 0.1% calf serum/0.1% Ficoll, 42°C, etc.; or (3) hybridization occurs only when the identity between the two sequences is at least 90%, preferably 95% or more. Moreover, the polypeptide encoded by the hybridizable polynucleotide has the same biological function and activity as the mature polypeptide.

The polynucleotide molecule may be prepared and obtained using a conventional method in the art, and the corresponding vector may be constructed, usually by a PCR amplification method, a recombination method or a synthetic method. One feasible method is to synthesize a relevant sequence by the synthetic method, especially when a fragment length is short. Typically, a fragment with a very long sequence can be obtained by first synthesizing a plurality of small fragments and then linking these small fragments. In addition, a coding sequence of a heavy chain and an expression tag (e.g., 6His) can be fused together to form a fusion protein. Alternatively, sequences of parts of a fusion transposase can be obtained as above and then linked to obtain a full length of CAR.

Once obtained, the relevant sequence can be obtained in a large quantity by the recombination method. The sequence is usually cloned into the vector and then transferred into a cell. The relevant sequence is then isolated from the proliferated host cell by a conventional method. A recombinant vector may be constructed using a method well known to a person skilled in the art. Please see, for example, techniques described in Sambrook, et al, Ausubel (1989) or other standard textbooks. The vector comprising a nucleic acid molecule of the present invention may be transferred to a host cell by a well-known method, which varies depending on the type of cell host. For example, calcium chloride transfection is commonly used for prokaryotic cells, while calcium phosphate treatment or electrotransformation can be used for other cell hosts. Please see Sambrook *et al.*

In addition, a DNA sequence for encoding a protein (or a fragment thereof, or a derivative thereof) of the present invention can be obtained entirely by chemical synthesis. The DNA sequence can then be introduced into various existing DNA molecules (or e.g., a vector) and cells known in the art. In addition, a mutation may be introduced into a protein sequence of the present invention by chemical synthesis.

The present invention also relates to a nucleic acid construct, comprising nucleic acid sequences of the polynucleotide molecule described herein, and one or more regulatory sequences operatively linked to the sequences. The nucleic acid construct described herein can be operated in a variety of ways to ensure the expression of a transposase. The nucleic acid construct may be operated prior to insertion into the vector on the basis of the difference in the expression vector or requirements. The regulatory sequence and an operation method that are required to express a protein via DNA or mRNA are known in the art. A technique for altering a polynucleotide sequence using a recombinant DNA method is known in the art.

In certain embodiments, the nucleic acid construct is a vector, such as a cloning vector, an expression vector, or an integration vector. The expression of the polynucleotide sequence of the present invention is typically achieved by operatively linking the polynucleotide sequence of the present invention to the expression vector. A typical cloning vector comprises a transcriptional and translational terminator, an initiation sequence, and a promoter that may be used to regulate the expression of the desired nucleic acid sequence. The integration vector comprises a component that integrates a target sequence onto a cellular genome. These vectors can be used to transform an appropriate host cell to enable the host cell to express a protein. The vector typically comprises a sequence for plasmid maintenance and for cloning and expression of an exogenous nucleotide sequence. The sequence (it is always referred to as "a flanking sequence" in some embodiments) typically include one or more of the following nucleotide sequences: a promoter, one or more enhancer sequences, an origin of replication, a transcription termination sequence, a fully intronic sequence comprising a donor and an acceptor splice site, a sequence for encoding a leading sequence for polypeptide secretion, a ribosome binding site, a polyadenylation sequence, a multi-linker region for inserting into a nucleic acid for encoding an antibody to be expressed, and an optional labeling element.

The type of the vector is not limited, e.g., a plasmid, a phage, a phage derivative, an animal virus, and a cosmid, and may be varied according to the host cell to be introduced. A technique of a viral vector is well known in the art and is described in, for example, Sambrook et al. (2001, Molecular Cloning: A Laboratory Manual, Cold Spring Harbor Laboratory, New York) and other virology and molecular biology manuals. The virus that can be used as the vector includes, but is not limited to, a retrovirus, an adenovirus, an adeno-associated virus, a herpesvirus, and a lentivirus.

In order to assess the expression of the transposase, the vector that is introduced into the cell may also include either or both of a selectable marker gene or a reporter gene to facilitate identification and selection of a expressing cell from a population of the cells seeking to be transfected or infected via a viral vector.

### JL Transposon

A JL transposon of the present disclosure includes any one, two, or three of the following features as compared to a wild-type PS transposon: (1) a nucleotide mutation of at least one ITR (i.e., a mutation-type terminal reverse repeat sequence); (2) having a repetitive ITR at least one end; and (3) comprising a repetitive TA sequence outside the ITR at least one end.

In some embodiments, the mutation ITR has 1-5, preferably 1-4, more preferably 1-3 nucleotide mutations compared to SEQ ID NO: 2 (a wild-type PS transposon 5' TIR) or SEQ ID NO: 3 (a wild-type PS transposon 3' TIR); and/or is a sequence has at least 80%, at least 85%, at least 90%, at least 95%, at least 96%, at least 97%, at least 98%, or at least 99% identity with SEQ ID NO: 2 or 3. In some embodiments, the mutation ITR is selected from, but not limited to, sequences as shown in any one of SEQ ID NOs: 4-27 or comprises any combination mutation (it is referred to as a combination mutation sequence) of nucleotide mutations of SEQ ID NOs: 4-27 compared to the wild-type PS TIR, or is SEQ ID NOs: 4-27 or a reverse complementary sequence of a combined mutation sequence. Preferably, the mutation ITR is selected from SEQ ID NOs: 4-6 and a reverse complementary sequence thereof.

It should be understood that the ITRs at two ends of the transposon can be one of the ITRs having a nucleotide mutation, or a plurality of or all of the ITRs having the nucleotide mutation, and the nucleotide mutations of the respective ITRs can be the same or different.

In some embodiments, the ITRs of the JL transposon are each independently selected from SEQ ID NOs: 3-27 or the sequences having at least 50%, at least 60%, at least 70%, at least 80%, at least 90%, at least 95%, at least 98%, or at least 99% identity with SEQ ID NOs: 3-27, or reverse complementary sequences thereof.

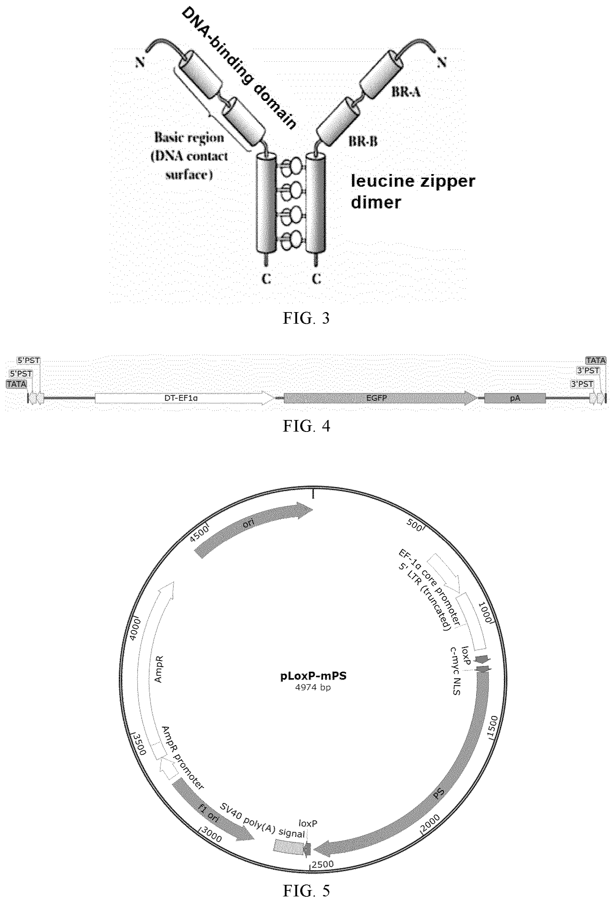

In one or more embodiments, the DNA sequence of the transposon has a multiplicity of the ITRs (i.e., multiple copies of the ITRs in tandem) at one end or both ends, which may be expressed as (TIR)n, n ≥ 2, e.g., as 2, 3, 4, and the like. The multiplicity includes, but is not limited to, 2-fold, 3-fold, or 4-fold, etc. The number of copies of the ITRs at both ends can be the same or different. For example, each end independently has 1, 2, 3, 4, or more repetitive ITRs. Both ends are not one ITR at the same time. The repetitive ITR may or may not comprise the nucleotide mutation described above compared to the wild-type ITR. In some embodiments, the transposon DNA comprises 2-fold ITRs at both ends, which is structurally schematically shown in FIG. 4. 5'PST refers to a TIR at a 5' terminal. 3'PST refers to a TIR at a 3' terminal, 5' PST5'PST and 3'PST3'PST refer to having the 2-fold ITRs. The ITR of the JL transposon is relatively short. By increasing the number of the ITRs and extending the length of the sequence recognizable by the JL transposase, transposition efficiency can be improved.

In one or more embodiments, the ITRs at both ends of the DNA sequence of the transposon have a cleavage site sequence for the transposase. The cleavage site sequence is a TA (the nucleotide sequence). The ITR may or may not comprise the nucleotide mutation described above compared to the wild-type ITR. In one or more embodiments, one or both of the ends of the transposon DNA has a multicopy cleavage site sequence expressed as (TA)m, m ≥ 2. The multicopy value m is preferably 2, 3, or 4. The number of TA repeats at two ends can be the same or different, e.g., having 1, 2, 3, 4 or more repetitive TA sequences at each end independently of each other. Both ends are not one TA sequence at the same time. In some embodiments, the transposon DNA comprises a 2-fold cleavage site sequence at two ends, which is structurally schematically shown in FIG. 4. TA refers to the cleavage site sequence, and TATA refers to the 2-fold cleavage site sequence. In some embodiments, the transposon DNA comprises a 4-fold cleavage site sequence, that is, TATATATA. By increasing the number of the cleavage site sequences and the number of cleavage sites of JL transposase, transposition efficiency can be improved.

In one or more embodiments, the JL transposon comprises (TA)ₘ(TIR)ₙ at the left end and (TIR)ₚ(TA)_{q} at the right end, wherein m, n, p, and q are integers, m ≥ 1, preferably 2-4; n ≥ 1, preferably 2-4; p ≥ 1, preferably 2-4; q ≥ 1, preferably 2-4. The TIR can be a wild-type PS transposon TIR or a mutation TIR. When the TIR is a wild-type TIR, m, n, p, and q are not all 1 at the same time (i.e., at least one of m, n, p, and q is ≥ 2). In addition, when the TIR has repeats, each of the repeats can each be independently selected from any sequence of a wild-type TIR sequence and a mutant thereof. In a preferable embodiment, m=2-4, preferably m=4; n=2; p=2-4, preferably p=4, and q=2. In a more preferable embodiment, the TIR is a mutation TIR, m=2-4, preferably m=4, and n=2.

In one or more embodiments, the DNA sequence of the JL transposon preferably comprises (TA)₂(TIR1)₂, (TA)₃(TIR1)₂, or (TA)₄(TIR1)₂, wherein the TIR1 is a mutation TIR, and most preferably in the form of (TA)₄(TIR1)₂, with a nucleotide sequence as shown in SEQ ID NO: 32. In one or more embodiments, the DNA sequence of the JL transposon comprises (TA)₄(TIR1)₂ at one or both ends.

The JL transposon may comprise a combination of DNA elements, such as a target gene expression frame, positioned between the ITRs. The combination of the DNA elements includes, but is not limited to, a promoter, an enhancer, a target gene (an expression gene), 5-UTR, 3-UTR, and other sequence elements known to a person skilled in the art. As used herein throughout, the term "exogenous gene" or "target gene" refers to a nucleic acid or a protein sequence not naturally associated with the target gene or the host cell that is a subject of introduction, comprising a naturally occurring nucleic acid sequence positioned at a non-naturally occurring genomic location, or a plurality of non-naturally occurring copies of a naturally occurring nucleic acid (e.g., a DNA sequence).

In some embodiments, the combination of the DNA elements comprises an expression gene. Accordingly, the combination of the DNA elements may further comprise a promoter selected from, but not limited to, CMV, EFS, MND, EF1α, CAGC, PGK, UBC, U6, H1, and Cumate. In some embodiments, the expression gene encodes a protein selected from a cellular receptor, an immune checkpoint protein, a cytokine, and any combination thereof. In some embodiments, the expression gene encodes a protein selected from the cellular receptor, the immune checkpoint protein, the cytokine, a T cell receptor (TCR), a B cell receptor (BCR), a chimeric antigen receptor, and any combination thereof.

In some embodiments, the expression gene encodes CAR. The CAR may sequentially comprise a binding antigen binding domain, a hinge region, a transmembrane region, and an intracellular signal region. The hinge region, the transmembrane region, and the intracellular signal region that are known in the art for constructing the CAR may be used to construct the CAR of the present invention. Typically, the antigen binding domain is capable of binding, with moderate affinity, a membrane antigen widely expressed by a tumor cell. The polypeptide is typically inserted with an antigenic epitope at a position selected from any 1, 2 or 3 of the following 3 positions: at a N-terminal of the polypeptide, between the polypeptide and the hinge region, and within the polypeptide. The antigen binding domain may be a natural polypeptide or a synthetic polypeptide. The chimeric antigen receptor may target one or more of the following antigens: CD19, CD20, CEA, GD2, FR, PSMA, PMEL, CA9, CD171/L1-CAM, IL-13RL1, MART-1, ERBB2, NY-ESO-1, AFP, MUC1, CD22, CD23, CD30, CD33, CD44v7/8, CD70, VEGFR1, VEGFR2, IL-11R/, EGP-2, EGP-40, FBP, GD3, PSCA, FSA, PSA, HMGA2, LeY, EpCAM, MSLN, IGFR1, EGFR, EGFRvIII, ERBB3, ERBB4, CA125, CA15-3, CA19-9, CA72-4, CA242, CA50, CYFRA21-1, SCC, AFU, EBV-VCA, POA and PROGRP.

In some embodiments, the antigen binding domain may comprise an antibody, an antibody mimetic, a protein scaffold, or a fragment thereof. In some embodiments, the antibody is a chimeric antibody, a recombinant antibody, a humanized antibody, or a human antibody. In certain embodiments, the antibody is affinity-tuned. A non-limiting example of the antibody of the present invention includes a single chain variable fragment (scFv), VHH, a single domain antibody (sdAB), a small modular immunopharmaceutical (SMIP) molecule, or a nanobody. In certain embodiments, the VHH is of the Camelidae family. Alternatively or additionally, in certain embodiments, the VHH is humanized. A non-limiting example of an antibody fragment of the present invention includes a complementarity region, a variable region, a heavy chain, a light chain, or any combination thereof. A non-limiting example of an antibody mimic of the present invention includes: an affibody, an Afflilin molecule, an affimer, an Affitin molecule, an alphabody, an anticalin, and an Avimer molecule, DARPin, Fynomer, a Kunitz structural domain peptide, or a monobody. A non-limiting example of the protein scaffold of the present invention includes Centyrin.

In some embodiments, the CAR suitable for the present invention can be referenced in CN 202111681582.0, which is incorporated herein by reference in its entirety.

In some embodiments, the expression gene encodes an immune checkpoint inhibitor, e.g., a PD-1 antibody, a CTLA-4 antibody, and the like. A non-limiting example of the antibody includes the single chain variable fragment (scFv), the VHH, the single domain antibody (sdAB), the small modular immunopharmaceutical (SMIP) molecule, or the nanobody.

In some embodiments, the PD-1 antibody suitable for the present invention can be referenced in CN 202111681582.0, which is incorporated herein by reference in its entirety.

In some embodiments, the expression gene encodes the CAR and the PD-1 antibody as described in CN 202111681582.0.

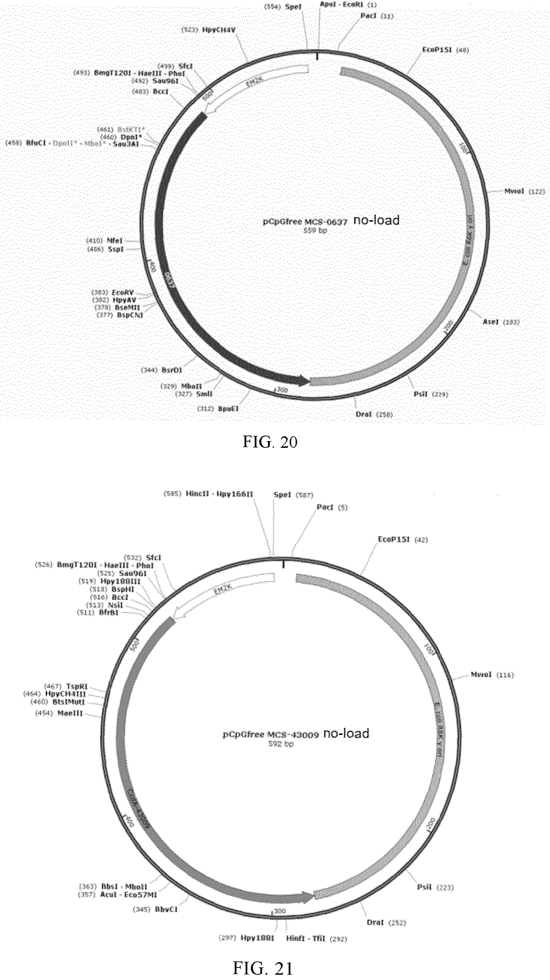

The transposon is typically provided in the form of a nucleic acid construct, in particular a DNA vector. In this regard, any DNA vector in the art that facilitates the introduction of the transposon into the cell may be used, including, but not limited to, a conventional circular DNA plasmid, a linear DNA plasmid, a microcyclic plasmid, a nanoplasmid, Doggybone, and other forms of DNA that does not include an antibiotic or/and a replicon DNA sequence, and the like. In some embodiments, the DNA vector is a DNA minivector. A DNA backbone sequence of the DNA minivector does not comprise an expression frame of the antibiotic, is preferably limited in a length of 600 bp or less, and/or does not comprise a CpG DNA motif. In some embodiments, the DNA vector is an antibiotic resistance gene-free miniplasmid, i.e., a miniplasmid free of an antibiotic resistance gene (an antibiotic expression frame-free miniplasmid), which is also referred to as a tiniplasmid (a tiny). For an antibiotic resistance gene-free miniplasmid suitable for the present invention, reference is made to the Chinese patent application entitled "ANTIBIOTIC RESISTANCE GENE-FREE MINIPLASMID AND PREPARATION METHOD THEREFOR AND USE THEREOF(Application No. 202310072956.1) filed by MAXIRNA (SHANGHAI) PHARMACEUTICAL CO., LTD. and the like on January 13, 2023, which is incorporated herein by reference in its entirety. In some embodiments, the antibiotic resistance gene-free miniplasmid comprises a nucleotide sequence encoding an antitoxin protein and a replicon; the amino acid sequence of the antitoxin protein includes the following: (1) the amino acid sequence shown in SEQ ID NO: 49, or an amino acid sequence having one or more mutations of E24D, I35V, V43I compared to SEQ ID NO: 49; or (2) the amino acid sequence shown in SEQ ID NO: 50, or an amino acid sequence having one or more mutations of T6I, T43A, K47E, A50S, E51D, G52A, N54K compared to SEQ ID NO: 50; the length of the replicon is ≤800 bp, preferably ≤600 bp or ≤ 300 bp. In some embodiments, the amino acid sequence of the antitoxin protein is as shown in any one of SEQ ID NOs: 49-55. In some embodiments, the replicon is selected from ColE1, ColE2, pMB1, pSC101, RSF, R6K, pUC57, RK2, and p15A; preferably R6K or pUC57. In some embodiments, the length of the plasmid backbone of the antibiotic resistance gene-free miniplasmid is ≤1000 bp, preferably ≤900 bp, ≤800 bp, or ≤ 600 bp. In some embodiments, the nucleotide sequence encoding the antitoxin protein does not contain CpG motifs; preferably, the nucleotide sequence encoding the antitoxin protein is as shown in SEQ ID NO: 56 or SEQ ID NO: 57. In some embodiments, the nucleotide sequence of the replicon does not contain CpG motifs. In a preferred embodiment, the length of the backbone sequence of the antibiotic resistance gene-free miniplasmid is ≤600 bp, and the replicon is an R6K replicon without CpG motifs. The nucleotide sequence of the R6K replicon without CpG motifs is as shown in SEQ ID NO: 58. In some embodiments, the nucleotide sequence of the antibiotic resistance gene-free miniplasmid (empty vector) is as shown in SEQ ID NO: 59 or 60; the map structure is as shown in FIG. 20 or 21.

### Transposon System

The present invention also relates to a genome editing system (a transposon system), comprising a transposase and a transposon recognizable by the transposase. In some embodiments, the present invention provides a transposon system, comprising a JL transposase or a polynucleotide for encoding the JL transposase, and a transposon recognizable by the transposase. In some embodiments, the present invention provides a transposon system, comprising a JL transposon and a transposase recognizable by the transposon or a polynucleotide for encoding the transposase. In some embodiments, the transposon system provided is a JL transposon system, comprising (1) the JL transposase as described herein, or a polynucleotide for encoding the JL transposase, and (2) the JL transposon as described herein or a wild-type PS transposon.

In some embodiments, the ITRs of the transposon of the transposon system are each independently selected from SEQ ID NOs: 2-27 or the sequences having at least 50%, at least 60%, at least 70%, at least 80%, at least 90%, at least 95%, at least 98%, or at least 99% identity with SEQ ID NOs: 2-27, or reverse complementary sequences thereof.

In some embodiments, the transposon can be present in an expression vector. In many situations, the expression vector can be a DNA plasmid, including, but not limited to, a conventional circular DNA plasmid, a linear DNA plasmid, a microcyclic plasmid, a nanoplasmid, Doggybone, and other forms of DNA that does not include an antibiotic or/and a replicon DNA sequence. Sometimes, the expression vector may be a minicircle vector. As used herein, the term "minicircle vector" may refer to a minicyclic plasmid derivative that does not comprise most, if not all, of a prokaryotic vector portion (e.g., a control sequence or a non-functional sequence of a prokaryotic origin). In some embodiments, cellular toxicity by transfection or electroporation can be mitigated by the use of "a minicircle" as described herein. In some embodiments, the expression vector is a DNA minivector. A DNA backbone sequence of the DNA minivector does not comprise an expression frame of the antibiotic, is preferably limited in a length of 600 bp or less, and/or does not comprise a CpG DNA motif. In some embodiments, the expression vector is an antibiotic resistance gene-free miniplasmid of any of the above embodiments.

In some embodiments, the polynucleotide for encoding the transposase of the transposon system may be DNA, which may be present in the expression vector and may be present in the same expression vector or in different expression vectors as the transposon. The expression vector may be as described in any embodiment herein.

In some embodiments, the polynucleotide for encoding the transposase of the transposon system may be a messenger RNA (mRNA). The mRNA may be generated by many methods known to a person skilled in the art, such as, but not limited to, in vivo transcription and RNA purification, in vitro transcription, and de novo synthesis. In many cases, the mRNA can be chemically modified. The chemically modified mRNA can be resistant to degradation or can be degraded more rapidly than an unmodified or natural mRNA. In many cases, the chemical modification of the mRNA may allow the mRNA to be translated with greater efficiency. The chemical modification of the mRNA can be performed using well-known techniques available to a person skilled in the art or by a commercial vendor.

The JL transposase, the JL transposon, or the JL transposon system of the present disclosure provides a significant increase in integration efficiency and transgene expression amount over a wild-type PS transposase, a PS transposon, or a PS transposon system. The JL transposase of the present disclosure can be applied in the forms of a virus, a plasmid DNA, mRNA, or a protein for stabilizing a transfected cell, cell line development, genome modification, gene therapy, cellular therapy, a transgenic animal, and the like.

### Host Cell

Herein, when expressing an exogenous nucleic acid sequence, "host cell" refers to a prokaryotic cell or a eukaryotic cell that is capable of replicating a vector and/or expressing an exogenous gene encoded by the vector. The host cell may be used as a recipient of the vector or mRNA. A host cell can be "transfected" or "transformed", which refers to a process of transfection or transduction of an exogenous nucleic acid into the host cell. The transformed cell includes a primary target cell and a progeny thereof. The terms "engineered" and "recombinant" cell or host cell as used herein often refer to a cell into which an exogenous nucleic acid sequence, such as the vector or the mRNA, has been introduced. Thus, the recombinant cell may be distinguished from a naturally occurring cell that does not comprise an introduced recombinant nucleic acid.

As used herein, the host cell comprises a cell that carries and/or produces the transposase or a coding sequence thereof, the transposon or the transposon system described herein. Specifically, the present invention provides a cell carrying the transposase and/or the coding sequence thereof described herein. The cell may also comprise the nucleic acid sequence for encoding the transposon recognizable by the transposase.

The type of the cell is not limited as long as the cell can express the transposase described herein or can utilize the transposase described herein to achieve the transposition of the transposon. In some embodiments, the cell is a primary cell isolated from a subject. The subject is a healthy subject or has been diagnosed with a disease (e.g., a cancer or a tumor).

In some embodiments, the cell is isolated from the blood of the subject, e.g., PBMC or a derivative cell thereof. The cell may include a primary immune cell, e.g., a primary white blood cell. In some embodiments, the cell includes a primary T cell. The primary T cell includes a γδ T cell, a T helper cell, a memory T cell, a natural killer T cell, an effector T cell, or any combination thereof. In some embodiments, the primary immune cell includes a CD3+ cell.

In some embodiments, the cell includes a stem cell. The stem cell is selected from: an embryonic stem cell, a hematopoietic stem cell, an epidermal stem cell, an epithelial stem cell, a bronchoalveolar stem cell, a mammary stem cell, a mesenchymal stem cell, an intestinal stem cell, an endothelial stem cell, a neural stem cell, an olfactory adult stem cell, a neural crest stem cell, a testicular cell, and any combination thereof. The stem cell includes an induced pluripotent stem cell.

The transposase or the coding sequence thereof, the transposon, or the transposon system of the present invention may be introduced into a cell of interest. An introducing method, as used herein, includes transfecting the cell with the aid of electroporation, microinjection, calcium phosphate precipitation, a cationic polymer, a dendrimer, a liposome, a lipid nanoparticle (LNP), a particulate bombardment, a fugene, direct acoustic loading, cell extrusion, optical transfection, protoplasmic fusion, impalefection, magneto-transfection, nucleo-transfection, or any combination thereof. In some embodiments, the introduction employs electroporation.

An obtained transformant can be cultured by a conventional method to express the JL transposase of the present invention. On the basis of the host cell used, a medium used in culture may be selected from a variety of conventional media. The medium is cultured under conditions suitable for the growth of the host cell. In an embodiment where an inducible promoter is used to express the transposase, after the host cell has been grown to an appropriate cell density, the selected promoter is induced by a suitable method (e.g., temperature shift or chemical induction), and the cell is cultured for an additional period of time.

The polypeptide in the above method may be expressed inside the cell, or on a cell membrane, or secreted outside the cell. If desired, the recombinant protein can be isolated and purified by various separation methods using physical, chemical and other properties thereof. These methods are well known to a person skilled in the art. Examples of these methods include, but are not limited to: a conventional repletion treatment, treatment with a protein precipitant (a salting-out method), centrifugation, osmotic bacterial breakage, ultra-treatment, ultra-centrifugation, molecular sieve chromatography (gel filtration), adsorption chromatography, ion-exchange chromatography, high-performance liquid chromatography (HPLC), and a variety of other liquid chromatography techniques and combinations of these methods.

### Method

The present invention further relates to a method of preparing a cell, including: the step of introducing the JL transposon system as described herein into a cell.

In some embodiments, the introduction includes contacting the cell with a polynucleotide for encoding the JL transposase. The polynucleotide includes DNA or a messenger RNA (mRNA) for encoding the JL transposase.

In some embodiments, the introduction includes contacting the cell with the JL transposase, preferably by providing a transposase protein by adding the JL transposase directly to the medium comprising the cell (preferably to the cell medium of the target biological cell). In the direct contact of the JL transposase according to the present invention with a target cell, no reagent, vector, or method which alters the penetration of the protein across a cell membrane can be used.

In some embodiments, the introduction includes contacting the cell with a DNA vector comprising the transposon. In some embodiments, the DNA vector includes a minivector plasmid. Preferably, a DNA backbone sequence of the DNA minivector does not comprise an expression frame of the antibiotic, is preferably limited in a length of 600 bp or less, and/or does not comprise a CpG DNA motif. In some embodiments, the introduction includes contacting the cell with a plasmid vector including the transposon and a polynucleotide for encoding the JL transposase. In some embodiments, the introduction includes contacting the cell with mRNA for encoding the transposase and the plasmid vector comprising the transposon. In some embodiments, the introduction includes contacting the cell with a plasmid vector comprising the polynucleotide and the transposon. In some embodiments, the introduction includes contacting the cell with the plasmid vector comprising the polynucleotide and the plasmid vector comprising the transposon.

In some embodiments, the transposon system described herein can be delivered to the cell via a virus, including a retrovirus (e.g., a lentivirus, etc.), an adenovirus, an adeno-associated virus (AAV), a herpes virus, and the like. A transposon gene and a transposase gene can be included together on the same recombinant viral genome. A single infection can deliver both parts of the transposon system so that the expression of the transposase can direct cleavage of the transposon from the recombinant viral genome and be subsequently inserted into a cell chromosome. In another example, the transposase and the transposon can be delivered separately by a combination of viral and/or non-viral systems (e.g., a lipid-including reagent). In these cases, the transposon gene and/or transposase gene can be delivered by a recombinant virus. In each case, the expressed transposase gene directs the transposon to be liberated from a vector DNA (a viral genome) thereof and inserted into a chromosomal DNA. The JL transposase of the present invention is small and particularly suitable for delivery with AAV.

The present invention also provides a method for genetically engineering a cell using a transposase. The method involves causing the transposase to penetrate a cell membrane but does not include a protein transfection step. In particular, the method does not include the use of a protein transfection reagent or procedure for introducing a transposase protein into a cell. The method includes a procedure of directly contacting the transposase with the cell to be receptive to the transposase, e.g., incubating the cell with a cell medium comprising the transposase. The method of the present invention includes the step of introducing a transposase protein without the use of any vector, reagent or method that alters the penetration of the protein across a cell membrane.

The term "protein transfection" in the context of the present invention is to be understood as broadly relating to any method or reagent that is sufficient to introduce into a target cell a protein that is unable to enter the target cell efficiently. A popular protein transfection system and reagent include a commercial protein transfection reagent such as PULSin^{™}, ProteoJuice^{™}, Xfect^{™} and BioPorter^{®}, a Pierce^{™} protein transfection reagent (ThermoFisher), TransPass^{™}, protein electroporation and other methods.

Also provided herein are a transgenic animal, and a transgenic cell obtained by the above methods.

Also provided herein is a method of treatment, including: (a) introducing a transposon system into a cell, thereby generating a genetically modified cell comprising a transgene introduced by a transposon, and (b) administering the genetically modified cell to a patient in need of treatment. In some embodiments, the patient has been diagnosed with a cancer or tumor. In some embodiments, the administering includes infusing the genetically modified cell into a blood vessel of the patient.

The present invention also relates to use of a JL transposase, a coding sequence (DNA or RNA) thereof or a nucleic acid construct thereof, a cell, or a genome editing system described herein in the preparation of a product. The product is, for example, a gene editing kit, an engineered immune cell, or a pharmaceutical composition. The immune cell includes a γδ T cell, a helper T cell, a memory T cell, a natural killer T cell, an effector T cell, and the like.

The scope of the present invention also encompasses a kit comprising a JL transposase, a coding sequence (DNA or RNA) thereof or a nucleic acid construct thereof, a cell, or a genome editing system as described herein. The kit further comprises one or more of a coding nucleic acid of a transposon recognizable by the JL transposase or a nucleic acid construct thereof (e.g., a DNA vector comprising the transposon), a host cell, a cell culture medium suitable for the host cell, a cytokine, and a use instruction.

### Pharmaceutical Composition and Administration

A transposase, a nucleic acid molecule, a transposon, a transposon system, and a cell of the present invention may be administered alone or administered as a pharmaceutical composition in combination with a diluent and/or with other components such as an associated cytokine or a cell population. Accordingly, the present invention also provides a pharmaceutical composition comprising a transposase, a nucleic acid construct, a gene editing system or a cell, and a pharmaceutically acceptable excipient prepared by a method described herein.

As used in the present invention, the "pharmaceutically acceptable excipient" is a pharmaceutically or food acceptable vector, solvent, suspension, or excipient for delivering the transposase, the nucleic acid molecule, the transposon, the transposon system, or the cell of the present invention to an animal or a human. The pharmaceutically acceptable excipient herein is non-toxic to a recipient of the composition at the dosage and concentration employed. Various types of vectors or excipients commonly used in the delivery of a protein, a nucleic acid, or a cell in therapeutics known in the art may be included. An exemplary excipient can be a liquid or a solid and includes, but is not limited to: a pH modifier, a surfactant, a carbohydrate, an adjuvant, an antioxidant, a chelating agent, an ionic strength enhancer, a preservative, a vector, a flow aid, a sweetener, a dye/a colorant, a flavor enhancer, a wetting agent, a dispersant, a suspending agent, a stabilizer, an isotonic agent, a solvent, or an emulsifier. In some embodiments, the pharmaceutically acceptable excipient can comprise one or more inactive ingredients, including, but not limited to: a stabilizer, a preservative, an additive, an adjuvant, an aerosol, compressed air or other suitable gases, or other suitable inactive ingredients in combination with a pharmaceutically effective compound. See, e.g., REMINGTON'S PHARMACEUTICAL SCIENCES, 18th Edition, edited by A. R. Genrmo, 1990, Mack Publishing Company. An optimal pharmaceutical composition may be determined depending on an intended route of administration, a mode of delivery, and a desired dosage.

The pharmaceutical composition of the present invention may be selected for parenteral delivery, for inhalation, or for delivery through a GI tract (e.g., orally), e.g., for delivery by intravenous infusion. The preparation of the composition is within the art of the art. Other pharmaceutical compositions are apparent to a person skilled in the art, comprising a formulation comprising an immune cell, particularly the immune cell (e.g., T-cell), in a sustained or controlled release delivery formulation. The pharmaceutical composition of the present invention can also be administered in a manner suitable for a disease to be treated (or prevented).

A pharmaceutical composition for in vivo administration is typically provided in the form of a sterile preparation. Sterilization is achieved by filtration through a sterile filter membrane. A composition for parenteral administration may be stored in a cryopreserved form or in a solution (e.g., a cryopreserved preparation). A parenteral composition is typically placed in a container with sterile entry holes, such as an intravesical solution strip or a vial that has a stopper capable of being pierced by a hypodermic needle.

Once formulated, the pharmaceutical composition is stored in a sterile vial as a solution, a suspension, a gel, an emulsion, a solid, a crystal, a frozen substance, or as in a form of a dehydrated or cryopreserved powder. The pharmaceutical formulation (e.g., a cryopreserved preparation) may be stored in a ready-to-use form or in a form that is further formulated prior to administration. For example, the pharmaceutical composition suitable for delivery as described herein can be a cryopreserved preparation so that the pharmaceutical composition can tolerate long-distance transport without damaging the cell. In addition to the cell itself, the cryopreserved preparation typically comprises a component such as a cell cryopreservative, a human serum albumin (HSA). Prior to administration (e.g., intravenous infusion), the cryopreserved pharmaceutical composition is stored cryogenically (e.g., in a liquid nitrogen). After being thawed, the cryopreserved preparation can be infused into a patient either directly or formulated as an infusion composition. A person skilled in the art knows a component and a concentration of a conventional cryopreserved solution. For example, the cryopreserved solution or the infusion composition may also comprise dimethyl sulfoxide, sodium chloride, glucose, sodium acetate, potassium chloride, magnesium chloride, or the like in a concentration that can be determined by a person skilled in the art (e.g., an experienced physician) based on the condition of a cell, a disease, a patient, and the like.

In some embodiments of the present invention, a genetically modified cell of the present invention or a composition thereof may be combined with other therapies known in the art.

"Patient," "subject", "individual" and the like are used interchangeably herein to refer to a living organism, e.g., a mammal, that may elicit an immune response. An example includes, but is not limited to, a human, a dog, a cat, a mouse, a rat, and transgenic species thereof.

### Application

A subject matter provided herein can be used in a wide range of applications related to genome editing, such as preparation of a genetically modified organism, gene therapy, cellular therapy, and the like.

One non-limiting example relates to generating a genetically modified cell for use in research and clinical applications. For example, as described above, a genetically modified T cell can be prepared using the subject matter provided herein, which can be applied to help people fight a variety of diseases, such as, but not limited to, a cancer and an infectious disease.

One specific example includes generating a genetically modified primary white blood cell using a method provided herein, and administering the genetically modified primary white blood cell to a patient in need. The generation of the genetically modified primary white blood cell can include introducing a transposon system into a leukocyte, thereby generating a genetically modified leukocyte. In many cases, the transposon may comprise a transgene. The transgene may be a cellular receptor, an immune checkpoint protein, a cytokine, an antibody, and any combination thereof. Sometimes, the cellular receptor may include, but is not limited to, a T cell receptor (TCR), a B cell receptor (BCR), a chimeric antigen receptor (CAR), or any combination thereof. In some other embodiments, the transposon system is designed to delete or modify an endogenous gene, such as a cytokine, an immune checkpoint protein, an oncogene, or any combination thereof. Genetic modification of the primary white blood cell may be designed to promote immunity against an infectious pathogen or a cancer cell that causes a patient to be in a diseased state.

Another non-limiting example relates to generating a genetically modified organism for use in agriculture, food production, medicine, and pharmacy. There is a wide range of species that can be genetically modified, including, but not limited to, a plant and an animal. The genetically modified organism, such as a genetically modified crop or a livestock, can be modified in certain aspects of a physiological property thereof. An example in an edible crop includes resistance to certain pests, diseases, or environmental conditions, reduction of spoilage, or resistance to chemical treatments (e.g., resistance to a herbicide), or improved nutrient conditions of the crop. An example in a non-edible crop includes the production of a drug, a biofuel and other industrially useful commodities, and for bioremediation. An example in the livestock includes resistance to certain parasites, production of certain nutrients, increased growth rates, and increased milk production.

Partial embodiments of the present invention.
1. A transposase which is a mutant transposase or a fusion transposase, wherein the mutant transposase comprises an amino acid mutation as compared to a wild-type PS transposase as shown in SEQ ID NO: 1, wherein the amino acid mutation is that any one or more amino acids in a double-stranded DNA binding oligomerization domain of a wild-type PS transposase are mutated to a positively charged amino acid; and/or the amino acid mutation comprises one or more mutations selected from the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V, the fusion transposase is formed by fusing a functional polypeptide onto the wild-type PS transposase or a mutant thereof, and the functional polypeptide is a DNA sequence-specific or non-specific binding domain, and/or a nuclear localization signal domain.
2. The transposase according to item 1, wherein the mutant, compared to the wild-type PS transposase shown in SEQ ID NO: 1, includes one or more mutations selected from the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V, Q69R, L100I, K96R, Q162R, A271K, R372K, H20Y, TYCR150-153KFVI, N232K, A363N, A377C, Q346M, A17V, N105G, Q76E, I354V, VS133-134IA, S253K, V370I, SV297-298TE, EH286-286DS, A352S, ANS241-243VHE, S297T, A79I, T147S, I178M, I276V, Q331E, R257E, T339S, A79V, H104L, V74L, S107E, Q142E, R375K, S243E, R195K, Q22E, A328Q, G111A, R110K, I365V, VTE380-382SNA, R190K, RA372-373KS, K73L, K121R, R102K, C152V, IY276-277VW, V292I, K73E, H104M, I239V, K42E, K356Q, T150K, G316S, IY259-260VW, V53M, E108D, V53L, DKV72-74EEL, M280F, S85G, N63K, V91L, K251L, H124N, K308S, N10D, H7R, G112S, H7K, TA327-328KE.
3. The transposase according to item 1 or 2, wherein the mutant, compared to the wild-type PS transposase shown in SEQ ID NO: 1, includes one or more mutations selected from the group consisting of: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V, Q69R, L100I, K96R, Q162R, A271K, R372K, H20Y, TYCR150-153KFVI, N232K, A363N, A377C, Q346M, A17V, N105G, Q76E, I354V, VS133-134IA, S253K, V370I, SV297-298TE, EH286-286DS, A352S, ANS241-243VHE, S297T, A79I, T147S, I178M, I276V, Q331E, R257E, T339S, A79V, H104L, V74L, S107E, Q142E, R375K, S243E, R195K, Q22E, A328Q, G111A, R110K, 1365V, VTE380-382SNA.
4. The transposase according to any one of items 1-3, wherein the DNA sequence-specific or non-specific binding domain comprises a leucine zipper structural domain, a CRISPR/Cas structural domain, a TALE domain, a zinc finger domain, an AAV Rep DNA-binding domain, or any combination thereof, and preferably, the leucine zipper structural domain has an amino acid sequence as shown in any one of SEQ ID NOs: 28-31, and/or the nuclear localization signal domain comprises SV40 NLS, C-myc NLS, TAF1 NLS, TP53 NLS, STAT3 NLS or any combination thereof, and/or the functional polypeptide comprises or does not comprise a linker for connecting to the wild-type PS transposase or the mutant thereof.
5. A polynucleotide for encoding the transposase of any one of items 1-4, preferably, the polynucleotide is DNA or a messenger RNA.
6. A transposon which is recognizable by the transposase according to any one of items 1-4, wherein the transposon has any one, two or three features selected from (1) to (3) below:
   (1) the transposon has a mutation inverted terminal repeat, the mutation inverted terminal repeat has 1-5 nucleotide mutations compared to a wild-type PS transposon inverted terminal repeat shown in SEQ ID NO: 2 or 3;
   (2) the transposon has more than two inverted terminal repeats at least one end; and
   (3) the transposon has more than two repeats of the TA sequence outside the inverted terminal repeat at least one end.
7. The transposon according to item 6, the mutation inverted terminal repeat is as shown in any one of SEQ ID NOs: 4-27, preferably as shown in any one of SEQ ID NOs: 4-6, or is a nucleotide sequence comprising any combination of nucleotide mutations in SEQ ID NOs: 4-27, or is a reverse complementary sequence thereof.
8. The transposon according to item 6 or 7, wherein the transposon has two inverted terminal repeats at each of two ends and comprises four TA sequence repeats outside the inverted terminal repeats at each of the two ends.
9. The transposon according to any one of items 6-8, wherein the transposon comprises a target gene expression frame positioned between the inverted terminal repeats, preferably, the target gene encodes a protein selected from a cellular receptor, an immune checkpoint protein, a cytokine, a T cell receptor, a B cell receptor, a chimeric antigen receptor, and any combination thereof.
10. A transposon system comprising the transposase according to any one of items 1-4 or the polynucleotide for encoding the transposase according to item 5; and a transposon being a PS transposon or the transposon according to any one of items 6-9.
11. The transposon system according to item 10, wherein the polynucleotide for encoding the transposase is an mRNA, preferably, the mRNA is chemically modified, and comprises but is not limited to pseudouridine (ψ), N1-methylpseudouridine (m1ψ), 5-methylcytosine nucleoside (m5C), 5-methoxyuridine (5moU), etc.
12. The transposon system according to item 10 or 11, wherein the polynucleotide for encoding the transposase and/or the transposon is present in a DNA vector, preferably, the DNA vector is a plasmid vector or a minicircular DNA vector, and is preferably an antibiotic resistance gene-free miniplasmid vector; and/or the DNA vector is a minivector, and a backbone sequence of the minivector is in a length of 600 bp or less, and has a reduced CpG DNA motif and/or a CpG-free DNA motif; or the polynucleotide for encoding the transposase and/or the transposon is present in a viral vector, preferably, the viral vector is selected from an adenovirus vector, an adeno-associated virus vector, a retrovirus vector, a herpes simplex virus vector, or a poxvirus vector.
13. The transposon system according to item 12, wherein the polynucleotide for encoding the transposase and the transposon is present in the same vector or in different vectors.
14. A host cell comprising or capable of producing the transposase according to any one of items 1-4, the polynucleotide according to item 5, the transposon according to any one of items 6-9, or the transposon system according to any one of items 10-13.
15. A method of preparing a cell including: the step of introducing into the cell the transposase according to any one of items 1-4, the polynucleotide according to item 5, the transposon according to any one of items 6-9, or the transposon system according to any one of items 10-13,
   preferably, the introduction includes contacting the cell with the polynucleotide, and/or
   the introduction includes contacting the cell with a DNA vector comprising the transposon, and/or
   the introduction includes contacting the cell with mRNA for encoding the transposase and a plasmid vector comprising the transposon, and/or
   the introduction includes contacting the cell with a plasmid vector comprising the polynucleotide and the transposon, and/or
   the introduction includes contacting the cell with the plasmid vector comprising the polynucleotide and the plasmid vector comprising the transposon, and/or
   the introduction includes contacting the cell with the transposase.
16. The method according to item 15, wherein the cell is derived from an animal, such as a vertebrate or an invertebrate, preferably, a mammal, and further preferably a human; and/or the cell is an immune cell, and preferably, a T cell.
17. The method according to item 15 or 16, wherein the introduction includes transfecting the cell with aid of electroporation, microinjection, calcium phosphate precipitation, a cationic polymer, a dendrimer, a liposome, a lipid nanoparticle, particulate bombardment, fugene, direct acoustic loading, cell extrusion, optical transfection, protoplasmic fusion, impalefection, magneto-transfection, nuclear transfection, virus-mediated transformation or any combination thereof.
18. The method according to item 16 or 17, wherein the introduction includes electroporation of the cell.
19. The method according to item 18, wherein the introduction includes contacting the cell with the mRNA for encoding the transposase and a plasmid comprising the transposon, preferably, the plasmid is a minivector, a backbone sequence of the minivector is in a length of 600 bp or less, and has a reduced CpG DNA motif and/or a CpG-free DNA motif, and/or the plasmid is an antibiotic resistance gene-free miniplasmid.
20. A kit comprising the transposase according to any one of items 1-4, the polynucleotide according to item 5, the transposon according to any one of items 6-9, the transposon system according to any one of items 10-13, or the cell according to item 14.
21. A pharmaceutical composition comprising a pharmaceutically acceptable excipient and a cell prepared by the method according to any one of items 15-19.
22. Application of the transposase according to any one of items 1-4, the polynucleotide according to item 5, the transposon according to any one of items 6-9, the transposon system according to any one of items 10-13, the cell according to item 14, or the pharmaceutical composition according to item 21 being selected from any one of (1)-(6) below:
   (1) application in preparation of a drug or a reagent for integrating a target gene expression frame into a host cell genome;
   (2) application in preparation of a tool for integrating the target gene expression frame into the host cell genome;
   (3) application in preparation of a transgenic animal and a transgenic cell;
   (4) application in preparation of a drug or a formulation for genomic research, gene therapy, cell therapy, or stem cell induction and post-induction differentiation
   (5) application in preparation of a tool for the genomic research, the gene therapy, the cell therapy, or the stem cell induction and the post-induction differentiation; and
   (6) application in preparation of a kit, an engineered immune cell, or the pharmaceutical composition.

The present invention is described in further detail by reference to the following experimental embodiments. These embodiments are provided for illustrative purposes only and are not intended to be limiting unless otherwise specified. Accordingly, the present invention should in no way be construed to be limited to the following embodiments, but rather should be construed to include any and all variations that become apparent as a result of the teachings provided herein. A method and a reagent used in the embodiments are conventional in the art unless otherwise indicated.

### Examples

The following method is employed herein.

### (1) Site-directed mutagenesis for JL mutant preparation

Phusion^{®} High-Fidelity DNA Polymerase (New England BioLabs) was used for all site-directed mutagenesis. For a single-site mutation, after rolling circle PCR mutagenesis was performed, digestion was performed using a DpnI restriction endonuclease. 5 µL of the digestion reaction product was taken and transformed in TOP10 *E. coli* competent cells. For a combination mutation, after mutagenesis was performed using multiple PCR reactions, a PCR product was purified using an agarose gel DNA recovery kit (Beijing Tianmo Sci&Tech Development Co., Ltd). 10 µL of the purified PCR product was performed a ligation reaction using the Hieff Clone^{®} Plus Multi One Step Cloning Kit (YEASEN). 5 µL of the ligation reaction product was taken and transformed in the TOP10 *E. coli* competent cells. For each mutant, three single clones were picked for culture, and half of the three single clones were sequenced for identification and bacterial preservation. For the mutant with a correctly identified sequence, a plasmid for transfection was prepared using a NucleoBond Xtra MiDi EF Plasmid Preparation Kit (MACHEREY-NAGEL). The superhelix ratio and endotoxin content of the plasmid sample were sampled and identified.

### (2) Transposition efficiency detection in a CHO cell

On the day of electrotransformation, a CHO-K1 cell was digested with trypsin comprising 0.25% EDTA, collected, centrifuged, and counted by resuspension with 1×DPBS. 1.5-2.5×10⁶ cells were taken. A supernatant was removed after centrifugation. According to the instruction of a Lonza 2B electrotransformation reagent, 100 ul of an electrotransformation solution was taken. After 4 µg of a transposase plasmid and 4 µg of a transposon plasmid were mixed with the electrotransformation reagent, the mixture was added to a cell precipitate after centrifugation, then added to an electrotransformation cup after resuspension and placed into a Lonza 2B electrotransformation instrument. Electrotransformation program was set as H-014. After the electrotransformation, the cell was transferred to a six-well plate with a complete medium. A fluorescence photography was taken on Days D5-D13 after electrotransformation. The transposition efficiency of a transposase in the CHO-K1 cell was detected by flow cytometry.

### (3) Cell transposition efficiency detection of PBMC (a peripheral blood mononuclear cell)

On the day of the electrotransformation, the cryopreserved PBMC were resuscitated and counted. 1×10⁷ cells were taken, and the supernatant was removed after centrifugation. According to the instruction of the Lonza 2B electrotransformation reagent, 100 ul of the electrotransformation solution was taken. After 4 µg of the transposase plasmid and 4 µg of the transposon plasmid were mixed with the electrotransformation reagent, the mixture was added to the cell precipitate after centrifugation, then added to the electrotransformation cup after resuspension and placed into the Lonza 2B electrotransformation instrument. The electrotransformation program was set as U-014. After the electrotransformation, the cells were transferred into a six-well plate with a complete medium (AIM-V+2% FBS) for cultivation and supplemented with 500 U/ml IL-2. A plate was turned on Day 5 after the electrotransformation. The fluorescence photography was performed on Days D5-D13. A number was counted. The flow cytometry for transposition efficiency of the transposase was performed in the PBMC.

A transposon plasmid in examples 1-6 comprises an eGFP (also documented herein as GFP and EGFP) fluorescent protein expression frame.

For fluorescence photographic analysis, GFP fluorescence was photographed with an Olympus reverse fluorescence microscope at Days D5, D9, and D13 with an exposure time of 500 ms and a magnification of 100×. The transposition efficiency of the transposase in the cell was assessed by the number of GFP fluorescent cells and the fluorescence intensity.

For flow cytometry analysis, the stable integration of a gene was assessed by detecting the GFP fluorescence in the cell grown in the absence of drug selection. The transfected cell was harvested at an indicated time point, washed once with 1× DPBS, and then resuspended with 500 ul DPBS and added to a flow tube or a 96-well plate for machine detection. The cell was analyzed using Cytek's SpectroFlo. GFP expression was assessed using a FITC channel.

### (4) Screening of a transposase mutant in a CHO cell

On the day of the electrotransformation, the CHO-K1 cell was digested with trypsin comprising 0.25% EDTA, collected, centrifuged, and counted by resuspension with 1 × DPBS. 1.5-2.5×10⁶ cells were taken. The supernatant was removed after centrifugation. According to the instruction of the Lonza 2B electrotransformation reagent, 100 ul of the electrotransformation solution was taken. After 4 µg of different mutant plasmids of the transposase and 4 µg of the transposon plasmid (e.g., PPS-DT-GFP(ta)) were mixed with the electrotransformation reagent, the mixture was added to the cell precipitate after centrifugation, then added to the electrotransformation cup after resuspension and placed into the Lonza 2B electrotransformation instrument. The electrotransformation program was set as H-014. After the electrotransformation, the cell was transferred to a six-well plate with the complete medium. Fluorescence photography was taken at Days D3-D14 after electrotransformation. The transfected cell was harvested, washed once with 1 × DPBS, and then resuspended with 500 ul DPBS and added to the flow tube or the 96-well plate for machine detection. The cell was analyzed using Cytek's SpectroFlo. GFP expression was assessed using the FITC channel. Different transposase mutants were screened and assessed based on final transposition efficiency.

### (5) Screening of a transposase mutant in a PBMC

On the day of the electrotransformation, the cryopreserved PBMC was resuscitated and counted. 1×10⁷ cells were taken, and the supernatant was removed after centrifugation. According to the instruction of the Lonza 2B electrotransformation reagent, 100 ul of the electrotransformation solution was taken. After 4 µg of the transposase plasmid and 4 µg of the transposon plasmid (e.g., PPS-DT-GFP(ta)) were mixed with the electrotransformation reagent, the mixture was added to the cell precipitate after centrifugation, then added to the electrotransformation cup after resuspension and placed into the Lonza 2B electrotransformation instrument. The electrotransformation program was set as U-014. After the electrotransformation, the cells were transferred into a six-well plate with the complete medium (AIM-V+2% FBS) and supplemented with 500 U/ml IL-2. The plate was transferred on Day 5 after electrotransformation. Fluorescence photography was taken at Days D3-D14. The number was counted. The transfected cell was harvested, washed once with 1 × DPBS, and then resuspended with 500 ul DPBS and added to the flow tube or the 96-well plate for machine detection. The cell was analyzed using Cytek's SpectroFlo. GFP expression was assessed using the FITC channel. Different transposase mutants were screened and assessed based on final transposition efficiency.

### Example 1: Fusing a functional polypeptide to improve the transposition efficiency of a PS transposase

### The objective of an experiment:

When a transposase was to exert a transposition activity, the transposition activity was accompanied by a dimerization process of a protein and a DNA complex. This dimerization efficiency might be reduced due to cumulative mutations in a natural transposase, which in turn affected transposition efficiency. As shown in FIG. 3, a leucine zipper was a structural motif in a DNA-bound protein that often comprises a regular leucine residue. A leucine zipper dimer was formed when the hydrophobic surfaces of two dual-purpose α-helices from the same or different polypeptide chains interacted to form a coil-to-coil dimer structure (Coiled coil region). In this experiment, the fact that a leucine zipper polypeptide also had similar DNA binding and dimerization characteristics was utilized to strengthen the dimerization efficiency of a PS transposase and achieve the objective of improving the transposition efficiency.

### Experimental method:

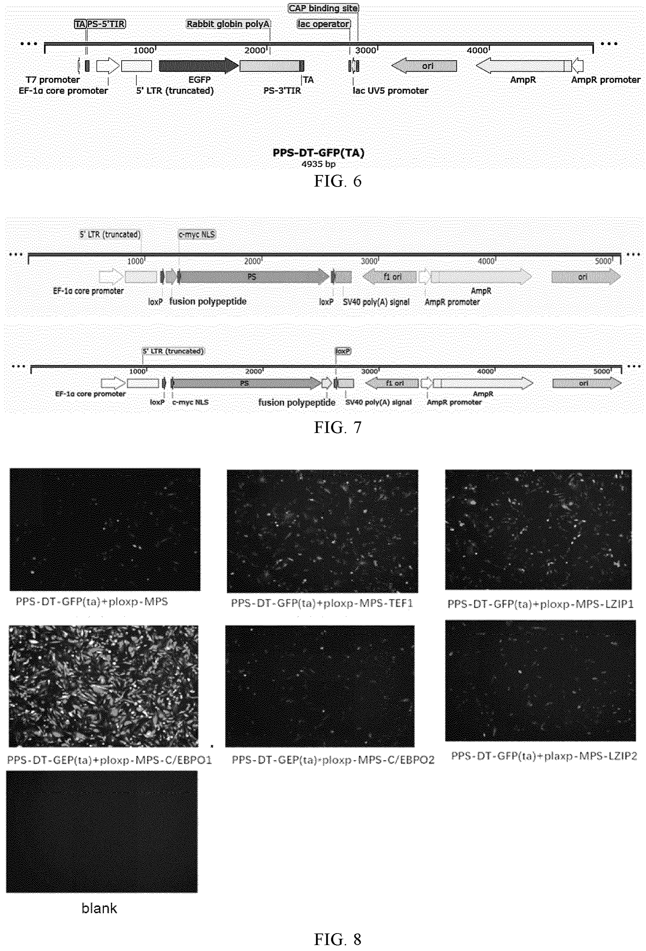

In this example, pLoxP-mPS was a wild-type PS transposase expression plasmid (plasmid mapping was shown in FIG. 5, and a sequence was shown in SEQ ID NO: 33). An amino acid sequence of the wild-type PS transposase was shown in SEQ ID NO: 1. Amino acid sequences of fusion peptides C/EBPO, LZIP, and TEF were shown in SEQ ID NOs: 28, 29, and 31, respectively. Nucleotide sequences were shown in SEQ ID NOs: 34, 35, and 37, respectively. The structure of a transposon plasmid PPS-DT-GFP(ta) was shown in FIG. 6. A nucleotide sequence of the transposon plasmid PPS-DT-GFP(ta) was shown in SEQ ID NO: 38.

Different kinds of leucine zipper structural domains and/or C-myc NLS polypeptides were fused at a N-terminal or a C-terminal on the basis of a natural PS transposase polypeptide in a pLoxP-mPS plasmid, which were structurally schematically shown in FIG. 7. The transposition efficiency of the fusion-type PS transposase was detected according to a detection method of CHO cell transposition efficiency described herein. EGFP expression was assessed by fluorescence photography and flow cytometry to verify the effect of the leucine zipper structural domain on the natural PS transposase.

### Experimental result

The detection results of a fluorescence photo and the flow cytometry were shown in FIG. 8 and Table 1. Ploxp-MPS referred to a transposase plasmid without a leucine zipper structural domain. Ploxp-mps-TEF1, ploxp-mps-LZIP1, and ploxp-mps-C/EBPO1 referred to the leucine zipper structural domain at the N-terminal of the transposase. Ploxp-mps-C/EBPO2 and ploxp-mps-LZIP2 referred to the leucine zipper structural domain at the C-terminal of the transposase. NTC was a control group. Mps in a plasmid name was sometimes written as mPS or MPS. In this specification and drawings, the English letters of some plasmids' designations were not case-sensitive. From the results, compared with 7.17% in the wild-type PS transposase group, it could be seen that the N-terminal was fused with LZIP, TEF, and C/EBPO leucine zipper structural domains, which was able to dramatically increase the transposition efficiency, with the highest increase of the C/EBPO leucine zipper structural domain. The 14-day positive rate of electrotransformation reached 40.01%.

**Table 1 Effect of fusion polypeptide on PS transposition efficiency**

| Name of transposase plasmid | 14-day positive rate (eGFP-positive cell) |
|---|---|
| ploxp-mps | 7.17% |
| ploxp-mps-TEF1 | 13.30% |
| ploxp-mps-LZIP1 | 15.20% |
| ploxp-mps-C/EBPO1 | 40.01% |
| ploxp-mps-C/EBPO2 | 4.40% |
| ploxp-mps-LZIP2 | 4.21% |
| NTC | 0.17% |

### Example 2: Optimization of a transposon TIR element DNA sequence could continue to improve the transposition efficiency of a fusion peptide PS transposase.

The objective of an experiment: Optimization of the TIR element DNA sequence and the repeat multicopy and tandem of a TA shear recognition sequence at a flank thereof continued to improve the transposition efficiency of the PS transposase.

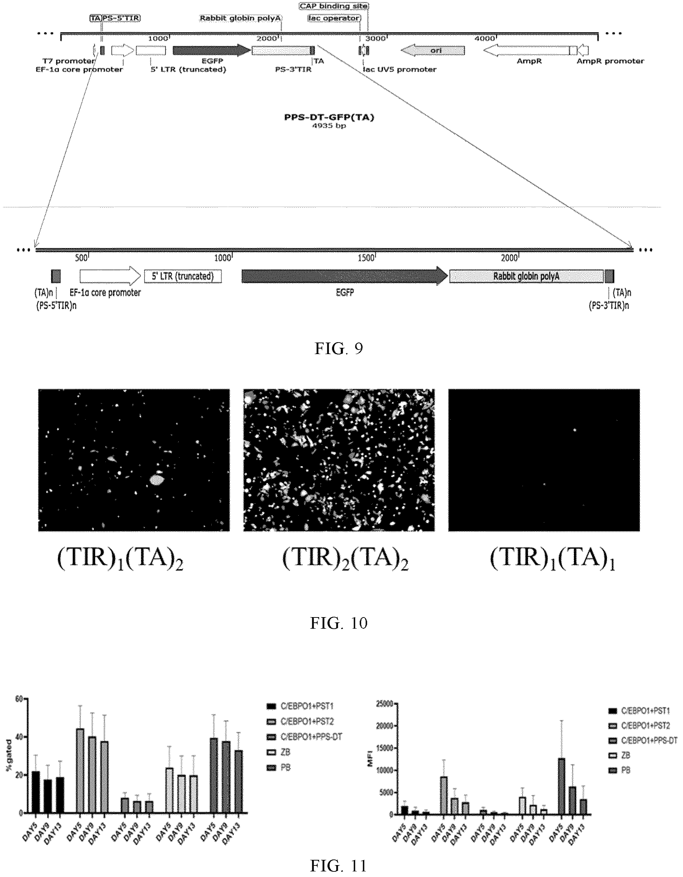

Experimental method: as shown in Fig. 9, a transposon TIR element DNA sequence and a TA shear recognition sequence at a flank thereof in a plasmid expression vector PPS-DT-GFP(ta) were subjected to n-fold sequence repeat multicopy design (the same design was carried out for the two ends of TIR and TA in this example). According to the detection method for the transposition efficiency of the CHO/PBMC cell described herein, eGFP expression was assessed by fluorescence photography and flow cytometry to verify whether the optimization of the TIR element DNA sequence could continue to improve the transposition efficiency of the PS transposase of the fusion polypeptide. In this experiment, only one PS transposase of the fusion polypeptide, pLoxP-mPS-C/EBPO1, was tested. PST1 and PST2 in the transposon plasmids PO-PST1-DCGEGFP and PO-PST2-DCGEGFP stood for (TIR)₁(TA)₂ and (TIR)₂(TA)₂, respectively, and transposon plasmids PO-PST1-DCGEGFP and PO-PST2-DCGEGFP were obtained by PPS-DT-GFP(ta) plasmid following the modified TIR functional sequence as shown in FIG. 9. The sequence of PO-PST2-DCGEGFP was shown in SEQ ID NO: 61. Ploxp-hTZB+pZB-dCGEGFP referred to a ZB transposon system (see CN105018523A), which was abbreviated as ZB. Ploxp-fHyPB+pC23S-dCGEGFP referred to a highly efficient hyPB transposon system (see CN105154473A), which was abbreviated as PB.

Three replicate wells were set up for each subgroup in a CHO cell experiment, and detected by fluorescence photography and flow cytometry on Days 3-14 after transfection. Six replicate wells were set up for each subgroup in a PBMC experiment, and detected by fluorescence photography and flow cytometry on Days 5, 9, and 13 after transfection. The positive rate and amount of eGFP expression in the cell could reflect whether the transposition efficiency was improved or not.

### Experimental result

The result in the CHO cell was shown in FIG. 10 and Table 2. The result showed that the optimization of a TIR element DNA motif and/or a TA motif in repeat and tandem copies based on the leucine zipper C/EBPO at the N-terminal fused with the PS transposase could further improve the transposition efficiency of the transposon system. eGFP fluorescence intensity was stronger.

**Table 2 Transposition effect of the optimized TIR element DNA motif in the CHO cell (a positive rate)**

| Grouping | Plasmid | DAY7 | DAY14 |
|---|---|---|---|
| (TIR)₁(TA)₂ | ploxP-MPS-C/EBPO1+PO-PST1-DCGEGFP | 60.7% | 59.9% |
| (TIR)₂(TA)₂ | ploxP-MPS-C/EBPO1+PO-PST2-DCGEGFP | 84.4% | 83.9% |
| (TIR)₁(TA)₁ | ploxP-MPS-C/EBPO1+PPS-DT -GFP (ta) | 41.8% | 41.3% |
| NTC | None | 0.13 | 0 |

The result in PBMC was shown in FIG. 11. The result showed that the optimization of a TIR element DNA motif and/or a TA motif in repeat and tandem copies could further improve the transposition efficiency of the transposon system. EGFP fluorescence intensity was stronger. It was indicated that this improvement could give a reproducible result in a primary immune cell and had a synergistic function with the fusion-type PS transposase.

Compared with PB and ZB plasmids, the cell positivity of pLoxP-mPS-C/EBPO1 + PO-PST2-dCGEGFP was slightly higher than that of the PB group, but MFI thereof was slightly lower than that of the PB plasmid. The above results indicated that the transposition efficiency of the JL transposon system after the leucine zipper C/EBPO at the N-terminal fused with the PS transposase and the optimization of the TIR motif and the TA motif in the tandem multiple copies were comparable to that of the PB transposon system.

### Example 3: Screening of a PS transposase mutant for efficient gene integration in a CHO cell

### Implementation objective:

To test the transposition efficiency of different PS transposase mutants in the CHO cell and to identify the transposase mutant for efficient transposition integration.

### Implementation method

An electrotransformed plasmid for a validation system of the example included pLoxP-mPS-C-EBPO1 comprising an expression frame of a fused wild-type PS transposase and a mutant plasmid thereof, and a transposon vector plasmid PPS-DT-GFP(ta) comprising an expression frame of an eGFP fluorescent protein. The sequence of a vector pLoxP-mPS-C-EBPO1 comprising the fused wild-type PS transposase was shown in SEQ ID NO: 39. Different vector sequences for an engineered PS transposase could be obtained by substituting a codon in an amino acid at the corresponding mutation position according to the sequence of SEQ ID NO: 39.

To test the transposition efficiency of different engineered PS transposase mutants, the CHO cell was transfected with a constructed engineered transposase or a transposon at a certain mass ratio using an electroporation method according to Method 4. The CHO cell was transfected with a fused wild-type PS transposase and an unfused wild-type PS transposase (the PS transposase) as a control transposase together. The cell grew in a complete medium (without drug selection). Further, eGFP expression was assessed by fluorescence photography and flow cytometry on Days 3-14 after transfection.

### Implementation result

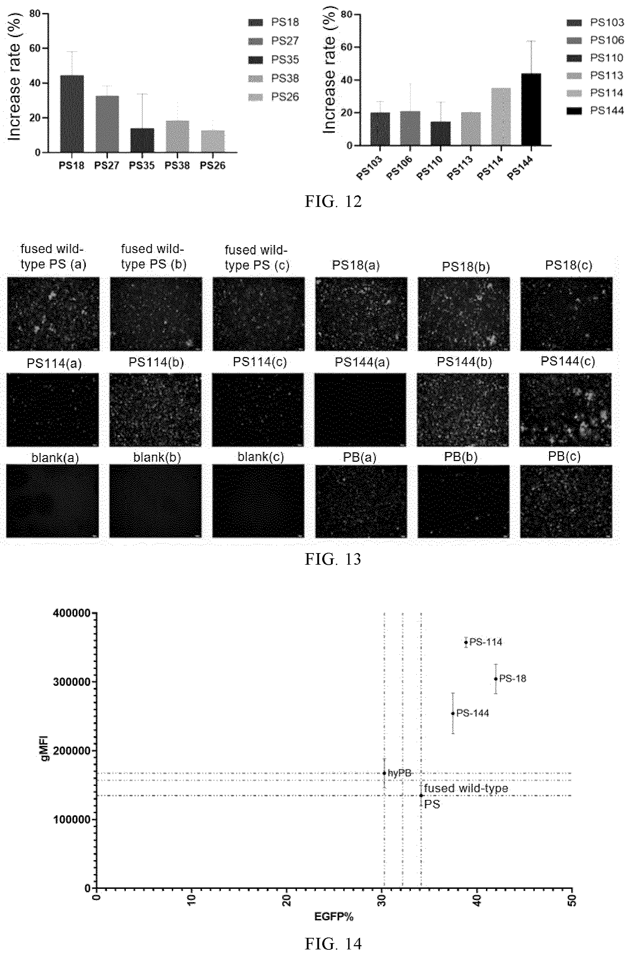

Based on the test result of the CHO cell, a series of fusion-type PS transposase mutants with an increased transposition positive rate compared to the fused wild-type PS transposase and the unfused wild-type PS transposase were screened, as shown in Table 3, Table 4, and FIG. 12.

**Table 3 Increased efficiency of a dominant mutant relative to the fused wild-type PS transposase on Day 14 after transfection**

| Serial number | Mutation site | Increased positive rate (%) |
|---|---|---|
| PS18 | TQS57-59KKA | 60 |
| PS154 | T129R | 59 |
| PS144 | T129K | 58 |
| PS27 | I98K | 39 |
| PS114 | TQ57-58RK | 35 |
| PS106 | E32K | 33 |
| PS168 | E32K\T129K | 29.33 |
| PS35 | R123H | 28 |
| PS38 | Q136K | 27 |
| PS103 | K16R | 25 |
| PS110 | E47K | 23 |
| PS153 | TQ57-58RR | 21.90 |
| PS167 | E32K\T57R\Q58R | 21.76 |
| PS113 | T57R | 21 |
| PS26 | M95L | 21 |
| PS14 | Y46Q | 19 |
| PS06 | A8S | 18 |
| PS50 | T187K | 17 |
| PS11 | I35V | 16 |
| PS54 | N199H | 12.76 |
| PS52 | N193S | 12.33 |
| PS86 | T350S | 10.39 |
| PS104 | Q22K | 10 |
| PS93 | T368E | 9.89 |
| PS55 | N213D | 9.74 |
| PS105 | H24R | 8 |
| PS146 | T150A | 8 |
| PS148 | H165D | 8 |
| PS112 | K55R | 7 |
| PS122 | K73R | 7 |
| PS56 | L228M | 6.86 |
| PS83 | E335S | 4.35 |
| PS47 | K159H | 4 |
| PS90 | V359L | 3.51 |
| PS145 | T129Q | 3 |
| PS151 | H215K | 3 |
| PS16 | R51K | 3 |
| PS125 | A84L | 2 |
| PS19 | Q69E | 2 |
| PS71 | I284L | 1.21 |
| PS108 | K45R | 1 |
| PS150 | H215E | 1 |
| PS152 | H215Q | 1 |
| PS59 | 1237V | 0.22 |
| C-EBPO1 (mPS-C-EBPO1) | | 0 |

| | | |
|---|---|---|
| C-EBPO1 in the table is a fused wild-type PS transposase mPS-C-EBPO1. | | |

Among these mutants, A8S, I35V, Y46Q, TQS57-59KKA, M95L, I98K, R123H, Q136K, K16R, Q22K, E47K, K55R, TQ57-58RK, T129R, T129K, and Q136K had significantly increased transposition activity. In particular, when an amino acid in a HTH structure of a double-stranded DNA-binding oligomerization domain region of the PS transposase was mutated to a positively charged amino acid such as histidine (H), lysine (K), or arginine (R) (e.g., K16R, E32K, T57R, TQ57-58RK, TQS57-59KKA, I98K, R123H, T129K, T129R, etc.), transposition efficiency was increased more significantly.

In addition, although not increased relative to the fused wild-type PS transposase (mPS-C-EBPO1), the positive rates of a part of the fusion-type PS transposase mutants were more increased than that of the wild-type PS (mPS). A result was shown in Table 4.

**Table 4 Increased efficiency of a part of the mutants and the wild-type PS transposase relative to the fused wild-type PS transposase on Day 14 after transfection**

| Serial number | Mutation site | Increased positive rate (%) |
|---|---|---|
| C-EBPO1 (mPS-C-EBPO1) | | 0 |
| PS117 | Q69R | -1 |
| PS28 | L100I | -1 |
| PS129 | K96R | -2 |
| PS147 | Q162R | -2 |
| PS67 | A271K | -2.56 |
| PS96 | R372K | -2.65 |
| PS09 | H20Y | -3 |
| PS44 | TYCR150-153KFVI | -3 |
| PS57 | N232K | -3.11 |
| PS91 | A363N | -3.12 |
| PS99 | A377C | -3.47 |
| PS85 | Q346M | -3.77 |
| PS08 | A17V | -4 |
| PS136 | N105G | -4 |
| PS23 | Q76E | -4 |
| PS88 | I354V | -4.08 |
| PS37 | VS133-134IA | -5 |
| PS64 | S253K | -5.09 |
| PS94 | V370I | -5.67 |
| PS76 | SV297-298TE | -5.73 |
| PS72 | EH286-286DS | -5.76 |
| PS87 | A352S | -5.76 |
| PS61 | ANS241-243VHE | -5.90 |
| PS75 | S297T | -5.96 |
| PS24 | A791 | -6 |
| PS41 | T147S | -6 |
| PS49 | I178M | -6 |
| PS68 | I276V | -6.32 |
| PS82 | Q331E | -6.72 |
| PS65 | R257E | -6.75 |
| PS84 | T339S | -6.76 |
| PS124 | A79V | -7 |
| PS135 | H104L | -7 |
| PS22 | V74L | -7 |
| PS31 | S107E | -7 |
| PS39 | Q142E | -7 |
| PS98 | R375K | -7.24 |
| PS62 | S243E | -7.45 |
| PS53 | R195K | -7.83 |
| PS10 | Q22E | -8 |
| PS81 | A328Q | -8.29 |
| PS137 | G111A | -9 |
| PS33 | R110K | -9 |
| PS92 | I365V | -9.24 |
| PS100 | VTE380-382SNA | -9.91 |
| PS51 | R190K | -11.06 |
| PS95 | RA372-373KS | -11.24 |
| PS121 | K73L | -12 |
| PS142 | K121R | -12 |
| PS29 | R102K | -12 |
| PS43 | C152V | -14 |
| PS69 | IY276-277VW | -14.54 |
| PS73 | V292I | -14.96 |
| PS21 | K73E | -16 |
| PS30 | H104M | -19 |
| PS60 | 1239V | -19.22 |
| PS13 | K42E | -20 |
| PS89 | K356Q | -20.51 |
| PS42 | T150K | -22 |
| PS78 | G316S | -22.65 |
| PS66 | IY259-260VW | -23.72 |
| PS17 | V53M | -24 |
| PS32 | E108D | -24 |
| PS111 | V53L | -25 |
| PS20 | DKV72-74EEL | -26 |
| PS70 | M280F | -29.43 |
| PS25 | S85G | -33 |
| PS116 | N63K | -36 |
| PS127 | V91L | -38 |
| PS63 | K251L | -40.03 |
| PS143 | H124N | -44 |
| PS77 | K308S | -44.44 |
| PS07 | N10D | -48 |
| PS102 | H7R | -51 |
| PS34 | G112S | -55 |
| PS101 | H7K | -64 |
| PS80 | TA327KE | -65.79 |
| mPS (Non-fused wild type) | | -87 |

### Example 4: Validation of a PS transposase mutant for efficient transposition gene integration in PBMC

### Implementation objective:

To continue the validation review of 3 dominant mutants in a CHO cell in a primary immune cell PBMC, and to verify whether the mutants had the same significant enhancement effect in different types of primary cells.

### Experimental steps:

An electrotransformation plasmid for a validation system of the example included pLoxP-mPS-C-EBPO1 comprising an expression frame of a fused wild-type PS transposase and a mutant plasmid thereof, and a transposon vector plasmid PPS-DT-GFP(ta) comprising an expression frame of an eGFP fluorescent protein.

To test the transposition efficiency of a dominant engineered PS transposase mutant, the PBMC was transfected with a constructed engineered transposase or a transposon at a certain mass ratio using an electroporation method according to Method 5. The PBMC was transfected with a fused wild-type PS transposase and a *PiggyBac* (hyPB) transposase as a control transposase together. The cell grew in a complete medium (without drug selection). Further, eGFP expression was assessed by fluorescence photography and flow cytometry on Days 3-14 after transfection.

### Experimental result:

A sample was taken on Day 7 and Day 14 after electrotransformation, and EGFP expression was detected by flow cytometry. Analyzed data were samples on Day 14. As shown in FIG. 13, by combining the cellular data of the 3 groups of PBMC, that is, PS18, PS114, and PS144, a positive rate and fluorescence intensity were somewhat increased, whether using a fused wild-type PS or ploxp-fHyPB as a reference. Dominant PS transposase mutants 18, 114, 144, etc. could basically maintain the level of 40%. An expression amount was doubled compared with the fused wild-type PS transposase.

A\B\C in FIG. 14 shows the positive rate and eGFP expression level of the mutant and a control group, respectively; the magnitude of the increase of the positive rate and eGFP expression level of a PS mutant relative to the fused wild-type PS transposase; and the magnitude of the increase of the positive rate and eGFP expression level of the PS mutant relative to a highly efficient hyPB transposase.

### Example 5: Evaluation of the efficiency of a JL transposon system comprising a TIR DNA motif mutation and/or multiple copies and a fused mutation type PS transposase

### Implementation objective:

A PS transposon TIR DNA motif was mutated to evaluate the screening for a dominant mutation DNA motif in a Hela cell.

The screened dominant mutation TIR DNA motifs were grouped in the form of (TIR)n(TA)n and combined with the fused mutation type PS transposase for the final evaluation of screening an optimal JL transposition system.

### Test method:

A starting transposon integration plasmid pPG-PGK-NEO (PSTIR0) was constructed with the plasmid sequence as shown in SEQ ID NO: 40. PPG-PGK-NEO (PSTIR1), pPG-PGK-NEO (PSTIR2), pPG-PGK-NEO (PSTIR3) were constructed by sequentially performing nucleotide mutations on TIR, respectively. The above plasmid pPG-PGK-NEO (PSTIR1) was denoted as 1TA-1TIR1. Transposon DNA functional elements of 1TA-2TIR1, 2TA-1TIR1, 2TA-2TIR1, 2TA-2TIR1, 4TA-1TIR1, 4TA- 2TIR1 in multicopy and tandem were constructed according to the form (TIR)n(TA)n, respectively. TIR and TA at both ends in the example underwent the same design. A transposase plasmid used in the example was pLoxP-mPS-C-EBPO1 in which a PS transposase amino acid mutation was a PS18 mutant sequence, i.e., the transposase was a PS18 mutant fused with C-EBPO at the N-terminal.

**Table 6**

| TIR sequence number | Nucleotide sequence |
|---|---|
| Wild-type TIR, PSTIR0 | ccgtattttccgcactataaggcgcacc (SEQ ID NO: 2) |
| Mutation TIR, PSTIR1 | ccgtattttccgcactataagAcgcacc (SEQ ID NO: 4) |
| Mutation TIR, PSTIR2 | ccgtattttccgcaGtataaggcgcacc (SEQ ID NO: 5) |
| Mutation TIR, PSTIR3 | ccgtattttccgGactataaggcgcacc (SEQ ID NO: 6) |

The Hela cell was cultured in conditions of a 10% fetal bovine serum. 0.1 mg/mL of penicillin in a high glucose medium DMEM (Gibco, USA) at 37°C in a 5% CO2 cell culture incubator.

Cells were spread on a six-well plate at a density of 3 × 10⁵/well one day before transfection. Three replicate wells were set up in each group. The cells were mixed with a Transfectamine 5000 Transfection Reagent (AAT Bioquest, USA) at a ratio of 1:5 and transfected according to instructions. After 24 h of transfection of the cells, 1% of cells per well were inoculated into a 10 cm dish. When the cells grew to 10%, the medium was changed to a 600 ng/µL G418 screening medium. Thereafter, a screening medium was changed every three days for about 2 weeks. After the cells in a control group were completely dead, each group was stained with Giemsa for counting. ImageJ software was used for counting, and GraphPad software was used for statistical analysis.

### Experimental result:

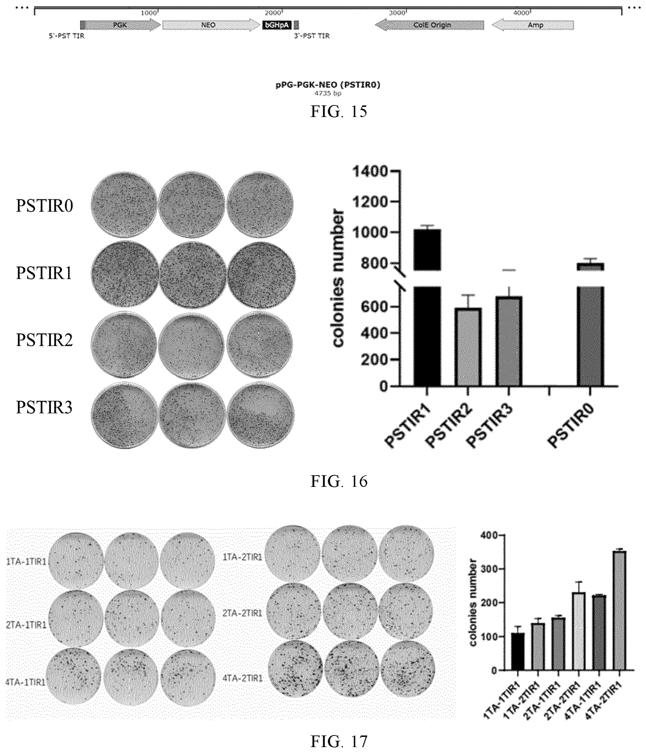

As shown in FIG. 16, the example demonstrated that by designing a TIR DNA motif nucleotide mutation, a transposon could still achieve a transposition integration function, wherein a TIR1 mutation can achieve an increase in transposition efficiency. As shown in FIG. 17, combining multiple copies of dominant screened TIR1 DNA motif functional elements in tandem could further improve the gene integration transposition efficiency of the transposon system.

### Example 6: Evaluation of transposition integration efficiency of an engineered JL transposase mRNA and a sequence-optimized transposon TIR functional sequence on PBMC

### 1. The objective of an experiment:

To verify the effectiveness of an engineered JL transposon system to integrate plasmids using different plasmid-type transposons in combination with a JL transposase mRNA.

### 2. Experimental method:

A fusion-type PS18 transposase mutant in Example 5 was selected to construct an mRNA synthetic DNA transcription template. The PS18 mRNA was synthesized and purified as described in Chinese Patent CN115197327A, titled "RNA-MODIFIED CHIMERIC PROTEIN AND APPLICATION THEREOF". A sequence of the fusion-type PS18 transposase mutant PS18 mRNA was shown in SEQ ID NO: 41.

Different plasmid-type transposon integration plasmids were constructed, wherein pTini-DPS(CES)-dCGEGFP was an antibiotic resistance gene-free miniplasmid (it also referred to as a miniplasmid or a tiniplasmid in the examples) with a replicon of R6K and comprised a coding sequence for an antitoxin protein (an amino acid sequence of the antitoxin protein was shown in SEQ ID NO: 52), the sequence of which was shown in SEQ ID NO: 42. PTini-DPS(CES)-dCGEGFP comprised (TIR)₂(TA)₂ as described above, and was distinct from pO-PST2-dCGEGFP by replacing a standard STD plasmid with an antibiotic resistance gene-free miniplasmid. A sequence of pO-PST2-dCGEGFP was shown in Example 2 above. PC23S-dCGEGFP was a standard STD plasmid comprising PB transposon TIR, the sequence of which was shown in SEQ ID NO: 43.

To test the transposition efficiency of a dominant fusion-type PS transposase mutant mRNA, PBMC was transfected with a constructed engineered transposase or a transposon at a certain mass ratio using an electroporation method according to Method 5. The PBMC was transfected with WT PS transposase and *PiggyBac* (hyPB) transposase as a control transposase. The cell grew in a complete medium (without drug selection). Further, eGFP expression was assessed by fluorescence photography and flow cytometry on Days 3-14 after transfection. Grouping and plasmid use dosage were shown in Table 7 below, and mRNA use dosage was 20 µg.

**Table 7 Grouping and plasmid use dosage**

| No. | Grouping | Plasmid | Plasmid use dosage |
|---|---|---|---|
| 1 | Miniplasmid control of JL transposon | pTini-DPS(CES)-dCGEGFP | 4 µg |
| 2 | JL transposase mRNA+JL transposon miniplasmid | PS18 mRNA+pTini-DPS(CES)-dCGEGFP | 4 µg |
| 3 | JL transposase mRNA+JL transposon miniplasmid | | 2.9 µg |
| 4 | PB transposon standard STD plasmid control | PC23S(DTS)-dCGEGFP | 4 µg |
| 5 | PB transposase mRNA+PB transposon standard STD plasmid | PB mRNA+PC23S-dCGEGFP | 4 µg |
| 6 | JL transposon standard STD plasmid | pO-PST2-dCGEGFP | 4 µg |
| 7 | JL transposase mRNA+JL transposon standard STD plasmid | PS18 mRNA+pO-PST2-dCGEGFP | 4 µg |

### Experimental result

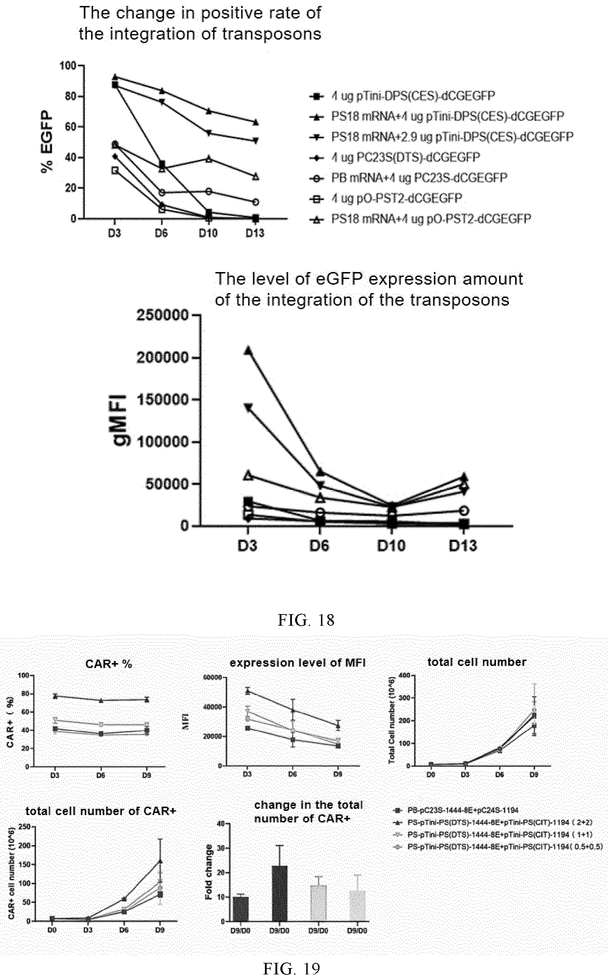

As shown in FIG. 18, the grouping of JL transposase mRNA+JL transposon miniplasmid exhibited the best transposition efficiency and integrated gene expression levels. The grouping of the JL transposase mRNA+JL transposon miniplasmid was superior to the grouping of JL transposase mRNA+standard STD plasmid. The JL transposase mRNA as a whole was superior to a PB mRNA group of a control group. It was indicated that a novel JL transposase in the form of mRNA in combination with a miniplasmid transposon plasmid could further improve the overall efficiency of the JL transposon.

### Example 7: Preparation and efficiency evaluation of an engineered JL transposon system for a CAR-T immune cell

### The objective of an experiment

To validate the transposon integration efficiency and transgene expression of a JL transposon system in the preparation of a CAR-T immune cell.

### Experimental method

A plasmid and grouping used in this experiment were shown in Table 8 below. Sequences of the corresponding plasmids pC23S-1444-8E, pC24S-1194, pTini-PS(DTS)-1444-8E, pTini-PS(CIT)-1194, and pC23S-EGFP-8E were shown in SEQ ID NOs: 44-48, wherein a JL transposon in a tiniplasmid comprised (TIR)₁(TA)₂. The mRNA used in this experiment was the same as that in Example 6. Specifically, CAR-T was MSLN CAR-T of an autocrine PD-1 nanobody, as described in Patent CN202111681582.0. The CAR-T used in the example was a CAR-T cell comprising mesothelin nanobody MSLN3[1444] (referred to as "1444+1194nla" or "1444-1194") of an autocrine PD1 nanobody 1194nla in Example 6 of Patent CN202111681582.0. A PB control group was set up.

PBMC was transfected with a constructed engineered transposase or a transposon at a certain mass ratio by an electroporation method according to Method 5. The PBMC was transfected with a *PiggyBac* (hyPB) transposase as a control transposase together. A cell grew in a complete medium (without drug selection). CAR gene expression was assessed by fluorescence photography and flow cytometry on Days 3-14 after transfection. An antibody secretion expression level was detected by ELISA. Grouping and plasmid use dosage were shown in the table below. The mRNA use dosage was 20 µg.

**Table 8 Plasmid and grouping**

| No. | Grouping | Plasmid 1 | Plasmid use dosage | Plasmid 2 | Plasmid use dosage |
|---|---|---|---|---|---|
| 1 | MockT (GFP) control group | pC23S-EGFP-8E | 6 µg | | |
| 2 | PB mRNA + transposon standard STD plasmid (1444 CART + 1194 autocrine antibody) | pC23S-1444-8E | 4 µg | pC24S-1194 | 4 µg |
| 3 | JL transposase mRNA + transposon tiniplasmid (1444 CART + 1194 autocrine antibody) | pTini-PS(DTS)-1444-8E | 2 µg | pTini-PS(CIT)-1194 | 2 µg |
| 4 | JL transposase mRNA + transposon tiniplasmid (1444 CART + 1194 autocrine antibody) | pTini-PS(DTS)-1444-8E | 1 µg | pTini-PS(CIT)-1194 | 1 µg |
| 5 | JL transposase mRNA + transposon tiniplasmid (1444 CART + 1194 autocrine antibody) | pTini-PS(DTS)-1444-8E | 0.5 µg | pTini-PS(CIT)-1194 | 0.5 µg |

### Experimental result

As shown in FIG. 19, a JL transposase showed more efficient in terms of a CAR positive rate, an expression level, and the total number and an absolute number of positive cells. The positive rate and the expression level were increased in a dosage-dependent manner. The dosages of the JL transposase and the transposon plasmid were much lower than that of a classical hyPB system when an antibiotic resistance gene-free miniplasmid was used as a donor DNA under the premise of the same transgene effect. Table 9 showed the expression amount of a PD-1 nanoantibody at Days 3-9. As can be seen from Table 9, the use of the JL transposon system of the present invention in combination with an anti-antibody-free minivector (tiniplasmid) could reduce the dosage of the plasmid while also dramatically increasing the expression level of the autocrine antibody genes compared to a PB transposon system.

**Table 9 Autocrine PD-1 nanobody expression amount on D3-D9**

| Subgroup No. | Plasmid 2 | Plasmid use dosage | D3-D9 expression amount (ng/CAR+ cell) | | |
|---|---|---|---|---|---|
| | | | D3 expression amount | D6 expression amount | D9 expression amount |
| 1 | | | | | |
| 2 | pC24S-1194 | 4 µg | 130.33 | 1.96 | 1.54 |
| 3 | pTini-PS(CIT)-1194 | 2 µg | 317.59 | 22.21 | 10.17 |
| 4 | pTini-PS(CIT)-1194 | 1 µg | 322.65 | 7.07 | 4.25 |
| 5 | pTini-PS(CIT)-1194 | 0.5 µg | 188.52 | 4.36 | 2.62 |

In summary, a novel JL transposon system (especially with an antibiotic resistance gene-free miniplasmid as a vector) for immune cell CAR-T preparation showed good performance. An overall effect thereof was far superior to that of a current gold standard piggybac transposon system. Both the efficiency of gene integration and transposition and the expression level of transgene were much higher than those of a PB control group. When the antibiotic resistance gene-free miniplasmid was used as the transposon donor DNA, the novel efficient JL transposon system had advantages in both efficiency and administered dosage.

### Sequence of the present invention

SEQ ID NO: 1>PS transposase amino acid sequence 
MAPTKRHAYNAEFKLKAISHAQEHGNRAAAREFNINESMVRKWRKYEDELRQVKKTTQSFRGNKARWPQLEDKV
EQWVAEQRAASRSVSTVTIRMKAIALAREHNISEFRGGPSWCFRFMKRRHLSIRTRTTVSQQLPADYQEKLATFRTY
CRNKITEKKIQPEHIINMDEVPLTFDIPVNRTVDKTGARTVNIRTTGNEKTSFTVVLACQANGHKLPPMVIFKRKTLP
KENFPAGIVIKANSKGWMDEEKMSEWLREIYVKRPGGFFHTAPSLLIYDSMRAHITEHVKKQVKHTNSVLAVIPGG
LTKELQPLDVGVNRAFKARLRTAWEQWMTEGEHTFTKTGRQRRTTYANICKWIVNAWAGISVTTVVRAFRKAGIV
TELPDNSSDTDSVNDDFDKTEPGVLDAAIAQLFNSDTEEEVFEGF*
SEQ ID NO: 2> DNA sequence of PS transposon TIR (5')
   CCGTATTTTCCGCACTATAAGGCGCACC
SEQ ID NO: 3> DNA sequence of PS transposon TIR (3')
   ggtgcgcctt atagtgcgga aaatacgg
SEQ ID NO: 4>TIR1
   CCGTA TTTTC CGCAC TATAA GACGCACC
SEQ ID NO: 5>TIR2
   CCGTA TTTTC CGCAGTATAAGGCGCACC
SEQ ID NO: 6>TIR3
   CCGTA TTTTC CGGACTATAAGGCGCACC
SEQ ID NO: 7>Dare-PasserC-ray-finned fishes
   CCGTATTTTCCGCACTATAAGGCGCACC
SEQ ID NO: 8>Lach-PasserC-lobe-finned fishes
   CCGTATATCGCGGCGTATAAGTCGACCT
SEQ ID NO: 9>Anro-PasserC-ray-finned fishes
   CCGTATTTTCCGGACTATAAGGCGCACT
SEQ ID NO: 10>Fuhe-PasserC-ray-finned fishes
   CCGTATTTTCCGCACTATAAGGCGCACC
SEQ ID NO: 11>Maze-PasserC-ray-finned fishes
   CCGTATTTTCACGACCATAAGGCGCACT
SEQ ID NO: 12>Puny-PasserC-ray-finned fishes
   CCGTATTTTCACGACCATAAGGCGCACT
SEQ ID NO: 13>Tafl-PasserC-ray-finned fishes
   CCGTATTTTCACAACCATAAGGCGCGCC
SEQ ID NO: 14>Anca-PasserC-lizards
   CCGTATTTTTCGCTTTATAGGACGCACCT
SEQ ID NO: 15>Crpo-PasserB-crocodilians
   CCGTGTTTCCCCGAAAATAAGACA
SEQ ID NO: 16>Mate-PasserC-turtles
   CCGTATTTTCCGGCGTATAAGACGACT
SEQ ID NO: 17>Napa-PasserC-frogs
   CCGTATTTTTCCGTGTATAAGACGC
SEQ ID NO: 18>Epfu-PasserC-bats
   CTGTATTTTCCGGCGTATAAGACAAC
SEQ ID NO: 19>Myda-PasserC-bats
   CCGTATTTGCCGGCGTATAAGACG
SEQ ID NO: 20>Prmu-PasserC-snakes
   CCGTATATTCCGGCTTATAAGATGACT
SEQ ID NO: 21>Hero-PasserB-annelida
   CCGTGTTTCCTCATATATAAGACA
SEQ ID NO: 22>Lahe-PasserC-arthropoda
   CTGTATTTATGCTAATCTAATGCGCAT
SEQ ID NO: 23>Hala-PasserC-arthropoda
   CCGTATTTTTCGGACCATAAGACGCACC
SEQ ID NO: 24>Orfa-PasserC-cnidaria
   CCGTATTTACTCATGTAGAAGTCGACCT
SEQ ID NO: 25>Crvi-PasserC-mollusca
   CCGTAAAGTCTGGTGTATAGCACGC
SEQ ID NO: 26>Scme-PasserC2-platyhelminthes
   CTGTAAAATCCCGAGTATAAGCTGCAGC
SEQ ID NO: 27>Amqu-PasserB-porirera
   CCGTAAATGTTCAAATAAAAGCCCGG
SEQ ID NO: 28>C-EBPO amino acid sequence
   TQQKVLELTSDNDRLRKRVEQLSRELDTLRG
SEQ ID NO: 29>LZIP amino acid sequence
   LQNKVQRLEEQNLSLLDQLRKLQAMVIEI
SEQ ID NO: 30>CREPT amino acid sequence
   KDVLSEKEKKLEEYKQKLARV
SEQ ID NO: 31>TEF amino acid sequence
   LKENQITIRAAFLEKENTALRTEVAELRKEVGKCKT
SEQ ID NO: 32>(TA)₄(TIR)₂
   TATATATAccgtattttccgcactataagAcgcaccccgtattttccgcactataagAcgcacc
SEQ ID NO: 33> wild-type PS transposase expression plasmid (pLoxP-mPS) 
aacgccagcaacgcggcctttttacggttcctggccttttgctggccttttgctcacatgttctttcctgcgttatcccctgattctgtggataaccgtattaccgcctttgagtgagctgatacc
gctcgccgcagccgaacgaccgagcgcagcgagtcagtgagcgaggaagcggaagagcgcccaatacgcaaaccgcctctccccgcgcgttggccgattcattaatgcagCT
GACTGAGCTCaatgggaaagtaccgttcggagttccgcgttacataacttacggtaaatggcccgcctggctgaccgcccaacgacccccgcccattgacgtcaataatgac
gtatgttcccatagtaacgccaatagggactttccattgacgtcaatgggtggagtatttacggtaaactgcccacttggcagtacatcaagtgtatcatatgccaagtacgccccctattg
acgtcaatgacggtaaatggcccgcctggcattatgcccagtacatgaccttatgggactttcctacttggcagtacatctacgtattagtcatcgctattaccaAAGGATCTGC
GATCGCTCCGGTGCCCGTCAGTGGGCAGAGCGCACATCGCCCACAGTCCCCGAGAAGTTGGGGGGAGGGGTCG
GCAATTGAACGGGTGCCTAGAGAAggtggcgcggggtaaactgggaaagtgatgtcgtgtactggctccgcctttttcccgagggtgggggagaaccgtatat
aagtgcagtagtcgccgtgaacgttctttttcgcaacgggtttgccgccagaacacagctgaagcttcgaggggctcgcatctctccttcacgcgcccgccgccctacctgaggccgc
catccacgccggttgagtcgcgttctgccgcctcccgcctgtggtgcctcctgaactgcgtccgccgtctaggtaagtttaaagctcaggtcgagaccgggcctttgtccggcgctccct
tggagcctacctagactcagccggctctccacgctttgcctgaccctgcttgctcaactctacgtctttgtttcgttttctgttctgcgccgttacagatccaagctgtgaccggcgcctacG
AATTCAATTCATAACTTCGTATAGCATACATTATACGAAGTTATACTAGTgccaccatcctgctgccaagagggtcaagttggacGC
CCCCACCAAGAGACACGCCTACAACGCCGAGTTCAAGCTGAAGGCCATCAGCCACGCCCAGGAGCACGGCAA
CAGAGCCGCCGCCAGAGAGTTCAACATCAACGAGAGCATGGTGAGAAAGTGGAGAAAGTACGAGGACGAGCT
GAGACAGGTGAAGAAGACCACCCAGAGCTTCAGAGGCAACAAGGCCAGGTGGCCCCAGCTGGAGGACAAGGT
GGAGCAGTGGGTGGCCGAGCAGAGAGCCGCCAGCAGAAGCGTGAGCACCGTGACCATCAGAATGAAGGCCAT
CGCCCTGGCCAGAGAGCACAACATCAGCGAGTTCAGAGGCGGCCCCAGCTGGTGCTTCAGATTCATGAAGAGA
AGACACCTGAGCATCAGAACCAGAACCACCGTGAGCCAGCAGCTGCCCGCCGACTACCAGGAGAAGCTGGCC
ACCTTCAGAACCTACTGCAGAAACAAGATCACCGAGAAGAAGATCCAGCCCGAGCACATCATCAACATGGAC
GAGGTGCCCCTGACCTTCGACATCCCCGTGAACAGAACCGTGGACAAGACCGGCGCCAGAACCGTGAACATCA
GAACCACCGGCAACGAGAAGACCAGCTTCACCGTGGTGCTGGCCTGCCAGGCCAACGGCCACAAGCTGCCCCC
TATGGTGATCTTCAAGAGAAAGACCCTGCCCAAGGAGAACTTCCCCGCCGGCATCGTGATCAAGGCCAACAGC
AAGGGCTGGATGGACGAGGAGAAGATGAGCGAGTGGCTGAGAGAGATCTACGTGAAGAGACCCGGCGGCTTC
TTCCACACCGCCCCCAGCCTGCTGATCTACGACAGCATGAGAGCCCACATCACCGAGCACGTGAAGAAGCAGG
TGAAGCACACCAACAGCGTGCTGGCCGTGATCCCCGGCGGCCTGACCAAGGAGCTGCAGCCCCTGGACGTGGG
CGTGAACAGAGCCTTCAAGGCCAGACTGAGAACCGCCTGGGAGCAGTGGATGACCGAGGGCGAGCACACCTT
CACCAAGACCGGCAGACAGAGAAGAACCACCTACGCCAACATCTGCAAGTGGATCGTGAACGCCTGGGCCGG
 
CATCAGCGTGACCACCGTGGTGAGAGCCTTCAGAAAGGCCGGCATCGTGACCGAGCTGCCCGACAACAGCAGC
GACACCGACAGCGTGAACGACGACTTCGACAAGACCGAGCCCGGCGTGCTGGACGCCGCCATCGCCCAGCTGT
TCAACAGCGACACCGAGGAGGAGGTGTTCGAGGGCTTCTGATAAGGATCCGCTAGCATAACTTCGTATAGCAT
ACATTATACGAAGTTATAACTTGTTTATTGCAGCTTATAATGGTTACAAATAAAGCAATAGCATCACAAATTTC
ACAAATAAAGCATTTTTTTCACTGCATTCTAGTTGTGGTTTGTCCAAACTCATCAATGTATCTTATCATGTCTGG
ATCTTAGTCAGTTACTCGAGACTGGTACCTGActggcgtaatagcgaagaggcccgcaccgatcgcccttcccaacagttgcgcagcctgaatggcga
atggaaattgtaagcgttaatattttgttaaaattcgcgttaaatttttgttaaatcagctcattttttaaccaataggccgaaatcggcaaaatcccttataaatcaaaagaatagaccgagata
gggttgagtgttgttccagtttggaacaagagtccactattaaagaacgtggactccaacgtcaaagggcgaaaaaccgtctatcagggcgatggcccactacgtgaaccatcaccct a
atcaagttttttggggtcgaggtgccgtaaagcactaaatcggaaccctaaagggagcccccgatttagagcttgacggggaaagccggcgaacgtggcgagaaaggaagggaag
aaagcgaaaggagcgggcgctagggcgctggcaagtgtagcggtcacgctgcgcgtaaccaccacacccgccgcgcttaatgcgccgctacagggcgcgtcaggtggcactttt
cggggaaatgtgcgcggaacccctatttgtttatttttctaaatacattcaaatatgtatccgctcatgagacaataaccctgataaatgcttcaataatattgaaaaaggaagagtatgagta
ttcaacatttccgtgtcgcccttattcccttttttgcggcattttgccttcctgtttttgctcacccagaaacgctggtgaaagtaaaagatgctgaagatcagttgggtgcacgagtgggttac
atcgaactggatctcaacagcggtaagatccttgagagttttcgccccgaagaacgttttccaatgatgagcacttttaaagttctgctatgtggcgcggtattatcccgtattgacgccgg
gcaagagcaactcggtcgccgcatacactattctcagaatgacttggttgagtactcaccagtcacagaaaagcatcttacggatggcatgacagtaagagaattatgcagtgctgccat
aaccatgagtgataacactgcggccaacttacttctgacaacgatcggaggaccgaaggagctaaccgcttttttgcacaacatgggggatcatgtaactcgccttgatcgttgggaac
cggagctgaatgaagccataccaaacgacgagcgtgacaccacgatgcctgtagcaatggcaacaacgttgcgcaaactattaactggcgaactacttactctagcttcccggcaaca
attaatagactggatggaggcggataaagttgcaggaccacttctgcgctcggcccttccggctggctggtttattgctgataaatctggagccggtgagcgtgggtctcgcggtatcat
tgcagcactggggccagatggtaagccctcccgtatcgtagttatctacacgacggggagtcaggcaactatggatgaacgaaatagacagatcgctgagataggtgcctcactgatt
aagcattggtaactgtcagaccaagtttactcatatatactttagattgatttaaaacttcatttttaatttaaaaggatctaggtgaagatcctttttgataatctcatgaccaaaatcccttaacg
tgagttttcgttccactgagcgtcagaccccgtagaaaagatcaaaggatcttcttgagatcctttttttctgcgcgtaatctgctgcttgcaaacaaaaaaaccaccgctaccagcggtgg
tttgtttgccggatcaagagctaccaactctttttccgaaggtaactggcttcagcagagcgcagataccaaatactgtccttctagtgtagccgtagttaggccaccacttcaagaactct
gtagcaccgcctacatacctcgctctgctaatcctgttaccagtggctgctgccagtggcgataagtcgtgtcttaccgggttggactcaagacgatagttaccggataaggcgcagcg
gtcgggctgaacggggggttcgtgcacacagcccagcttggagcgaacgacctacaccgaactgagatacctacagcgtgagctatgagaaagcgccacgcttcccgaagggag
aaaggcggacaggtatccggtaagcggcagggtcggaacaggagagcgcacgagggagcttccagggggaaacgcctggtatctttatagtcctgtcgggtttcgccacctctgac
ttgagcgtcgatttttgtgatgctcgtcaggggggcggagcctatggaaa
SEQ ID NO: 34>C-EBPO nucleotide sequence 
ACGCAGCAGAAGGTGCTGGAGCTGACCAGTGACAATGACCGCCTGCGCAAGCGGGTGGAACAGCTGAGCCGC
GAACTGGACACGCTGCGGGGC
SEQ ID NO: 35>LZIP nucleotide sequence 
CTGCAGAACAAGGTGCAGCGCCTGGAGGAGCAGAACCTGAGCCTGCTGGACCAGCTGCGCAAGCTGCAGGCC
ATGGTGATCGAGATC
SEQ ID NO: 36>CREPT nucleotide sequence
   AAGGACGTGCTGAGCGAGAAGGAGAAGAAGCTGGAGGAGTACAAGCAGAAGCTGGCCCGCGTG
SEQ ID NO: 37>TEF nucleotide sequence 
CTGAAAGAGAATCAGATCACCATCCGGGCAGCCTTCCTGGAGAAGGAGAACACAGCCCTGCGGACGGAGGTG
GCCGAGCTACGCAAGGAGGTGGGCAAGTGCAAGACC
SEQ ID NO: 38>PPS-DT-GFP(ta): 
gcccctgcagccgaattatattatttttgccaaataatttttaacaaaagctctgaagtcttcttcatttaaattcttagatgatacttcatctggaaaattgtcccaattagtagcatcacgctgtg
agtaagttctaaaccatttttttattgttgtattatctctaatcttactactcgatgagttttcggtattatctctatttttaacttggagcaggttccattcattgtttttttcatcatagtgaataaaatca
actgctttaacacttgtgcctgaacaccatatccatccggcgtaatacgactcactatagggagagcggccgccagatcttccggatggctcgagtttttcagcaagattaccgtattttcc
gcactataaggcgcacctcgcgagtcgacattgattattgaCtagTCAGCAACCAAGGATCTGcgatcgctccggtgcccgtcagtgggcagagcgcacatcgccc
acagtccccgagaagttggggggaggggtcggcaattgaacgggtgcctagagaaggtggcgcggggtaaactgggaaagtgatgtcgtgtactggctccgcctttttcccgaggg
tgggggagaaccgtatataagtgcagtagtcgccgtgaacgttctttttcgcaacgggtttgccgccagaacacagctgaagcttcgaggggctcgcatctctccttcacgcgcccgcc
gccctacctgaggccgccatccacgccggttgagtcgcgttctgccgcctcccgcctgtggtgcctcctgaactgcgtccgccgtctaggtaagtTTATagctcaggtcgagaccg
ggcctttgtccggcgctcccttggagcctacctagactcagccggctctccacgctttgcctgaccctgcttgctcaactctacgtctttgtttcgttttctgttctgcgccgttacagatccaa
gctgtgaccggcgcctacgaattctgcagtcgacggtaccgcgggcccgggatccaccggtcgccaccatggtgagcaagggcgaggagctgttcaccggggtggtgcccatcct
ggtcgagctggacggcgacgtaaacggccacaagttcagcgtgtccggcgagggcgagggcgatgccacctacggcaagctgaccctgaagttcatctgcaccaccggcaagct
gcccgtgccctggcccaccctcgtgaccaccctgacctacggcgtgcagtgcttcagccgctaccccgaccacatgaagcagcacgacttcttcaagtccgccatgcccgaaggcta
cgtccaggagcgcaccatcttcttcaaggacgacggcaactacaagacccgcgccgaggtgaagttcgagggcgacaccctggtgaaccgcatcgagctgaagggcatcgacttc
aaggaggacggcaacatcctggggcacaagctggagtacaactacaacagccacaacgtctatatcatggccgacaagcagaagaacggcatcaaggtgaacttcaagatccgcc
acaacatcgaggacggcagcgtgcagctcgccgaccactaccagcagaacacccccatcggcgacggccccgtgctgctgcccgacaaccactacctgagcacccagtccgccc
tgagcaaagaccccaacgagaagcgcgatcacatggtcctgctggagttcgtgaccgccgccgggatcactctcggcatggacgagctgtacaagtaaagcggccgcactcctca
ggtgcaggctgcctatcagaaggtggtggctggtgtggccaatgccctggctcacaaataccactgagatctttttccctctgccaaaaattatggggacatcatgaagccccttgagca
tctgacttctggctaataaaggaaatttattttcattgcaatagtgtgttggaattttttgtgtctctcactcggaaggacatatgggagggcaaatcatttaaaacatcagaatgagtatttggtt
tagagtttggcaacatatgcccatatgctggctgccatgaacaaaggtggctataaagaggtcatcagtatatgaaacagccccctgctgtccattccttattccatagaaaagccttgact
tgaggttagattttttttatattttgttttgtgttatttttttctttaacatccctaaaattttccttacatgttttactagccagatttttcctcctctcctgactactcccagtcatagctgtccctcttctctt
atgaagatccctcgacctcgcgaggtgcgccttatagtgcggaaaatacggTAatctttctagaagatctcctacaatattctcagctgccatggaaaatcgatgttcttcttttattctctc
aagattttcaggctgtatattaaaacttatattaagaactatgctaaccacctcatcaggaaccgttgtaggtggcgtgggttttcttggcaatcgactctcatgaaaactacgagctaaatatt
caatatgttcctcttgaccaactttattctgcattttttttgaacgaggtttagagcaagcttcaggaaactgagacaggaattttattaaaaatttaaattttgaagaaagttcagggttaatagc
atccattttttgctttgcaagttcctcagcattcttaacaaaagacgtctcttttgacatgtttaaagtttaaacctcctgtgtgaaattgttatccgctcacaattccacacattatacgagccgga
agcataaagtgtaaagcctggggtgcctaatgagtgagctaactcacattaattgcgttgcgctcactgccaattgctttccagtcgggaaacctgtcgtgccagctgcattaatgaatcg
gccaacgcgcggggagaggcggtttgcgtattgggcgctcttccgcttcctcgctcactgactcgctgcgctcggtcgttcggctgcggcgagcggtatcagctcactcaaaggcggt
aatacggttatccacagaatcaggggataacgcaggaaagaacatgtgagcaaaaggccagcaaaaggccaggaaccgtaaaaaggccgcgttgctggcgtttttccataggctcc
gcccccctgacgagcatcacaaaaatcgacgctcaagtcagaggtggcgaaacccgacaggactataaagataccaggcgtttccccctggaagctccctcgtgcgctctcctgttc
cgaccctgccgcttaccggatacctgtccgcctttctcccttcgggaagcgtggcgctttctcatagctcacgctgtaggtatctcagttcggtgtaggtcgttcgctccaagctgggctgt
gtgcacgaaccccccgttcagcccgaccgctgcgccttatccggtaactatcgtcttgagtccaacccggtaagacacgacttatcgccactggcagcagccactggtaacaggatta
 
gcagagcgaggtatgtaggcggtgctacagagttcttgaagtggtggcctaactacggctacactagaaggacagtatttggtatctgcgctctgctgaagccagttaccttcggaaaa
agagttggtagctcttgatccggcaaacaaaccaccgctggtagcggtggtttttttgtttgcaagcagcagattacgcgcagaaaaaaaggatctcaagaagatcctttgatcttttctac
ggggtctgacgctcagtggaacgaaaactcacgttaagggattttggtcatgagattatcaaaaaggatcttcacctagatccttttaaattaaaaatgaagttttaaatcaatctaaagtata
tatgagtaaacttggtctgacagttaccaatgcttaatcagtgaggcacctatctcagcgatctgtctatttcgttcatccatagttgcctgactccccgtcgtgtagataactacgatacggg
agggcttaccatctggccccagtgctgcaatgataccgcgagacccacgctcaccggctccagatttatcagcaataaaccagccagccggaagggccgagcgcagaagtggtcct
gcaactttatccgcctccatccagtctattaattgttgccgggaagctagagtaagtagttcgccagttaatagtttgcgcaacgttgttgccattgctacaggcatcgtggtgtcacgctcg
tcgtttggtatggcttcattcagctccggttcccaacgatcaaggcgagttacatgatcccccatgttgtgcaaaaaagcggttagctccttcggtcctccgatcgttgtcagaagtaagttg
gccgcagtgttatcactcatggttatggcagcactgcataattctcttactgtcatgccatccgtaagatgcttttctgtgactggtgagtactcaaccaagtcattctgagaatagtgtatgc
ggcgaccgagttgctcttgcccggcgtcaatacgggataataccgcgccacatagcagaactttaaaagtgctcatcattggaaaacgttcttcggggcgaaaactctcaaggatctta
ccgctgttgagatccagttcgatgtaacccactcgtgcacccaactgatcttcagcatcttttactttcaccagcgtttctgggtgagcaaaaacaggaaggcaaaatgccgcaaaaaag
ggaataagggcgacacggaaatgttgaatactcatactcttcctttttcaatattattgaagcatttatcagggttattgtctcatgagcggatacatatttgaatgtatttagaaaaataaacaa
ataggggttccgcgcacatttccccgaaaagtgccacctgacgtctaagaaaccattattatcatgacattaacctataaaaataggcgtatcacgaggcccc
SEQ ID NO: 39> the sequence of vector pLoxP-mPS-C-EBPα1 harboring wild-type PS transposase fused to C-EBPα 
aacgccagcaacgcggcctttttacggttcctggccttttgctggccttttgctcacatgttctttcctgcgttatcccctgattctgtggataaccgtattaccgcctttgagtgagctgatacc
gctcgccgcagccgaacgaccgagcgcagcgagtcagtgagcgaggaagcggaagagcgcccaatacgcaaaccgcctctccccgcgcgttggccgattcattaatgcagCT
GACTGAGCTCaatgggaaagtaccgttcggagttccgcgttacataacttacggtaaatggcccgcctggctgaccgcccaacgacccccgcccattgacgtcaataatgac
gtatgttcccatagtaacgccaatagggactttccattgacgtcaatgggtggagtatttacggtaaactgcccacttggcagtacatcaagtgtatcatatgccaagtacgccccctattg
acgtcaatgacggtaaatggcccgcctggcattatgcccagtacatgaccttatgggactttcctacttggcagtacatctacgtattagtcatcgctattaccaAAGGATCTGC
GATCGCTCCGGTGCCCGTCAGTGGGCAGAGCGCACATCGCCCACAGTCCCCGAGAAGTTGGGGGGAGGGGTCG
GCAATTGAACGGGTGCCTAGAGAAggtggcgcggggtaaactgggaaagtgatgtcgtgtactggctccgcctttttcccgagggtgggggagaaccgtatat
aagtgcagtagtcgccgtgaacgttctttttcgcaacgggtttgccgccagaacacagctgaagcttcgaggggctcgcatctctccttcacgcgcccgccgccctacctgaggccgc
catccacgccggttgagtcgcgttctgccgcctcccgcctgtggtgcctcctgaactgcgtccgccgtctaggtaagtttaaagctcaggtcgagaccgggcctttgtccggcgctccct
tggagcctacctagactcagccggctctccacgctttgcctgaccctgcttgctcaactctacgtctttgtttcgttttctgttctgcgccgttacagatccaagctgtgaccggcgcctacG
AATTCAATTCATAACTTCGTATAGCATACATTATACGAAGTTATACTAGTgccaccatgACGCAGCAGAAGGTGCTG
GAGCTGACCAGTGACAATGACCGCCTGCGCAAGCGGGTGGAACAGCTGAGCCGCGAACTGGACACGCTGCGG
GGCGGCcctgctgccaagagggtcaagttggacGCCCCCACCAAGAGACACGCCTACAACGCCGAGTTCAAGCTGAAGGCCAT
CAGCCACGCCCAGGAGCACGGCAACAGAGCCGCCGCCAGAGAGTTCAACATCAACGAGAGCATGGTGAGAAA
GTGGAGAAAGTACGAGGACGAGCTGAGACAGGTGAAGAAGACCACCCAGAGCTTCAGAGGCAACAAGGCCA
GGTGGCCCCAGCTGGAGGACAAGGTGGAGCAGTGGGTGGCCGAGCAGAGAGCCGCCAGCAGAAGCGTGAGCA
CCGTGACCATCAGAATGAAGGCCATCGCCCTGGCCAGAGAGCACAACATCAGCGAGTTCAGAGGCGGCCCCA
GCTGGTGCTTCAGATTCATGAAGAGAAGACACCTGAGCATCAGAACCAGAACCACCGTGAGCCAGCAGCTGCC
CGCCGACTACCAGGAGAAGCTGGCCACCTTCAGAACCTACTGCAGAAACAAGATCACCGAGAAGAAGATCCA
GCCCGAGCACATCATCAACATGGACGAGGTGCCCCTGACCTTCGACATCCCCGTGAACAGAACCGTGGACAAG
ACCGGCGCCAGAACCGTGAACATCAGAACCACCGGCAACGAGAAGACCAGCTTCACCGTGGTGCTGGCCTGCC
AGGCCAACGGCCACAAGCTGCCCCCTATGGTGATCTTCAAGAGAAAGACCCTGCCCAAGGAGAACTTCCCCGC
CGGCATCGTGATCAAGGCCAACAGCAAGGGCTGGATGGACGAGGAGAAGATGAGCGAGTGGCTGAGAGAGAT
CTACGTGAAGAGACCCGGCGGCTTCTTCCACACCGCCCCCAGCCTGCTGATCTACGACAGCATGAGAGCCCAC
ATCACCGAGCACGTGAAGAAGCAGGTGAAGCACACCAACAGCGTGCTGGCCGTGATCCCCGGCGGCCTGACC
AAGGAGCTGCAGCCCCTGGACGTGGGCGTGAACAGAGCCTTCAAGGCCAGACTGAGAACCGCCTGGGAGCAG
TGGATGACCGAGGGCGAGCACACCTTCACCAAGACCGGCAGACAGAGAAGAACCACCTACGCCAACATCTGC
AAGTGGATCGTGAACGCCTGGGCCGGCATCAGCGTGACCACCGTGGTGAGAGCCTTCAGAAAGGCCGGCATCG
TGACCGAGCTGCCCGACAACAGCAGCGACACCGACAGCGTGAACGACGACTTCGACAAGACCGAGCCCGGCG
TGCTGGACGCCGCCATCGCCCAGCTGTTCAACAGCGACACCGAGGAGGAGGTGTTCGAGGGCTTCTGATAAGG
ATCCGCTAGCATAACTTCGTATAGCATACATTATACGAAGTTATAACTTGTTTATTGCAGCTTATAATGGTTAC
AAATAAAGCAATAGCATCACAAATTTCACAAATAAAGCATTTTTTTCACTGCATTCTAGTTGTGGTTTGTCCAA
ACTCATCAATGTATCTTATCATGTCTGGATCTTAGTCAGTTACTCGAGACTGGTACCTGActggcgtaatagcgaagaggcc
cgcaccgatcgcccttcccaacagttgcgcagcctgaatggcgaatggaaattgtaagcgttaatattttgttaaaattcgcgttaaatttttgttaaatcagctcattttttaaccaataggcc
gaaatcggcaaaatcccttataaatcaaaagaatagaccgagatagggttgagtgttgttccagtttggaacaagagtccactattaaagaacgtggactccaacgtcaaagggcgaaa
aaccgtctatcagggcgatggcccactacgtgaaccatcaccctaatcaagttttttggggtcgaggtgccgtaaagcactaaatcggaaccctaaagggagcccccgatttagagctt
gacggggaaagccggcgaacgtggcgagaaaggaagggaagaaagcgaaaggagcgggcgctagggcgctggcaagtgtagcggtcacgctgcgcgtaaccaccacaccc
gccgcgcttaatgcgccgctacagggcgcgtcaggtggcacttttcggggaaatgtgcgcggaacccctatttgtttatttttctaaatacattcaaatatgtatccgctcatgagacaata
accctgataaatgcttcaataatattgaaaaaggaagagtatgagtattcaacatttccgtgtcgcccttattcccttttttgcggcattttgccttcctgtttttgctcacccagaaacgctggtg
aaagtaaaagatgctgaagatcagttgggtgcacgagtgggttacatcgaactggatctcaacagcggtaagatccttgagagttttcgccccgaagaacgttttccaatgatgagcact
tttaaagttctgctatgtggcgcggtattatcccgtattgacgccgggcaagagcaactcggtcgccgcatacactattctcagaatgacttggttgagtactcaccagtcacagaaaagc
atcttacggatggcatgacagtaagagaattatgcagtgctgccataaccatgagtgataacactgcggccaacttacttctgacaacgatcggaggaccgaaggagctaaccgcttttt
tgcacaacatgggggatcatgtaactcgccttgatcgttgggaaccggagctgaatgaagccataccaaacgacgagcgtgacaccacgatgcctgtagcaatggcaacaacgttgc
gcaaactattaactggcgaactacttactctagcttcccggcaacaattaatagactggatggaggcggataaagttgcaggaccacttctgcgctcggcccttccggctggctggtttat
tgctgataaatctggagccggtgagcgtgggtctcgcggtatcattgcagcactggggccagatggtaagccctcccgtatcgtagttatctacacgacggggagtcaggcaactatg
gatgaacgaaatagacagatcgctgagataggtgcctcactgattaagcattggtaactgtcagaccaagtttactcatatatactttagattgatttaaaacttcatttttaatttaaaaggatc
taggtgaagatcctttttgataatctcatgaccaaaatcccttaacgtgagttttcgttccactgagcgtcagaccccgtagaaaagatcaaaggatcttcttgagatcctttttttctgcgcgt
aatctgctgcttgcaaacaaaaaaaccaccgctaccagcggtggtttgtttgccggatcaagagctaccaactctttttccgaaggtaactggcttcagcagagcgcagataccaaatac
tgtccttctagtgtagccgtagttaggccaccacttcaagaactctgtagcaccgcctacatacctcgctctgctaatcctgttaccagtggctgctgccagtggcgataagtcgtgtcttac
cgggttggactcaagacgatagttaccggataaggcgcagcggtcgggctgaacggggggttcgtgcacacagcccagcttggagcgaacgacctacaccgaactgagataccta
cagcgtgagctatgagaaagcgccacgcttcccgaagggagaaaggcggacaggtatccggtaagcggcagggtcggaacaggagagcgcacgagggagcttccaggggga
aacgcctggtatctttatagtcctgtcgggtttcgccacctctgacttgagcgtcgatttttgtgatgctcgtcaggggggcggagcctatggaaa
SEQ ID NO: 40> initiation transposon integration plasmidpPG-PGK-NEO (PSTIR0) 
gcccctgcagccgaattatattatttttgccaaataatttttaacaaaagctctgaagtcttcttcatttaaattcttagatgatacttcatctggaaaattgtcccaattagtagcatcacgctgtg
agtaagttctaaaccatttttttattgttgtattatctctaatcttactactcgatgagttttcggtattatctctatttttaacttggagcaggttccattcattgtttttttcatcatagtgaataaaatca
actgctttaacacttgtgcctgaacaccatatccatccggcgtaatacgactcactatagggagagcggccgccagatcttccggatggctcgagtttttcagcaagatggatcctaccgt
attttccgcactataaggcgcacctcgcgagcttggctgcaggtcgtcgaaattctaccgggtaggggaggcgcttttcccaaggcagtctggagcatgcgctttagcagccccgctg
ggcacttggcgctacacaagtggcctctggcctcgcacacattccacatccaccggtaggcgccaaccggctccgttctttggtggccccttcgcgccaccttctactcctcccctagtc
aggaagttcccccccgccccgcagctcgcgtcgtgcaggacgtgacaaatggaagtagcacgtctcactagtctcgtgcagatggacagcaccgctgagcaatggaagcgggtag
gcctttggggcagcggccaatagcagctttgctccttcgctttctgggctcagaggctgggaaggggtgggtccgggggcgggctcaggggcgggctcaggggcggggcgggcg
cccgaaggtcctccggaggcccggcattctgcacgcttcaaaagcgcacgtctgccgcgctgttctcctcttcctcatctccgggcctttcgacctgcagcctgttgacaattaatcatcg
gcatagtatatcggcatagtataatacgacaaggtgaggaactaaaccctgatatgggatcggccattgaacaagatggattgcacgcaggttctccggccgcttgggtggagaggct
attcggctatgactgggcacaacagacaatcggctgctctgatgccgccgtgttccggctgtcagcgcaggggcgcccggttctttttgtcaagaccgacctgtccggtgccctgaatg
aactgcaggacgaggcagcgcggctatcgtggctggccacgacgggcgttccttgcgcagctgtgctcgacgttgtcactgaagcgggaagggactggctgctattgggcgaagtg
ccggggcaggatctcctgtcatctcaccttgctcctgccgagaaagtatccatcatggctgatgcaatgcggcggctgcatacgcttgatccggctacctgcccattcgaccaccaagc
gaaacatcgcatcgagcgagcacgtactcggatggaagccggtcttgtcgatcaggatgatctggacgaagagcatcaggggctcgcgccagccgaactgttcgccaggctcaag
gcgcgcatgcccgacggcgatgatctcgtcgtgacccatggcgatgcctgcttgccgaatatcatggtggaaaatggccgcttttctggattcatcgactgtggccggctgggtgtggc
ggaccgctatcaggacatagcgttggctacccgtgatattgctgaagagcttggcggcgaatgggctgaccgcttcctcgtgctttacggtatcgccgctcccgattcgcagcgcatcg
ccttctatcgccttcttgacgagttcttctgaggggatcaattctctagagctcgctgatcagcctcgactgtgccttctagttgccagccatctgttgtttgcccctcccccgtgccttccttg
accctggaaggtgccactcccactgtcctttcctaataaaatgaggaaattgcatcgcattgtctgagtaggtgtcattctattctggggggtggggtggggcaggacagcaaggggga
ggattgggaagacaatagcaggcatgctggggatcgtcgcgagcggccgcgaattcggtgcgccttatagtgcggaaaatacggtaggatccatctttctagaagatctcctacaatat
tctcagctgccatggaaaatcgatgttcttcttttattctctcaagattttcaggctgtatattaaaacttatattaagaactatgctaaccacctcatcaggaaccgttgtaggtggcgtgggttt
tcttggcaatcgactctcatgaaaactacgagctaaatattcaatatgttcctcttgaccaactttattctgcattttttttgaacgaggtttagagcaagcttcaggaaactgagacaggaattt
tattaaaaatttaaattttgaagaaagttcagggttaatagcatccattttttgctttgcaagttcctcagcattcttaacaaaagacgtctcttttgacatgtttaaagtttaaacctcctgtgtgaa
attgttatccgctcacaattccacacattatacgagccggaagcataaagtgtaaagcctggggtgcctaatgagtgagctaactcacattaattgcgttgcgctcactgccaattgctttcc
agtcgggaaacctgtcgtgccagctgcattaatgaatcggccaacgcgcggggagaggcggtttgcgtattgggcgctcttccgcttcctcgctcactgactcgctgcgctcggtcgtt
cggctgcggcgagcggtatcagctcactcaaaggcggtaatacggttatccacagaatcaggggataacgcaggaaagaacatgtgagcaaaaggccagcaaaaggccaggaac
cgtaaaaaggccgcgttgctggcgtttttccataggctccgcccccctgacgagcatcacaaaaatcgacgctcaagtcagaggtggcgaaacccgacaggactataaagataccag
gcgtttccccctggaagctccctcgtgcgctctcctgttccgaccctgccgcttaccggatacctgtccgcctttctcccttcgggaagcgtggcgctttctcatagctcacgctgtaggta
tctcagttcggtgtaggtcgttcgctccaagctgggctgtgtgcacgaaccccccgttcagcccgaccgctgcgccttatccggtaactatcgtcttgagtccaacccggtaagacacga
cttatcgccactggcagcagccactggtaacaggattagcagagcgaggtatgtaggcggtgctacagagttcttgaagtggtggcctaactacggctacactagaaggacagtatttg
gtatctgcgctctgctgaagccagttaccttcggaaaaagagttggtagctcttgatccggcaaacaaaccaccgctggtagcggtggtttttttgtttgcaagcagcagattacgcgcag
aaaaaaaggatctcaagaagatcctttgatcttttctacggggtctgacgctcagtggaacgaaaactcacgttaagggattttggtcatgagattatcaaaaaggatcttcacctagatcc
ttttaaattaaaaatgaagttttaaatcaatctaaagtatatatgagtaaacttggtctgacagttaccaatgcttaatcagtgaggcacctatctcagcgatctgtctatttcgttcatccatagtt
gcctgactccccgtcgtgtagataactacgatacgggagggcttaccatctggccccagtgctgcaatgataccgcgagacccacgctcaccggctccagatttatcagcaataaacc
agccagccggaagggccgagcgcagaagtggtcctgcaactttatccgcctccatccagtctattaattgttgccgggaagctagagtaagtagttcgccagttaatagtttgcgcaac
gttgttgccattgctacaggcatcgtggtgtcacgctcgtcgtttggtatggcttcattcagctccggttcccaacgatcaaggcgagttacatgatcccccatgttgtgcaaaaaagcggt
tagctccttcggtcctccgatcgttgtcagaagtaagttggccgcagtgttatcactcatggttatggcagcactgcataattctcttactgtcatgccatccgtaagatgcttttctgtgactg
gtgagtactcaaccaagtcattctgagaatagtgtatgcggcgaccgagttgctcttgcccggcgtcaatacgggataataccgcgccacatagcagaactttaaaagtgctcatcattg
gaaaacgttcttcggggcgaaaactctcaaggatcttaccgctgttgagatccagttcgatgtaacccactcgtgcacccaactgatcttcagcatcttttactttcaccagcgtttctgggt
gagcaaaaacaggaaggcaaaatgccgcaaaaaagggaataagggcgacacggaaatgttgaatactcatactcttcctttttcaatattattgaagcatttatcagggttattgtctcatg
agcggatacatatttgaatgtatttagaaaaataaacaaataggggttccgcgcacatttccccgaaaagtgccacctgacgtctaagaaaccattattatcatgacattaacctataaaaat
aggcgtatcacgaggcc
SEQ ID NO: 41> fused PS18 transposase mutant PS18 mRNA 
GGGAAUAAGAAUCAAAUAGAAGUAAGUACACAAGAAACCUCAAGAAGCCACCAUGACGCAGCAGAAGGUG
CUGGAGCUGACCAGUGACAAUGACCGCCUGCGCAAGCGGGUGGAACAGCUGAGCCGCGAACUGGACACGCU
GCGGGGCGGCCCUGCUGCCAAGAGGGUCAAGUUGGACGCCCCCACCAAGAGACACGCCUACAACGCCGAGU
UCAAGCUGAAGGCCAUCAGCCACGCCCAGGAGCACGGCAACAGAGCCGCCGCCAGAGAGUUCAACAUCAAC
GAGAGCAUGGUGAGAAAGUGGAGAAAGUACGAGGACGAGCUGAGACAGGUGAAGAAGACCAAGAAGGCCU
UCAGAGGCAACAAGGCCAGGUGGCCCCAGCUGGAGGACAAGGUGGAGCAGUGGGUGGCCGAGCAGAGAGCC
GCCAGCAGAAGCGUGAGCACCGUGACCAUCAGAAUGAAGGCCAUCGCCCUGGCCAGAGAGCACAACAUCAG
CGAGUUCAGAGGCGGCCCCAGCUGGUGCUUCAGAUUCAUGAAGAGAAGACACCUGAGCAUCAGAACCAGAA
CCACCGUGAGCCAGCAGCUGCCCGCCGACUACCAGGAGAAGCUGGCCACCUUCAGAACCUACUGCAGAAAC
AAGAUCACCGAGAAGAAGAUCCAGCCCGAGCACAUCAUCAACAUGGACGAGGUGCCCCUGACCUUCGACAU
CCCCGUGAACAGAACCGUGGACAAGACCGGCGCCAGAACCGUGAACAUCAGAACCACCGGCAACGAGAAGA
CCAGCUUCACCGUGGUGCUGGCCUGCCAGGCCAACGGCCACAAGCUGCCCCCUAUGGUGAUCUUCAAGAGA
AAGACCCUGCCCAAGGAGAACUUCCCCGCCGGCAUCGUGAUCAAGGCCAACAGCAAGGGCUGGAUGGACGA
GGAGAAGAUGAGCGAGUGGCUGAGAGAGAUCUACGUGAAGAGACCCGGCGGCUUCUUCCACACCGCCCCCA
GCCUGCUGAUCUACGACAGCAUGAGAGCCCACAUCACCGAGCACGUGAAGAAGCAGGUGAAGCACACCAAC
AGCGUGCUGGCCGUGAUCCCCGGCGGCCUGACCAAGGAGCUGCAGCCCCUGGACGUGGGCGUGAACAGAGC
CUUCAAGGCCAGACUGAGAACCGCCUGGGAGCAGUGGAUGACCGAGGGCGAGCACACCUUCACCAAGACCG
GCAGACAGAGAAGAACCACCUACGCCAACAUCUGCAAGUGGAUCGUGAACGCCUGGGCCGGCAUCAGCGUG
ACCACCGUGGUGAGAGCCUUCAGAAAGGCCGGCAUCGUGACCGAGCUGCCCGACAACAGCAGCGACACCGA
CAGCGUGAACGACGACUUCGACAAGACCGAGCCCGGCGUGCUGGACGCCGCCAUCGCCCAGCUGUUCAACA
GCGACACCGAGGAGGAGGUGUUCGAGGGCUUCUGAUAACUUAAGUCUGACACAGACACGUUACUAACACAG
UUAGUAACCACUCUAUCACCGAUAGACACAUGAUAUCCCAUAUCAUAAGCUUAAAAAAAAAAAAAAAAAA
AAAAAAAAAAAAAAAAAAAAAAAAAAAAAAAAAAAAAGGAAAAAAAAAAAAAAAAAAAAAAAAAAAAAAAAAAAAA
AAAAAAAAAAAAAAAAAAAAAAA
SEQ ID NO: 42>pTini-DPS(CES)-dCGEGFP 
TTAGGTTCTGCTTTTCTCAATAACCTCTTTGGCTGAGGTGTATTCTTCCAGTGGTTCTTCTGCCATTGCCTGTTCA
ATCACTCTCAGGATTGGTTCTGGAATATCTGATGGGGTGCTCAGCAGATATTCAATGGCTTCCAGGGTTGGTTC
ATCAATATCTGAGGACACTTTTCTGATGATTTCCAGTTTTCTTTCATCAGTGCTCATGATGGCCCTCCTATAGTG
AGTTGTATTATACTATGCAGATATACTATGCCAATGTTTAATTGTCAACCTGCAGGAATATATACCGTATTTTCC
GCACTATAAGGCGCACCCCGTATTTTCCGCACTATAAGGCGCACCTCTAGACATCTTGGACCATTAGCTCCACA
GGTATCTTCTTCCCTCTAGTGGTCATAACAGCAGCTTCAGCTACCTCTCTCTCTGCTGCCAGTAGAGGGCACACT
TACTTTACTCATTCCTTTAATGGTCTTTTCTGGACACCACTAGGGGTCAGAAGTAGTTCATCAAACTTTCTTCCC
TCCCTAGAGTCAATGGGAAAAACCCATTGGAGCCAAGTACACTGACTCAATAGGGACTTTCCATTGGGTTTTGC
CCAGTACATAAGGTCAATAGGGGGTGAGTCAACAGGAAAGTCCCATTGGAGCCAAGTACATTGAGTCAATAG
GGACTTTCCAATGGGTTTTGCCCAGTACATAAGGTCAATGGGAGGTAAGCCAATGGGTTTTTCCCATTACTGAC
ATGTATACTGAGTCATTAGGGACTTTCCAATGGGTTTTGCCCAGTACATAAGGTCAATAGGGGTGAATCAACA
GGAAAGTCCCATTGGAGCCAAGTACACTGAGTCAATAGGGACTTTCCATTGGGTTTTGCCCAGTACAAAAGGT
CAATAGGGGGTGAGTCAATGGGTTTTTCCCATTATTGGCACATACATAAGGTCAATAGGGGTGACTAGTGGAG
AAGAGCATGCTTGAGGGCTGAGTGCCCCTCAGTGGGCAGAGAGCACATGGCCCACAGTCCCTGAGAAGTTGGG
GGGAGGGGTGGGCAATTGAACTGGTGCCTAGAGAAGGTGGGGCTTGGGTAAACTGGGAAAGTGATGTGGTGT
ACTGGCTCCACCTTTTTCCCCAGGGTGGGGGAGAACCATATATAAGTGCAGTAGTCTCTGTGAACATTCAAGCT
TCTGCCTTCTCCCTCCTGTGAGTTTGgtaagtcactgactgtctatgcctgggaaagggtgggcaggaggtggggcagtgcaggaaaagtggcactgtgaac
cctgcagccctagacaattgtactaaccttcttctctttcctctcctgacaggGGTCTCCAATTGCCACCATGGTGAGCAAGGGAGAGGAGCTGT
TCACTGGGGTGGTGCCCATCCTGGTGGAGCTGGATGGAGATGTGAATGGCCACAAGTTCTCTGTGTCTGGAGA
GGGAGAGGGAGATGCCACCTATGGCAAGCTGACCCTGAAGTTCATCTGCACCACAGGCAAGCTGCCTGTGCCC
TGGCCCACCCTGGTGACCACCCTGACCTATGGAGTGCAGTGCTTCAGCAGATACCCTGACCACATGAAGCAGC
ATGACTTCTTCAAGTCTGCCATGCCTGAGGGCTATGTCCAGGAGAGAACCATCTTCTTCAAGGATGATGGCAAC
TACAAGACCAGAGCTGAGGTGAAGTTTGAGGGAGACACCCTGGTGAACAGAATTGAGCTGAAGGGCATTGAC
TTCAAGGAGGATGGCAACATCCTGGGGCACAAGCTGGAGTACAACTACAACAGCCACAATGTCTATATCATGG
CTGACAAGCAGAAGAATGGCATCAAGGTGAACTTCAAGATCAGACACAACATTGAGGATGGCTCTGTGCAGCT
GGCTGACCACTACCAGCAGAACACCCCCATTGGAGATGGCCCTGTGCTGCTGCCTGACAACCACTACCTGAGC
ACCCAGTCTGCCCTGAGCAAGGACCCCAATGAGAAGAGAGATCACATGGTCCTGCTGGAGTTTGTGACAGCTG
CTGGGATCACTCTGGGCATGGATGAGCTGTACAAGTAATGAGGATCCTAAGATACATTGATGAGTTTGGACAA
ACCACAACTAGAATGCAGTGAAAAAAATGCTTTATTTGTGAAATTTGTGATGCTATTGCTTTATTTGTAACCAT
TATAAGCTGCAATAAACAAGTTTAGGGAGGGAAGAAAGTTTGATGAACTACTTCTGACCCCTAGTGGTGTCCA
GAAAAGACCATTAAAGGAATGAGTAAAGTAAGTGTGCCCTCTACTGGCAGCAGAGAGAGAGGTAGCTGAAGC
TGCTGTTATGACCACTAGAGGGAAGAAGATACCTGTGGAGCTAATGGTCCAAGATGCTTAAGGGTGCGCCTTA
TAGTGCGGAAAATACGGGGTGCGCCTTATAGTGCGGAAAATACGGTATATAAGAGCTCAATCAGCAGTTCAAC
CTGTTGATAGTATGTACTAAGCTCTCATGTTTAATGTACTAAGCTCTCATGTTTAATGAACTAAACCCTCATGGC
TAATGTACTAAGCTCTCATGGCTAATGTACTAAGCTCTCATGTTTCATGTACTAAGCTCTCATGTTTGAACAATA
AAATTAATATAAATCAGCAACTTAAATAGCCTCTAAGGTTTTAAGTTTTATAAGAAAAAAAAGAATATATAAG
GCTTTTAAAGGTTTTAAGGTTTCCTAGCT
SEQ ID NO: 43>PC23S-dCGEGFP 
tcgacagcactagtgcaggatccTTACTTGTACAGCTCATCCATGCCCAGAGTGATCCCAGCAGCTGTCACAAACTCCAGCA
GGACCATGTGATCTCTCTTCTCATTGGGGTCCTTGCTCAGGGCAGACTGGGTGCTCAGGTAGTGGTTGTCAGGC
AGCAGCACAGGGCCATCTCCAATGGGGGTGTTCTGCTGGTAGTGGTCAGCCAGCTGCACAGAGCCATCCTCAA
TGTTGTGTCTGATCTTGAAGTTCACCTTGATGCCATTCTTCTGCTTGTCAGCCATGATATAGACATTGTGGCTGT
TGTAGTTGTACTCCAGCTTGTGCCCCAGGATGTTGCCATCCTCCTTGAAGTCAATGCCCTTCAGCTCAATTCTGT
TCACCAGGGTGTCTCCCTCAAACTTCACCTCAGCTCTGGTCTTGTAGTTGCCATCATCCTTGAAGAAGATGGTTC
TCTCCTGGACATAGCCCTCAGGCATGGCAGACTTGAAGAAGTCATGCTGCTTCATGTGGTCAGGGTATCTGCTG
AAGCACTGCACTCCATAGGTCAGGGTGGTCACCAGGGTGGGCCAGGGCACAGGCAGCTTGCCTGTGGTGCAGA
TGAACTTCAGGGTCAGCTTGCCATAGGTGGCATCTCCCTCTCCCTCTCCAGACACAGAGAACTTGTGGCCATTC
ACATCTCCATCCAGCTCCACCAGGATGGGCACCACCCCAGTGAACAGCTCCTCTCCCTTGCTCACCATggtggcaatt
cgtaggcgccggtcacagcttggatctgtaacggcgcagaacagaaaacgaaacaaagacgtagagttgagcaagcagggtcaggcaaagcgtggagagccggctgagtctagg
taggctccaagggagcgccggacaaaggcccggtctcgacctgagctATAAacttacctagacggcggacgcagttcaggaggcaccacaggcgggaggcggcagaacgc
gactcaaccggcgtggatggcggcctcaggtagggcggcgggcgcgtgaaggagagatgcgagcccctcgaagcttcagctgtgttctggcggcaaacccgttgcgaaaaagaa
cgttcacggcgactactgcacttatatacggttctcccccaccctcgggaaaaaggcggagccagtacacgacatcactttcccagtttaccccgcgccaccttctctaggcaccggttc
aattgccgacccctccccccaacttctcggggactgtgggcgatgtgcgctctgcccactgacgggcaccggagcgatCGCAGATCCTTGGTTGCTGACTAA
TTGAGATGCATGCTTTGCATACTTCTGCCTGCTGGGGAGCCTGGGGACTTTCCACACCTGGTTGCTGACTAATT
GAGATGCATGCTTTGCATACTTCTGCCTGCTGGGGAGCCTGGGGACTTTCCACACCGCTAGCTAGGGAGGGAA
GAAAGTTTGATGAACTACTTCTGACCCCTAGTGGTGTCCAGAAAAGACCATTAAAGGAATGAGTAAAGTAAGT
GTGCCCTCTACTGGCAGCAGAGAGAGAGGTAGCTGAAGCTGCTGTTATGACCACTAGAGGGAAGAAGATACCT
GTGGAGCTAATGGTCCAAGATGTctagacatgcgtcaattttacgcagactatctttctagggttaatcgaagagcaaaaggccagcaaaaggccaggaaccgt
aaaaaggccgcgttgctggcgtttttccataggctccgcccccctgacgagcatcacaaaaatcgacgctcaagtcagaggtggcgaaacccgacaggactataaagataccaggc
gtttccccctggaagctccctcgtgcgctctcctgttccgaccctgccgcttacgggatacctgtccgcctttctcccttcgggaagcgtggcgctttctcatagctcacgctgtaggtatct
cagttcggtgtaggtcgttcgctccaagctgggctgtgtgcacgaaccccccgttcagcccgaccgctgcgccttatccggtaactatcgtcttgagtccaacccggtaagacacgactt
atcgccactggcagcagCCACtggtaacaggattagcagagcgaggtatgtaggcggtgctacagagttcttgaagtggtggcctaactacggctacactagaagaacagtatttg
gtatctgcgctctgctgaagccagttaccttcggaaaaagagttggtagctcttgatccggcaaacaaaccaccgctggtagcggtggtttttttgtttgcaagcagcagattacgcgcag
aaaaaaaggatctcaagaagatcctttgatcttttctacggggtctgacgctcagtggaacgaaaactcacggggattttggtcatgccgtctcagaagaactcgtcaagaaggcgatag
aaggcgatgcgctgcgaatcgggagcggcgataccgtaaagcacgaggaagcggtcagcccattcgccgccaagctcttcagcaatatcacgggtagccaacgctatgtcctgata
gcggtccgccacacccagccggccacagtcgatgaatccagaaaagcggccattttccaccatgatattcggcaagcaggcatcgccatgggtcacgacgagatcctcgccgtcgg
 
gcatgctcgccttgagcctggcgaacagttcggctggcgcgagcccctgatgctcttcgtccagatcatcctgatcgacaagaccggcttccatccgagtacgtgctctctcgatgcga
tgtttcgcttggtggtcgaatgggcaggtagccggatcaagcgtatgcagccgccgcattgcatcagccatgatggatactttctcggcaggagcaaggtgagatgacaggagatcct
gccccggcacttcgcccaatagcagccagtcccttcccgcttcagtgacaacgtcgagtacagctgcgcaaggaacgcccgtcgtggccagccacgatagccgcgctgcctcgtctt
gcagttcattcagggcaccggacaggtcggtcttgacaaaaagaaccgggcgcccctgcgctgacagccggaacacggcggcatcagagcagccgattgtctgttgtgcccagtca
tagccgaatagcctctccacccaagcggccggagaacctgcgtgcaatccatcttgttcaatcataatattattgaagcatttatcagggttcgtctcgtcccggtctcctcccatgcatgg
gCGCGCCTTAACCCTAGAAAGATAATCATATTGTGACGTACGTTAAAGATAATCATGCGTAAAATTGACGCATG
CTTAAGAGAGAAATGTTCTGGCACCTGCACTTGCACTGGGGACAGCCTATTTTGCTAGTTTGTTTTGTTTCGTTT
TGTTTTGATGGAGAGCGTATGTTAGTCATACTGATTCACACAAAAAACCAACACACAGATGTAATGAAAATAA
AGATATTTTATTGATATCCCATAGAGCCCACCGCATCCCCAGCATGCCTGCTATTGTCATCCCAATCCTCCCCCT
TGCTGTCCTGCCCCACCCCACCCCCCAGAATAGAATGACACCTACTCAGACAATGCGATGCAATTTCCTCATTT
TATTAGGAAAGGACAGTGGGAGTGGCACCTTCCAGGGTCAAGGAAGGCACGGGGGAGGGGCAAACAACAGAT
GGCTGGCAACTAGAAGGCACAGG
SEQ ID NO: 44>pC23S-1444-8E 
aattcgtaggcgccggtcacagcttggatctgtaacggcgcagaacagaaaacgaaacaaagacgtagagttgagcaagcagggtcaggcaaagcgtggagagccggctgagtct
aggtaggctccaagggagcgccggacaaaggcccggtctcgacctgagctATAAacttacctagacggcggacgcagttcaggaggcaccacaggcgggaggcggcagaa
cgcgactcaaccggcgtggatggcggcctcaggtagggcggcgggcgcgtgaaggagagatgcgagcccctcgaagcttcagctgtgttctggcggcaaacccgttgcgaaaaa
gaacgttcacggcgactactgcacttatatacggttctcccccaccctcgggaaaaaggcggagccagtacacgacatcactttcccagtttaccccgcgccaccttctctaggcaccc
gttcaattgccgacccctccccccaacttctcggggactgtgggcgatgtgcgctctgcccactgacgggcaccggagcgatCGCAGATCCTTGGTTGCTGACT
AATTGAGATGCATGCTTTGCATACTTCTGCCTGCTGGGGAGCCTGGGGACTTTCCACACCTGGTTGCTGACTAA
TTGAGATGCATGCTTTGCATACTTCTGCCTGCTGGGGAGCCTGGGGACTTTCCACACCGCTAGCTAGGGAGGGA
AGAAAGTTTGATGAACTACTTCTGACCCCTAGTGGTGTCCAGAAAAGACCATTAAAGGAATGAGTAAAGTAAG
TGTGCCCTCTACTGGCAGCAGAGAGAGAGGTAGCTGAAGCTGCTGTTATGACCACTAGAGGGAAGAAGATACC
TGTGGAGCTAATGGTCCAAGATGTctagacatgcgtcaattttacgcagactatctttctagggttaatcgaagagcaaaaggccagcaaaaggccaggaaccg
taaaaaggccgcgttgctggcgtttttccataggctccgcccccctgacgagcatcacaaaaatcgacgctcaagtcagaggtggcgaaacccgacaggactataaagataccaggc
gtttccccctggaagctccctcgtgcgctctcctgttccgaccctgccgcttacgggatacctgtccgcctttctcccttcgggaagcgtggcgctttctcatagctcacgctgtaggtatct
cagttcggtgtaggtcgttcgctccaagctgggctgtgtgcacgaaccccccgttcagcccgaccgctgcgccttatccggtaactatcgtcttgagtccaacccggtaagacacgactt
atcgccactggcagcagCCACtggtaacaggattagcagagcgaggtatgtaggcggtgctacagagttcttgaagtggtggcctaactacggctacactagaagaacagtatttg
gtatctgcgctctgctgaagccagttaccttcggaaaaagagttggtagctcttgatccggcaaacaaaccaccgctggtagcggtggtttttttgtttgcaagcagcagattacgcgcag
aaaaaaaggatctcaagaagatcctttgatcttttctacggggtctgacgctcagtggaacgaaaactcacggggattttggtcatgccgtctcagaagaactcgtcaagaaggcgatag
aaggcgatgcgctgcgaatcgggagcggcgataccgtaaagcacgaggaagcggtcagcccattcgccgccaagctcttcagcaatatcacgggtagccaacgctatgtcctgata
gcggtccgccacacccagccggccacagtcgatgaatccagaaaagcggccattttccaccatgatattcggcaagcaggcatcgccatgggtcacgacgagatcctcgccgtcgg
gcatgctcgccttgagcctggcgaacagttcggctggcgcgagcccctgatgctcttcgtccagatcatcctgatcgacaagaccggcttccatccgagtacgtgctctctcgatgcga
tgtttcgcttggtggtcgaatgggcaggtagccggatcaagcgtatgcagccgccgcattgcatcagccatgatggatactttctcggcaggagcaaggtgagatgacaggagatcct
gccccggcacttcgcccaatagcagccagtcccttcccgcttcagtgacaacgtcgagtacagctgcgcaaggaacgcccgtcgtggccagccacgatagccgcgctgcctcgtctt
gcagttcattcagggcaccggacaggtcggtcttgacaaaaagaaccgggcgcccctgcgctgacagccggaacacggcggcatcagagcagccgattgtctgttgtgcccagtca
tagccgaatagcctctccacccaagcggccggagaacctgcgtgcaatccatcttgttcaatcataatattattgaagcatttatcagggttcgtctcgtcccggtctcctcccatgcatgg
gCGCGCCTTAACCCTAGAAAGATAATCATATTGTGACGTACGTTAAAGATAATCATGCGTAAAATTGACGCATG
CTTAAGAGAGAAATGTTCTGGCACCTGCACTTGCACTGGGGACAGCCTATTTTGCTAGTTTGTTTTGTTTCGTTT
TGTTTTGATGGAGAGCGTATGTTAGTCATACTGATTCACACAAAAAACCAACACACAGATGTAATGAAAATAA
AGATATTTTATTGATATCCCATAGAGCCCACCGCATCCCCAGCATGCCTGCTATTGTCATCCCAATCCTCCCCCT
TGCTGTCCTGCCCCACCCCACCCCCCAGAATAGAATGACACCTACTCAGACAATGCGATGCAATTTCCTCATTT
TATTAGGAAAGGACAGTGGGAGTGGCACCTTCCAGGGTCAAGGAAGGCACGGGGGAGGGGCAAACAACAGAT
GGCTGGCAACTAGAAGGCACAGGtcgacttatcagcgagggggcagggcctgcatgtgaagggcgtcgtaggtgtccttggtggctgtactgagaccctggta
aaggccatcgtgccccttgcccctccggcgctcgcctttcatcccaatctcactgtaggcctccgccatcttatctttctgcagttcattgtacaggccttcctgagggttcttccttctcggct
ttccccccatctcagggtcccggccacgtctcttgtccaaaacatcgtactcctctcttcgtcctagattgagctcgttatagagctggttctggccctgctggtacgcgggggcgtctgcg
ctcctgctgaacttcactctggagcgataggctgcgaagtcgcgtggtggggcatagggctggtaatgcttgcgggtgggcccggggcggcggggagtcatgttcatgtagtcactgt
gcaggagcctgctcctcttactcctcacccagaaaataataaaggccactgttactagcaagctatagcaagccaggactccaccaaccaccaccagcacccaaaagggATCAC
AGGCGAAGTCCAGCCCCCTCGTGTGCACTGCGCCCCCCGCCGCTGGCCGGCACGCCTCTGGGCGCAGGGACAG
GGGCTGCGACGCGATGGTGGGCGCCGGTGTTGGTGGTCGCGGCGCTGGCGTCGTGGTGGGCTTCGCTGGCAGG
AAGACCGGCACGAAGCTGCTCACGGTCACCTGGGTGCCCTGGCCCCAGTCGGGGTCGTAGTCGTTGTAGTCCTT
GTTGCTGGCGGCGCAGTAGTACATGCCGGTGTCCTCGGGCTTCAGCATGTTCATCTGCAGGTACAGGGTGTTCT
TGGCGTTGTCGCGGCTGATGGTGAAGCGGCCCTTCACGCTGTCGGCGTAGTAGGTCTTGCCGCCGCTGTTGATG
GTGGCCACCAGCTGGCGCTCCTTCTCGCTGATCTGGCGGTACCAGCCCATGTAGGCGATGCTGATGATGGGGGT
GCTGGCGGCGCAGCTCAGGCGCAGGCTGCCGCCGGCCTGCACGAAGCCGCCGCCGCTCTCCACCACCTGCACC
TGGCTCGGCCTGGCGGCGTGGAGCAGCAAGGCCAGCGGCAGGAGCAAGGCGGTCACTGGTAAGGCCATGGTG
GCG
SEQ ID NO: 45>pC24S-1194 
tcgaCCTGTGCCTTCTAGTTGCCAGCCATCTGTTGTTTGCCCCTCCCCCGTGCCTTCCTTGACCCTGGAAGGTGCCA
CTCCCACTGTCCTTTCCTAATAAAATGAGGAAATTGCATCGCATTGTCTGAGTAGGTGTCATTCTATTCTGGGG
GGTGGGGTGGGGCAGGACAGCAAGGGGGAGGATTGGGATGACAATAGCAGGCATGCTGGGGATGCGGTGGGC
TCTATGGGATATCAATAAAATATCTTTATTTTCATTACATCTGTGTGTTGGTTTTTTGTGTGAATCAGTATGACT
AACATACGCTCTCCATCAAAACAAAACGAAACAAAACAAACTAGCAAAATAGGCTGTCCCCAGTGCAAGTGC
AGGTGCCAGAACATTTCTCTCTTAAGCATGCGTCAATTTTACGCATGATTATCTTTAACGTACGTCACAATATG
ATTATCTTTCTAGGGTTAAGGCGCGcccatgcatgggaggagaccgggacgagacgaaccctgataaatgcttcaataatattatgattgaacaagatggattg
cacgcaggttctccggccgcttgggtggagaggctattcggctatgactgggcacaacagacaatcggctgctctgatgccgccgtgttccggctgtcagcgcaggggcgcccggtt
ctttttgtcaagaccgacctgtccggtgccctgaatgaactgcaagacgaggcagcgcggctatcgtggctggccacgacgggcgttccttgcgcagctgtactcgacgttgtcactga
 
agcgggaagggactggctgctattgggcgaagtgccggggcaggatctcctgtcatctcaccttgctcctgccgagaaagtatccatcatggctgatgcaatgcggcggctgcatac
gcttgatccggctacctgcccattcgaccaccaagcgaaacatcgcatcgagcgagcacgtactcggatggaagccggtcttgtcgatcaggatgatctggacgaagagcatcaggg
gctcgcgccagccgaactgttcgccaggctcaaggcgagcatgcccgacggcgaggatctcgtcgtgacccatggcgatgcctgcttgccgaatatcatggtggaaaatggccgctt
ttctggattcatcgactgtggccggctgggtgtggcggaccgctatcaggacatagcgttggctacccgtgatattgctgaagagcttggcggcgaatgggctgaccgcttcctcgtgc
tttacggtatcgccgctcccgattcgcagcgcatcgccttctatcgccttcttgacgagttcttctgagacggcatgaccaaaatcccttaacgtgagttttcgttccactgagcgtcagaccccg
tagaaaagatcaaaggatcttcttgagatcctttttttctgcgcgtaatctgctgcttgcaaacaaaaaaaccaccgctaccagcggtggtttgtttgccggatcaagagctaccaactctttt
tccgaaggtaactggcttcagcagagcgcagataccaaatactgttcttctagtgtagccgtagttaggccaccacttcaagaactctgtagcaccgcctacatacctcgctctgctaatc
ctgttaccaGTGGctgctgccagtggcgataagtcgtgtcttaccgggttggactcaagacgatagttaccggataaggcgcagcggtcgggctgaacggggggttcgtgcacac
agcccagcttggagcgaacgacctacaccgaactgagatacctacagcgtgagctatgagaaagcgccacgcttcccgaagggagaaaggcggacaggtatccggtaagcggca
gggtcggaacaggagagcgcacgagggagcttccagggggaaacgcctggtatctttatagtcctgtcgggtttcgccacctctgacttgagcgtcgatttttgtgatgctcgtcaggg
gggcggagcctatggaaaaacgccagcaacgcggcctttttacggttcctggccttttgctggccttttgctcttcgattaaccctagaaagatagtctgcgtaaaattgacgcatgtctag
ACATCTTGGACCATTAGCTCCACAGGTATCTTCTTCCCTCTAGTGGTCATAACAGCAGCTTCAGCTACCTCTCTC
TCTGCTGCCAGTAGAGGGCACACTTACTTTACTCATTCCTTTAATGGTCTTTTCTGGACACCACTAGGGGTCAG
AAGTAGTTCATCAAACTTTCTTCCCTCCCTAGCTAGCgacattgattattgactagttattaatagtaatcaattacggggtcattagttcatagcccatat
atggagttccgcgttacataacttacggtaaatggcccgcctggctgaccgcccaacgacccccgcccattgacgtcaataatgacgtatgttcccatagtaacgccaatagggactttc
cattgacgtcaatgggtggagtatttacggtaaactgcccacttggcagtacatcaagtgtatcatatgccaagtacgccccctattgacgtcaatgacggtaaatggcccgcctggcatt
atgcccagtacatgaccttatgggactttcctacttggcagtacatctacgtattagtcatcgctattaccatgcgatCGAAAAGTGCCTTCAAAGAATCCCACC
AGAATGGCACAGGTGGGCATAATGGGTCTGTCTCATCGTCAAAGGACCCAAGGAGTCTAAAGGAAACTCTAAC
TACAACACCCAAATGCCACAAAACCTTAGTTATATATACAAACTATCATCCCTGCCTATCTGTCACCATCTCAT
CTATAAAAACTTGTGAAAATACGTAATCCTCAGGAGACTTCAATTAGGTATAAATACCAGCAGCCAGAGGAGG
TGCAGAagcttcgaggggctcgcatctctccttcacgcgcccgccgccctacctgaggccgccatccacgccggttgagtcgcgttctgccgcctcccgcctgtggtgcctcct
gaactgcgtccgccgtctaggtaagtttaaagctcaggtcgagaccgggcctttgtccggcgctcccttggagcctacctagactcagccggctctccacgctttgcctgaccctgcttg
ctcaactctacgtctttgtttcgttttctgttctgcgccgttacagatccaagctgtgaccggcgcctacgAATTCGCCACCATGGAGGCCCCAGCTCAGCTG
CTGTTCCTGCTGCTGCTGTGGCTGCCAGACACCACAGGAGAGGTGCAGCTGGTGGAGAGCGGCGGCGGCCTGG
TGCAGCCCGGCGGCAGCCTGAGACTGAGCTGCGCCGCCAGCGGCAGACCCTTCAGCATCTACGACATGGGCTG
GTTCAGACAGGCCCCCGACAAGGAGAGAGAGAGCGTGGCCGTGATCAACCTGGCCAGAGGCAACACCTACTA
CGCCGACAGCGTGAAGGGCAGATTCACCATCAGCAGAGACAACGCCAAGAACACCGTGTACCTCCAGATGAA
CAGCCTGAGAGCCGAGGACACCGCCGTGTACAGCTGCGGCGTGGACAGAAGACAGTACGGCCTGGGCATCCC
CCCCCTGGCCGACCACTGGGGCCAGGGCACCCAGGTGACCGTGAGCAGCTGATAAG
SEQ ID NO: 46>pTini-PS(DTS)-1444-8E 
CTTAAGGTGCGCCTTATAGTGCGGAAAATACGGTATAAGATCTAATCAGCAGTTCAACCTGTTGATAGTATGTA
CTAAGCTCTCATGTTTAATGTACTAAGCTCTCATGTTTAATGAACTAAACCCTCATGGCTAATGTACTAAGCTCT
CATGGCTAATGTACTAAGCTCTCATGTTTCATGTACTAAGCTCTCATGTTTGAACAATAAAATTAATATAAATC
AGCAACTTAAATAGCCTCTAAGGTTTTAAGTTTTATAAGAAAAAAAAGAATATATAAGGCTTTTAAAGGTTTTA
AGGTTTCCTAGCTTTAGGTTCTGCTTTTCTCAATAACCTCTTTGGCTGAAGTGTATTCCTCAAGAGGTTCTTCTG
CCATTGCTTGTTCAATAACTCTCAGTATTGGTTCTGGGATATCTGAAGGGGTTGATAACAAATATTCAATTGCTT
CCAGAGTAGGCTCATCAATATCAGAACTCACTTTTCTGATGATCTCCAATTTTCTTTCATCTGTTGACATGATGG
CCCTCCTATAGTGAGTTGTATTATACTATGCAGATATACTATGCCAATGTTTAATTGTCAACCTGCAGGAATAT
ACCGTATTTTCCGCACTATAAGGCGCACCTctagACATCTTGGACCATTAGCTCCACAGGTATCTTCTTCCCTCTA
GTGGTCATAACAGCAGCTTCAGCTACCTCTCTCTCTGCTGCCAGTAGAGGGCACACTTACTTTACTCATTCCTTT
AATGGTCTTTTCTGGACACCACTAGGGGTCAGAAGTAGTTCATCAAACTTTCTTCCCTCCCTAGCTAGCGGTGT
GGAAAGTCCCCAGGCTCCCCAGCAGGCAGAAGTATGCAAAGCATGCATCTCAATTAGTCAGCAACCAGGTGTG
GAAAGTCCCCAGGCTCCCCAGCAGGCAGAAGTATGCAAAGCATGCATCTCAATTAGTCAGCAACCAAGGATCT
GCGatcgctccggtgcccgtcagtgggcagagcgcacatcgcccacagtccccgagaagttggggggaggggtcggcaattgaacgggtgcctagagaaggtggcgcggggt
aaactgggaaagtgatgtcgtgtactggctccgcctttttcccgagggtgggggagaaccgtatataagtgcagtagtcgccgtgaacgttctttttcgcaacgggtttgccgccagaac
acagctgaagcttcgaggggctcgcatctctccttcacgcgcccgccgccctacctgaggccgccatccacgccggttgagtcgcgttctgccgcctcccgcctgtggtgcctcctga
actgcgtccgccgtctaggtaagtTTATagctcaggtcgagaccgggcctttgtccggcgctcccttggagcctacctagactcagccggctctccacgctttgcctgaccctgctt
gctcaactctacgtctttgtttcgttttctgttctgcgccgttacagatccaagctgtgaccggcGcctacgaattcGCCACCATGGCCTTACCAGTGACCGCCT
TGCTCCTGCCGCTGGCCTTGCTGCTCCACGCCGCCAGGCCGAGCCAGGTGCAGGTGGTGGAGAGCGGCGGCGG
CTTCGTGCAGGCCGGCGGCAGCCTGCGCCTGAGCTGCGCCGCCAGCACCCCCATCATCAGCATCGCCTACATG
GGCTGGTACCGCCAGATCAGCGAGAAGGAGCGCCAGCTGGTGGCCACCATCAACAGCGGCGGCAAGACCTAC
TACGCCGACAGCGTGAAGGGCCGCTTCACCATCAGCCGCGACAACGCCAAGAACACCCTGTACCTGCAGATGA
ACATGCTGAAGCCCGAGGACACCGGCATGTACTACTGCGCCGCCAGCAACAAGGACTACAACGACTACGACCC
CGACTGGGGCCAGGGCACCCAGGTGACCGTGAGCAGCTTCGTGCCGGTCTTCCTGCCAGCGAAGCCCACCACG
ACGCCAGCGCCGCGACCACCAACACCGGCGCCCACCATCGCGTCGCAGCCCCTGTCCCTGCGCCCAGAGGCGT
GCCGGCCAGCGGCGGGGGGCGCAGTGCACACGAGGGGGCTGGACTTCGCCTGTGATcccttttgggtgctggtggtggttggtg
gagtcctggcttgctatagcttgctagtaacagtggcctttattattttctgggtgaggagtaagaggagcaggctcctgcacagtgactacatgaacatgactccccgccgccccgggc
ccacccgcaagcattaccagccctatgccccaccacgcgacttcgcagcctatcgctccagagtgaagttcagcaggagcgcagacgcccccgcgtaccagcagggccagaacc
agctctataacgagctcaatctaggacgaagagaggagtacgatgttttggacaagagacgtggccgggaccctgagatggggggaaagccgagaaggaagaaccctcaggaag
gcctgtacaatgaactgcagaaagataagatggcggaggcctacagtgagattgggatgaaaggcgagcgccggaggggcaaggggcacgatggcctttaccagggtctcagta
cagccaccaaggacacctacgacgcccttcacatgcaggccCTGCCCCCTCGCTGATAAGTCgaCCTGTGCCTTCTAGTTGCCAGCCATC
TGTTGTTTGCCCCTCCCCCGTGCCTTCCTTGACCCTGGAAGGTGCCACTCCCACTGTCCTTTCCTAATAAAATGA
GGAAATTGCATCGCATTGTCTGAGTAGGTGTCATTCTATTCTGGGGGGTGGGGTGGGGCAGGACAGCAAGGGG
GAGGATTGGGATGACAATAGCAGGCATGCTGGGGATGCGGTGGGCTCTATGGGATATCAATAAAATATCTTTA
TTTTCATTACATCTGTGTGTTGGTTTTTTGTGTGAATCAGTATGACTAACATACGCTCTCCATCAAAACAAAACG
AAACAAAACAAACTAGCAAAATAGGCTGTCCCCAGTGCAAGTGCAGGTGCCAGAACATTTCTCT
SEQ ID NO: 47>pTini-PS(CIT)-1194 
AATCAGCAGTTCAACCTGTTGATAGTATGTACTAAGCTCTCATGTTTAATGTACTAAGCTCTCATGTTTAATGA
ACTAAACCCTCATGGCTAATGTACTAAGCTCTCATGGCTAATGTACTAAGCTCTCATGTTTCATGTACTAAGCT
CTCATGTTTGAACAATAAAATTAATATAAATCAGCAACTTAAATAGCCTCTAAGGTTTTAAGTTTTATAAGAAA
AAAAAGAATATATAAGGCTTTTAAAGGTTTTAAGGTTTCCTAGCTTTAGGTTCTGCTTTTCTCAATAACCTCTTT
GGCTGAAGTGTATTCCTCAAGAGGTTCTTCTGCCATTGCTTGTTCAATAACTCTCAGTATTGGTTCTGGGATATC
TGAAGGGGTTGATAACAAATATTCAATTGCTTCCAGAGTAGGCTCATCAATATCAGAACTCACTTTTCTGATGA
TCTCCAATTTTCTTTCATCTGTTGACATGATGGCCCTCCTATAGTGAGTTGTATTATACTATGCAGATATACTAT
GCCAATGTTTAATTGTCAACCTGCAGGAATATACCGTATTTTCCGCACTATAAGGCGCACCTctagACATCTTGGA
CCATTAGCTCCACAGGTATCTTCTTCCCTCTAGTGGTCATAACAGCAGCTTCAGCTACCTCTCTCTCTGCTGCCA
GTAGAGGGCACACTTACTTTACTCATTCCTTTAATGGTCTTTTCTGGACACCACTAGGGGTCAGAAGTAGTTCA
TCAAACTTTCTTCCCTCCCTAGCTAGCGACATTGATTATTGACTAGttattaatagtaatcaattacggggtcattagttcatagcccatatatgg
agttccgcgttacataacttacggtaaatggcccgcctggctgaccgcccaacgacccccgcccattgacgtcaataatgacgtatgttcccatagtaacgccaatagggactttccatt
gacgtcaatgggtggagtatttacggtaaactgcccacttggcagtacatcaagtgtatcatatgccaagtacgccccctattgacgtcaatgacggtaaatggcccgcctggcattatg
cccagtacatgaccttatgggactttcctacttggcagtacatctacgtattagtcatcgctattaccatgcgatCGAAAAGTGCCTTCAAAGAATCCCACCAG
AATGGCACAGGTGGGCATAATGGGTCTGTCTCATCGTCAAAGGACCCAAGGAGTCTAAAGGAAACTCTAACTA
CAACACCCAAATGCCACAAAACCTTAGTTATATATACAAACTATCATCCCTGCCTATCTGTCACCATCTCATCT
ATAAAAACTTGTGAAAATACGTAATCCTCAGGAGACTTCAATTAGGTATAAATACCAGCAGCCAGAGGAGGTG
CAGAagcttcgaggggctcgcatctctccttcacgcgcccgccgccctacctgaggccgccatccacgccggttgagtcgcgttctgccgcctcccgcctgtggtgcctcctgaa
ctgcgtccgccgtctaggtaagtttaaagctcAGGTCGAGACCGGGCCTTTGTCCGGCGCTCCCTTGGAGCCTACCTAGACTCAGC
CGGCTCTCCACGCTTTGCCTGACCCTGCTTGCTCAACTCTACGTCTTTGTTTCGTTTTCTGTTCTGCGCCGTTACA
GATCCAAGCTGTGACCGGCGCCTACGTCGAGAAGACAGGaattgccaCCATGGAGGCCCCAGCTCAGCTGCTGTTC
CTGCTGCTGCTGTGGCTGCCAGACACCACAGGAGAGGTGCAGCTGGTGGAGAGCGGCGGCGGCCTGGTGCAGC
CCGGCGGCAGCCTGAGACTGAGCTGCGCCGCCAGCGGCAGACCCTTCAGCATCTACGACATGGGCTGGTTCAG
ACAGGCCCCCGACAAGGAGAGAGAGAGCGTGGCCGTGATCAACCTGGCCAGAGGCAACACCTACTACGCCGA
CAGCGTGAAGGGCAGATTCACCATCAGCAGAGACAACGCCAAGAACACCGTGTACCTCCAGATGAACAGCCT
GAGAGCCGAGGACACCGCCGTGTACAGCTGCGGCGTGGACAGAAGACAGTACGGCCTGGGCATCCCCCCCCT
GGCCGACCACTGGGGCCAGGGCACCCAGGTGACCGTGAGCAGCTGATAAGGATCCTGCACTAGTGCtgtcgaCCT
GTGCCTTCTAGTTGCCAGCCATCTGTTGTTTGCCCCTCCCCCGTGCCTTCCTTGACCCTGGAAGGTGCCACTCCC
ACTGTCCTTTCCTAATAAAATGAGGAAATTGCATCGCATTGTCTGAGTAGGTGTCATTCTATTCTGGGGGGTGG
GGTGGGGCAGGACAGCAAGGGGGAGGATTGGGATGACAATAGCAGGCATGCTGGGGATGCGGTGGGCTCTAT
GGGATATCAATAAAATATCTTTATTTTCATTACATCTGTGTGTTGGTTTTTTGTGTGAATCAGTATGACTAACAT
ACGCTCTCCATCAAAACAAAACGAAACAAAACAAACTAGCAAAATAGGCTGTCCCCAGTGCAAGTGCAGGTG
CCAGAACATTTCTCTCTTAAGGTGCGCCTTATAGTGCGGAAAATACGGTATAAGATCT
SEQ ID NO: 48>pC23S-EGFP-8E 
CCTGTGCCTTCTAGTTGCCAGCCATCTGTTGTTTGCCCCTCCCCCGTGCCTTCCTTGACCCTGGAAGGTGCCACT
CCCACTGTCCTTTCCTAATAAAATGAGGAAATTGCATCGCATTGTCTGAGTAGGTGTCATTCTATTCTGGGGGG
TGGGGTGGGGCAGGACAGCAAGGGGGAGGATTGGGATGACAATAGCAGGCATGCTGGGGATGCGGTGGGCTC
TATGGGATATCAATAAAATATCTTTATTTTCATTACATCTGTGTGTTGGTTTTTTGTGTGAATCAGTATGACTAA
CATACGCTCTCCATCAAAACAAAACGAAACAAAACAAACTAGCAAAATAGGCTGTCCCCAGTGCAAGTGCAG
GTGCCAGAACATTTCTCTCTTAAGCATGCGTCAATTTTACGCATGATTATCTTTAACGTACGTCACAATATGATT
ATCTTTCTAGGGTTAAGGCGCGcccatgcatgggaggagaccgggacgagacgaaccctgataaatgcttcaataatattgattgaacaagatggattgcacg
caggttctccggccgcttgggtggagaggctattcggctatgactgggcacaacagacaatcggctgctctgatgccgccgtgttccggctgtcagcgcaggggcgcccggttcttttt
gtcaagaccgacctgtccggtgccctgaatgaactgcaagacgaggcagcgcggctatcgtggctggccacgacgggcgttccttgcgcagctgtactcgacgttgtcactgaagc
gggaagggactggctgctattgggcgaagtgccggggcaggatctcctgtcatctcaccttgctcctgccgagaaagtatccatcatggctgatgcaatgcggcggctgcatacgctt
gatccggctacctgcccattcgaccaccaagcgaaacatcgcatcgagagagcacgtactcggatggaagccggtcttgtcgatcaggatgatctggacgaagagcatcaggggct
cgcgccagccgaactgttcgccaggctcaaggcgagcatgcccgacggcgaggatctcgtcgtgacccatggcgatgcctgcttgccgaatatcatggtggaaaatggccgcttttc
tggattcatcgactgtggccggctgggtgtggcggaccgctatcaggacatagcgttggctacccgtgatattgctgaagagcttggcggcgaatgggctgaccgcttcctcgtgcttt
acggtatcgccgctcccgattcgcagcgcatcgccttctatcgccttcttgacgagttcttctgagacggcatgaccaaaatccccgtgagttttcgttccactgagcgtcagaccccgta
gaaaagatcaaaggatcttcttgagatcctttttttctgcgcgtaatctgctgcttgcaaacaaaaaaaccaccgctaccagcggtggtttgtttgccggatcaagagctaccaactctttttc
cgaaggtaactggcttcagcagagcgcagataccaaatactgttcttctagtgtagccgtagttaggccaccacttcaagaactctgtagcaccgcctacatacctcgctctgctaatcct
gttaccaGTGGctgctgccagtggcgataagtcgtgtcttaccgggttggactcaagacgatagttaccggataaggcgcagcggtcgggctgaacggggggttcgtgcacaca
gcccagcttggagcgaacgacctacaccgaactgagatacctacagcgtgagctatgagaaagcgccacgcttcccgaagggagaaaggcggacaggtatccggtaagcggcag
ggtcggaacaggagagcgcacgagggagcttccagggggaaacgcctggtatctttatagtcctgtcgggtttcgccacctctgacttgagcgtcgatttttgtgatgctcgtcagggg
ggcggagcctatggaaaaacgccagcaacgcggcctttttacggttcctggccttttgctggccttttgctcttcgattaaccctagaaagatagtctgcgtaaaattgacgcatgtctag
ACATCTTGGACCATTAGCTCCACAGGTATCTTCTTCCCTCTAGTGGTCATAACAGCAGCTTCAGCTACCTCTCTC
TCTGCTGCCAGTAGAGGGCACACTTACTTTACTCATTCCTTTAATGGTCTTTTCTGGACACCACTAGGGGTCAG
AAGTAGTTCATCAAACTTTCTTCCCTCCCTAGCTAGCGGTGTGGAAAGTCCCCAGGCTCCCCAGCAGGCAGAAG
TATGCAAAGCATGCATCTCAATTAGTCAGCAACCAGGTGTGGAAAGTCCCCAGGCTCCCCAGCAGGCAGAAGT
ATGCAAAGCATGCATCTCAATTAGTCAGCAACCAAGGATCTGCGatcgctccggtgcccgtcagtgggcagagcgcacatcgcccacagt
ccccgagaagttggggggaggggtcggcaattgaacgggtgcctagagaaggtggcgcggggtaaactgggaaagtgatgtcgtgtactggctccgcctttttcccgagggtggg
ggagaaccgtatataagtgcagtagtcgccgtgaacgttctttttcgcaacgggtttgccgccagaacacagctgaagcttcgaggggctcgcatctctccttcacgcgcccgccgccc
tacctgaggccgccatccacgccggttgagtcgcgttctgccgcctcccgcctgtggtgcctcctgaactgcgtccgccgtctaggtaagtTTATagctcaggtcgagaccgggcc
tttgtccggcgctcccttggagcctacctagactcagccggctctccacgctttgcctgaccctgcttgctcaactctacgtctttgtttcgttttctgttctgcgccgttacagatccaagctg
tgaccggcgcctacgaattCGCCACCATGGCCTTACCAGTGACCGCCTTGCTCCTGCCGCTGGCCTTGCTGCTCCACGCCG
CCAGGCCGagcaagggcgaggagctgttcaccggggtggtgcccatcctggtcgagctggacggcgacgtaaacggccacaagttcagcgtgtccggcgagggcgagg
 
gcgatgccacctacggcaagctgaccctgaagttcatctgcaccaccggcaagctgcccgtgccctggcccaccctcgtgaccaccctgacctacggcgtgcagtgcttcagccgct
accccgaccacatgaagcagcacgacttcttcaagtccgccatgcccgaaggctacgtccaggagcgcaccatcttcttcaaggacgacggcaactacaagacccgcgccgaggt
gaagttcgagggcgacaccctggtgaaccgcatcgagctgaagggcatcgacttcaaggaggacggcaacatcctggggcacaagctggagtacaactacaacagccacaacgt
ctatatcatggccgacaagcagaagaacggcatcaaggtgaacttcaagatccgccacaacatcgaggacggcagcgtgcagctcgccgaccactaccagcagaacacccccatc
ggcgacggccccgtgctgctgcccgacaaccactacctgagcacccagtccgccctgagcaaagaccccaacgagaagcgcgatcacatggtcctgctggagttcgtgaccgccg
ccgggatcactctcggcatggacgagctgtacaagTTCGTGCCGGTCTTCCTGCCAGCGAAGCCCACCACGACGCCAGCGCCGCGA
CCACCAACACCGGCGCCCACCATCGCGTCGCAGCCCCTGTCCCTGCGCCCAGAGGCGTGCCGGCCAGCGGCG
GGGGGCGCAGTGCACACGAGGGGGCTGGACTTCGCCTGTGATcccttttgggtgctggtggtggttggtggagtcctggcttgctatagcttgcta
gtaacagtggcctttattattttctgggtgaggagtaagaggagcaggctcctgcacagtgactacatgaacatgactccccgccgccccgggcccacccgcaagcattaccagccct
atgccccaccacgcgacttcgcagcctatcgctccagagtgaagttcagcaggagcgcagacgcccccgcgtaccagcagggccagaaccagctctataacgagctcaatctagg
acgaagagaggagtacgatgttttggacaagagacgtggccgggaccctgagatggggggaaagccgagaaggaagaaccctcaggaaggcctgtacaatgaactgcagaaag
ataagatggcggaggcctacagtgagattgggatgaaaggcgagcgccggaggggcaaggggcacgatggcctttaccagggtctcagtacagccaccaaggacacctacgac
gcccttcacatgcaggccctgccccctcgctgaTAAgtcga
SEQ ID NO: 49> truncated antitoxin 0637 (50aa)
   MSTDERKLEIIRKVSSDIDEPTLEAIEYLLSTPSDIPEPILRVIEQAMAE*
SEQ ID NO: 50> truncated antitoxin CcdA₄₃₀₀₉ (69aa)
   MPKKATNLSLNSELLAEAKRLNINLSATMEKALEKEVRQRLKTEWLKQNAEGINACNELTENHGLFSDS
SEQ ID NO: 51> truncated antitoxin 0637 (60aa)
   MSTDERKLEIIRKVSSDIDEPTLEAIEYLLSTPSDIPEPILRVIEQAMAEEPLEEYTSAK*
SEQ ID NO: 52>Antitoxin 0637 (68aa)
   MSTDERKLEIIRKVSSDIDEPTLEAIEYLLSTPSDIPEPILRVIEQAMAEEPLEEYTSAKEVIEKSRT*
SEQ ID NO: 53> truncated antitoxin CcdA₄₃₀₀₉ (73aa)
   MPKKATNLSLNSELLAEAKRLNINLSATMEKALEKEVRQRLKTEWLKQNAEGINACNELTENHGLFSDSYRVF
SEQ ID NO: 54> truncated antitoxin CcdA₄₃₀₀₉ (77aa) 
MRNAYSTQAPKKATNLSLNSELLAEAKRLNINLSATMEKALEKEVRQRLKTEWLKQNAEGINACNELTENHGLFS
DS
SEQ ID NO: 55>Antitoxin CcdA₄₃₀₀₉ 
MRNAYSTQAPKKATNLSLNSELLAEAKRLNINLSATMEKALEKEVRQRLKTEWLKQNAEGINACNELTENHGLFS
DSYRVF
SEQ ID NO: 56>Antitoxin 0637 (CpG free) 
ATGTCAACAGATGAAAGAAAATTGGAGATCATCAGAAAAGTGAGTTCTGATATTGATGAGCCTACTCTGGAAG
CAATTGAATATTTGTTATCAACCCCTTCAGATATCCCAGAACCAATACTGAGAGTTATTGAACAAGCAATGGCA
GAAGAACCTCTTGAGGAATACACTTCAGCCAAAGAGGTTATTGAGAAAAGCAGAACCTAA
SEQ ID NO: 57>Antitoxin CcdA₄₃₀₀₉ (CpG free) 
ATGAGAAATGCATATAGTACCCAAGCACCAAAGAAAGCTACAAACCTGAGCTTAAACAGTGAGTTACTTGCAG
AGGCTAAAAGATTAAATATAAACCTTAGTGCAACAATGGAAAAGGCACTTGAAAAAGAAGTAAGACAAAGAC
TGAAGACTGAATGGTTAAAGCAAAATGCTGAGGGCATTAATGCTTGTAATGAGCTAACTGAAAATCATGGACT
ATTTTCTGATTCTTACAGAGTATTCTAA
SEQ ID NO: 58>R6K replicator (CpG free) 
AATCAGCAGTTCAACCTGTTGATAGTATGTACTAAGCTCTCATGTTTAATGTACTAAGCTCTCATGTTTAATGA
ACTAAACCCTCATGGCTAATGTACTAAGCTCTCATGGCTAATGTACTAAGCTCTCATGTTTCATGTACTAAGCT
CTCATGTTTGAACAATAAAATTAATATAAATCAGCAACTTAAATAGCCTCTAAGGTTTTAAGTTTTATAAGAAA
AAAAAGAATATATAAGGCTTTTAAAGGTTTTAAGGTTTCCTAGCT
SEQ ID NO: 59>pCpGfree MCS-0637 empty miniplasmids 
GAATTCTTAATTAAAATCAGCAGTTCAACCTGTTGATAGTATGTACTAAGCTCTCATGTTTAATGTACTAAGCT
CTCATGTTTAATGAACTAAACCCTCATGGCTAATGTACTAAGCTCTCATGGCTAATGTACTAAGCTCTCATGTTT
CATGTACTAAGCTCTCATGTTTGAACAATAAAATTAATATAAATCAGCAACTTAAATAGCCTCTAAGGTTTTAA
GTTTTATAAGAAAAAAAAGAATATATAAGGCTTTTAAAGGTTTTAAGGTTTCCTAGCTTTAGGTTCTGCTTTTCT
CAATAACCTCTTTGGCTGAAGTGTATTCCTCAAGAGGTTCTTCTGCCATTGCTTGTTCAATAACTCTCAGTATTG
GTTCTGGGATATCTGAAGGGGTTGATAACAAATATTCAATTGCTTCCAGAGTAGGCTCATCAATATCAGAACTC
ACTTTTCTGATGATCTCCAATTTTCTTTCATCTGTTGACATGATGGCCCTCCTATAGTGAGTTGTATTATACTATG
CAGATATACTATGCCAATGTTTAATTGTCAACTAGT
SEQ ID NO: 60>pCpGfree MCS-43009 empty miniplasmids 
TTAATTAAAATCAGCAGTTCAACCTGTTGATAGTATGTACTAAGCTCTCATGTTTAATGTACTAAGCTCTCATGT
TTAATGAACTAAACCCTCATGGCTAATGTACTAAGCTCTCATGGCTAATGTACTAAGCTCTCATGTTTCATGTA
CTAAGCTCTCATGTTTGAACAATAAAATTAATATAAATCAGCAACTTAAATAGCCTCTAAGGTTTTAAGTTTTA
TAAGAAAAAAAAGAATATATAAGGCTTTTAAAGGTTTTAAGGTTTCCTAGCTTTAGAATACTCTGTAAGAATCA
GAAAATAGTCCATGATTTTCAGTTAGCTCATTACAAGCATTAATGCCCTCAGCATTTTGCTTTAACCATTCAGTC
TTCAGTCTTTGTCTTACTTCTTTTTCAAGTGCCTTTTCCATTGTTGCACTAAGGTTTATATTTAATCTTTTAGCCTC
TGCAAGTAACTCACTGTTTAAGCTCAGGTTTGTAGCTTTCTTTGGTGCTTGGGTACTATATGCATTTCTCATGAT
GGCCCTCCTATAGTGAGTTGTATTATACTATGCAGATATACTATGCCAATGTTTAATTGTCAACTAGT
SEQ ID NO: 61>pO-PST2-dCGEGFP

tcgaagagcaaaaggccagcaaaaggccaggaaccgtaaaaaggccgcgttgctggcgtttttccataggctccgcccccctgacgagcatcacaaaaatcgacgctcaagtcaga
ggtggcgaaacccgacaggactataaagataccaggcgtttccccctggaagctccctcgtgcgctctcctgttccgaccctgccgcttaccggatacctgtccgcctttctcccttcgg
gaagcgtggcgctttctcatagctcacgctgtaggtatctcagttcggtgtaggtcgttcgctccaagctgggctgtgtgcacgaaccccccgttcagcccgaccgctgcgccttatccg

gtaactatcgtcttgagtccaacccggtaagacacgacttatcgccactggcagcagCCACtggtaacaggattagcagagcgaggtatgtaggcggtgctacagagttcttgaag
tggtggcctaactacggctacactagaagaacagtatttggtatctgcgctctgctgaagccagttaccttcggaaaaagagttggtagctcttgatccggcaaacaaaccaccgctggt
agcggtggtttttttgtttgcaagcagcagattacgcgcagaaaaaaaggatctcaagaagatcctttgatcttttctacggggtctgacgctcagtggaacgaaaactcacggggattttg
gtcatgccgtctcagaagaactcgtcaagaaggcgatagaaggcgatgcgctgcgaatcgggagcggcgataccgtaaagcacgaggaagcggtcagcccattcgccgccaagc
tcttcagcaatatcacgggtagccaacgctatgtcctgatagcggtccgccacacccagccggccacagtcgatgaatccagaaaagcggccattttccaccatgatattcggcaagc
aggcatcgccatgggtcacgacgagatcctcgccgtcgggcatgctcgccttgagcctggcgaacagttcggctggcgcgagcccctgatgctcttcgtccagatcatcctgatcgac
aagaccggcttccatccgagtacgtgctctctcgatgcgatgtttcgcttggtggtcgaatgggcaggtagccggatcaagcgtatgcagccgccgcattgcatcagccatgatggata
ctttctcggcaggagcaaggtgagatgacaggagatcctgccccggcacttcgcccaatagcagccagtcccttcccgcttcagtgacaacgtcgagtacagctgcgcaaggaacg
cccgtcgtggccagccacgatagccgcgctgcctcgtcttgcagttcattcagggcaccggacaggtcggtcttgacaaaaagaaccgggcgcccctgcgctgacagccggaaca
cggcggcatcagagcagccgattgtctgttgtgcccagtcatagccgaatagcctctccacccaagcggccggagaacctgcgtgcaatccatcttgttcaatcataatattattgaagc
atttatcagggttcgtctcgtcccggtctccTCCCTATACCGTATTTTCCGCACTATAAGGCGCACCCCGTATTTTCCGCACTATAA
GGCGCACCTctagACATCTTGGACCATTAGCTCCACAGGTATCTTCTTCCCTCTAGTGGTCATAACAGCAGCTTCA
GCTACCTCTCTCTCTGCTGCCAGTAGAGGGCACACTTACTTTACTCATTCCTTTAATGGTCTTTTCTGGACACCA
CTAGGGGTCAGAAGTAGTTCATCAAACTTTCTTCCCTCCCTAGCTAGCGGTGTGGAAAGTCCCCAGGCTCCCCA
GCAGGCAGAAGTATGCAAAGCATGCATCTCAATTAGTCAGCAACCAGGTGTGGAAAGTCCCCAGGCTCCCCAG
CAGGCAGAAGTATGCAAAGCATGCATCTCAATTAGTCAGCAACCAAGGATCTGCGatcgctccggtgcccgtcagtgggcaga
gcgcacatcgcccacagtccccgagaagttggggggaggggtcggcaattgaacgggtgcctagagaaggtggcgcggggtaaactgggaaagtgatgtcgtgtactggctccg
cctttttcccgagggtgggggagaaccgtatataagtgcagtagtcgccgtgaacgttctttttcgcaacgggtttgccgccagaacacagctgaagcttcgaggggctcgcatctctcc
ttcacgcgcccgccgccctacctgaggccgccatccacgccggttgagtcgcgttctgccgcctcccgcctgtggtgcctcctgaactgcgtccgccgtctaggtaagtTTATagc
tcaggtcgagaccgggcctttgtccggcgctcccttggagcctacctagactcagccggctctccacgctttgcctgaccctgcttgctcaactctacgtctttgtttcgttttctgttctgcg
ccgttacagatccaagctgtgaccggcgcctacgaattgccaccATGGTGAGCAAGGGAGAGGAGCTGTTCACTGGGGTGGTGCCCATC
CTGGTGGAGCTGGATGGAGATGTGAATGGCCACAAGTTCTCTGTGTCTGGAGAGGGAGAGGGAGATGCCACCT
ATGGCAAGCTGACCCTGAAGTTCATCTGCACCACAGGCAAGCTGCCTGTGCCCTGGCCCACCCTGGTGACCAC
CCTGACCTATGGAGTGCAGTGCTTCAGCAGATACCCTGACCACATGAAGCAGCATGACTTCTTCAAGTCTGCCA
TGCCTGAGGGCTATGTCCAGGAGAGAACCATCTTCTTCAAGGATGATGGCAACTACAAGACCAGAGCTGAGGT
GAAGTTTGAGGGAGACACCCTGGTGAACAGAATTGAGCTGAAGGGCATTGACTTCAAGGAGGATGGCAACAT
CCTGGGGCACAAGCTGGAGTACAACTACAACAGCCACAATGTCTATATCATGGCTGACAAGCAGAAGAATGGC
ATCAAGGTGAACTTCAAGATCAGACACAACATTGAGGATGGCTCTGTGCAGCTGGCTGACCACTACCAGCAGA
ACACCCCCATTGGAGATGGCCCTGTGCTGCTGCCTGACAACCACTACCTGAGCACCCAGTCTGCCCTGAGCAA
GGACCCCAATGAGAAGAGAGATCACATGGTCCTGCTGGAGTTTGTGACAGCTGCTGGGATCACTCTGGGCATG
GATGAGCTGTACAAGTAAggatcctgcactagtgctgtcgaCCTGTGCCTTCTAGTTGCCAGCCATCTGTTGTTTGCCCCTCC
CCCGTGCCTTCCTTGACCCTGGAAGGTGCCACTCCCACTGTCCTTTCCTAATAAAATGAGGAAATTGCATCGCA
TTGTCTGAGTAGGTGTCATTCTATTCTGGGGGGTGGGGTGGGGCAGGACAGCAAGGGGGAGGATTGGGATGAC
AATAGCAGGCATGCTGGGGATGCGGTGGGCTCTATGGGATATCAATAAAATATCTTTATTTTCATTACATCTGT
GTGTTGGTTTTTTGTGTGAATCAGTATGACTAACATACGCTCTCCATCAAAACAAAACGAAACAAAACAAACT
AGCAAAATAGGCTGTCCCCAGTGCAAGTGCAGGTGCCAGAACATTTCTCTCTTAAGGTGCGCCTTATAGTGCG
GAAAATACGGGGTGCGCCTTATAGTGCGGAAAATACGGTATAAGATCT

## Claims

1. A transposase which is a mutant transposase or a fusion transposase, wherein
the mutant transposase comprises amino acid mutations as compared to a wild-type PS transposase as shown in SEQ ID NO: 1, the amino acid mutations are that any one or more amino acids in a double-stranded DNA binding oligomerization domain of a wild-type PS transposase are mutated to a positively charged amino acid; and/or the amino acid mutation comprises one or more mutations selected from the following group: TQS57-59KKA, T129R, T129K, 198K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, I237V, Q69R, L100I, K96R, Q162R, A271K, R372K, H20Y, TYCR150-153KFVI, N232K, A363N, A377C, Q346M, A17V, N105G, Q76E, 1354V, VS133-134IA, S253K, V370I, SV297-298TE, EH286-286DS, A352S, ANS241-243VHE, S297T, A79I, T147S, I178M, 1276V, Q331E, R257E, T339S, A79V, H104L, V74L, S107E, Q142E, R375K, S243E, R195K, Q22E, A328Q, G111A, R110K, I365V, VTE380-382SNA, R190K, RA372-373KS, K73L, K121R, R102K, C152V, IY276-277VW, V2921, K73E, H104M, I239V, K42E, K356Q, T150K, G316S, IY259-260VW, V53M, E108D, V53L, DKV72-74EEL, M280F, S85G, N63K, V91L, K251L, H124N, K308S, N10D, H7R, G112S, H7K, TA327-328KE; and
the fusion transposase is formed by fusing a functional polypeptide onto the wild-type PS transposase or a mutant thereof, and the functional polypeptide is a DNA sequence-specific or non-specific binding domain, and/or a nuclear localization signal domain.

2. The transposase according to claim 1, wherein the mutant, compared to the wild-type PS transposase shown in SEQ ID NO: 1, comprises one or more mutations selected from the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V, Q69R, L100I, K96R, Q162R, A271K, R372K, H20Y, TYCR150-153KFVI, N232K, A363N, A377C, Q346M, A17V, N105G, Q76E, I354V, VS133-134IA, S253K, V370I, SV297-298TE, EH286-286DS, A352S, ANS241-243VHE, S297T, A79I, T147S, I178M, 1276V, Q331E, R257E, T339S, A79V, H104L, V74L, S107E, Q142E, R375K, S243E, R195K, Q22E, A328Q, G111A, R110K, I365V, VTE380-382SNA, R190K, RA372-373KS, K73L, K121R, R102K, C152V, IY276-277VW, V2921, K73E, H104M, 1239V, K42E, K356Q, T150K, G316S, IY259-260VW, V53M, E108D, V53L, DKV72-74EEL, M280F, S85G, N63K, V91L, K251L, H124N, K308S, N10D, H7R, G112S, H7K, TA327-328KE.

3. The transposase according to claim 1 or 2, wherein the mutant, compared to the wild-type PS transposase shown in SEQ ID NO: 1, comprises one or more mutations selected from the following group: TQS57-59KKA, T129R, T129K, I98K, TQ57-58RK, TQ57-58RK\T129K, TQ57-58RK\T129R, E32K, E32K\T129K, E32K\T129R, TQ57-58RK\I98K, TQ57-58RK\I98K\T129K, TQS57-59KKA\I98K, TQS57-59KKA\I98K\T129K, R123H, Q136K, K16R, E47K, TQ57-58RR, E32K\T57R\Q58R, T57R, T57K, Q58K, Q58R, S59A, M95L, Y46Q, A8S, T187K, I35V, N199H, N193S, T350S, Q22K, T368E, N213D, H24R, T150A, H165D, K55R, K73R, L228M, E335S, K159H, V359L, T129Q, H215K, R51K, A84L, Q69E, I284L, K45R, H215E, H215Q, 1237V, Q69R, L100I, K96R, Q162R, A271K, R372K, H20Y, TYCR150-153KFVI, N232K, A363N, A377C, Q346M, A17V, N105G, Q76E, 1354V, VS133-134IA, S253K, V370I, SV297-298TE, EH286-286DS, A352S, ANS241-243VHE, S297T, A79I, T147S, I178M, 1276V, Q331E, R257E, T339S, A79V, H104L, V74L, S107E, Q142E, R375K, S243E, R195K, Q22E, A328Q, G111A, R110K, I365V, VTE380-382SNA, R190K, RA372-373KS, K73L, K121R, R102K, C152V, IY276-277VW, V292I, K73E, H104M, I239V, K42E, K356Q, T150K, G316S, IY259-260VW, V53M, E108D, V53L, DKV72-74EEL, M280F, S85G, N63K, V91L, K251L, H124N, K308S, N10D, H7R, G112S, H7K, TA327-328KE.

4. The transposase according to any one of claims 1-3, wherein the DNA sequence-specific or non-specific binding domain comprises a leucine zipper structural domain, a CRISPR/Cas structural domain, a TALE domain, a zinc finger domain, an AAV Rep DNA-binding domain, or any combination thereof, and preferably, the leucine zipper structural domain has an amino acid sequence as shown in any one of SEQ ID NOs: 28-31, and/or
the nuclear localization signal domain comprises SV40 NLS, C-myc NLS, TAF1 NLS, TP53 NLS, STAT3 NLS or any combination thereof, and/or
the functional polypeptide comprises or does not comprise a linker for connecting to the wild-type PS transposase or the mutant thereof.

5. A polynucleotide for encoding the transposase of any one of claims 1-4; preferably, the polynucleotide is DNA or a messenger RNA.

6. A transposon which is recognizable by the transposase according to any one of claims 1-4, wherein the transposon has any one, two or three features selected from (1) to (3) below:
(1) the transposon has a mutation inverted terminal repeat, the mutation inverted terminal repeat has 1-5 nucleotide mutations compared to a wild-type PS transposon inverted terminal repeat shown in SEQ ID NO: 2 or 3;
(2) the transposon has more than two inverted terminal repeats at least one end; and
(3) the transposon has more than two repeats of the TA sequence outside the inverted terminal repeat at least one end.

7. The transposon according to claim 6, the mutation inverted terminal repeat is as shown in any one of SEQ ID NOs: 4-27, preferably as shown in any one of SEQ ID NOs: 4-6, or is a nucleotide sequence comprising any combination of nucleotide mutations in SEQ ID NOs: 4-27, or is a reverse complementary sequence thereof.

8. The transposon according to claim 6 or 7, wherein the transposon has two inverted terminal repeats at each of two ends and comprises four TA sequence repeats outside the inverted terminal repeats at each of the two ends.

9. The transposon according to any one of claims 6-8, wherein the transposon comprises a target gene expression frame positioned between the inverted terminal repeats, preferably, the target gene encodes a protein selected from a cellular receptor, an immune checkpoint protein, a cytokine, a T cell receptor, a B cell receptor, a chimeric antigen receptor, and any combination thereof.

10. A transposon system comprising the transposase according to any one of claims 1-4 or the polynucleotide for encoding the transposase according to claim 5; and a transposon being a PS transposon or the transposon according to any one of claims 6-9.

11. The transposon system according to claim 10, wherein the polynucleotide for encoding the transposase is an mRNA, preferably, the mRNA is chemically modified, and comprises but is not limited to pseudouridine (ψ), N1-methylpseudouridine (m1ψ), 5-methylcytosine nucleoside (m5C), 5-methoxyuridine (5moU), etc.

12. The transposon system according to claim 10 or 11, wherein the polynucleotide for encoding the transposase and/or the transposon is present in a DNA vector, preferably, the DNA vector is a plasmid vector or a minicircular DNA vector, and is preferably an antibiotic resistance gene-free miniplasmid vector; and/or the DNA vector is a minivector, and a backbone sequence of the minivector is in a length of 600 bp or less, and has a reduced CpG DNA motif and/or a CpG-free DNA motif; or
the polynucleotide for encoding the transposase and/or the transposon is present in a viral vector, preferably, the viral vector is selected from an adenovirus vector, an adeno-associated virus vector, a retrovirus vector, a herpes simplex virus vector, or a poxvirus vector.

13. The transposon system according to claim 12, wherein the polynucleotide for encoding the transposase and the transposon is present in the same vector or in different vectors.

14. A host cell comprising or capable of producing the transposase according to any one of claims 1-4, the polynucleotide according to claim 5, the transposon according to any one of claims 6-9, or the transposon system according to any one of claims 10-13.

15. A method of preparing a cell comprising: the step of introducing into the cell the transposase according to any one of claims 1-4, the polynucleotide according to claim 5, the transposon according to any one of claims 6-9, or the transposon system according to any one of claims 10-13,
preferably,
the introduction comprises contacting the cell with the polynucleotide, and/or
the introduction comprises contacting the cell with a DNA vector comprising the transposon, and/or
the introduction comprises contacting the cell with mRNA for encoding the transposase and a plasmid vector comprising the transposon, and/or
the introduction comprises contacting the cell with a plasmid vector comprising the polynucleotide and the transposon, and/or
the introduction comprises contacting the cell with the plasmid vector comprising the polynucleotide and the plasmid vector comprising the transposon, and/or
the introduction comprises contacting the cell with the transposase.

16. The method according to claim 15, wherein the cell is derived from an animal, such as a vertebrate or an invertebrate, preferably, a mammal, and further preferably a human; and/or the cell is an immune cell, and preferably, a T cell.

17. The method according to claim 15 or 16, wherein the introduction comprises transfecting the cell with aid of electroporation, microinjection, calcium phosphate precipitation, a cationic polymer, a dendrimer, a liposome, a lipid nanoparticle, particulate bombardment, fugene, direct acoustic loading, cell extrusion, optical transfection, protoplasmic fusion, impalefection, magneto-transfection, nuclear transfection, virus-mediated transformation or any combination thereof.

18. The method according to claim 16 or 17, wherein the introduction comprises electroporation of the cell.

19. The method according to claim 18, wherein the introduction comprises contacting the cell with the mRNA for encoding the transposase and a plasmid comprising the transposon, preferably, the plasmid is a minivector, a backbone sequence of the minivector is in a length of 600 bp or less, and has a reduced CpG DNA motif and/or a CpG-free DNA motif, and/or the plasmid is an antibiotic resistance gene-free miniplasmid.

20. A kit comprising the transposase according to any one of claims 1-4, the polynucleotide according to claim 5, the transposon according to any one of claims 6-9, the transposon system according to any one of claims 10-13, or the cell according to claim 14.

21. A pharmaceutical composition comprising a pharmaceutically acceptable excipient and a cell prepared by the method according to any one of claims 15-19.

22. Application of the transposase according to any one of claims 1-4, the polynucleotide according to claim 5, the transposon according to any one of claims 6-9, the transposon system according to any one of claims 10-13, the cell according to claim 14, or the pharmaceutical composition according to claim 21 being selected from any one of (1)-(6) below:
(1) application in preparation of a drug or a reagent for integrating a target gene expression frame into a host cell genome;
(2) application in preparation of a tool for integrating the target gene expression frame into the host cell genome;
(3) application in preparation of a transgenic animal and a transgenic cell;
(4) application in preparation of a drug or a formulation for genomic research, gene therapy, cell therapy, or stem cell induction and post-induction differentiation;
(5) application in preparation of a tool for the genomic research, the gene therapy, the cell therapy, or the stem cell induction and the post-induction differentiation; and
(6) application in preparation of a kit, an engineered immune cell, or the pharmaceutical composition.
